# EUROPEAN PATENT APPLICATION

(11) **EP 2 108 704 A1**
(43) Date of publication of application: **14.10.2009**
(21) Application number: 08006933.9
(22) Date of filing: 07.04.2008
(51) Int. Cl.: C12Q 1/68

(54) **Androgenetic alopecia**

(71) Applicant: Life & Brain GmbH, 53127 Bonn (DE); Rheinische Friedrich-Wilhelms-Universität, 53113 Bonn (DE); Heinrich-Heine-Universität Düsseldorf, 40225 Düsseldorf (DE)
(72) Inventor: Nöthen, Markus M., 53115 Bonn (DE); Kruse, Roland, 41468 Neuss (DE); Hillmer, Axel M., 53129 Bonn (DE); Brockschmidt, Felix F., 53129 Bonn (DE)
(74) Representative: Vossius & Partner

(57) **Abstract**

The present invention relates to a genetic marker for androgenetic alopecia characterised in that the genetic marker is located in a chromosomal region of human chromosome 20p11 and wherein the genetic marker is selected from the group consisting of a single nucleotide polymorphism (SNP), variable number of tandem repeat (VNTR), microsatellites or short tandem repeats (STR). The invention also relates to a nucleic acid molecule selected from the group consisting of (a) a nucleic acid molecule consisting of at least 17 consecutive nucleotides of any one of SEQ ID NOs:1 to 292; wherein the at least 17 consecutive nucleotides contain an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292; (b) a nucleic acid molecule the complementary strand of which hybridises under stringent conditions to the nucleic acid molecule of (a), wherein said nucleic acid molecule has at a position corresponding to position 201 of any one of SEQ ID NOs:1 to 77 an adenosine, corresponding to position 201 of any one of SEQ ID NOs:78 to 149 a guanosine, corresponding to position 201 of any one of SEQ ID NOs:150 to 219 a thymidine or corresponding to position 201 of any one of SEQ ID NOs:220 to 292 a cytidine; (c) a nucleic acid molecule identical to the nucleic acid molecule of (a) or (b) wherein each thymidine is replaced by uridine. Further, the invention also relates to a nucleic acid molecule selected from the group consisting of (a) a nucleic acid molecule consisting of at least 17 consecutive nucleotides of any one of SEQ ID NOs:1 to 292; wherein the at least 17 consecutive nucleotides contain an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; (b) a nucleic acid molecule the complementary strand of which hybridises under stringent conditions to the nucleic acid molecule of (a), wherein said nucleic acid molecule has at a position corresponding to position 201 of any one of SEQ ID NOs: SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264 an adenosine, corresponding to position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215 a cytidine, corresponding to position 201 of any one of SEQ ID NOs:1, 2, 4, 5, 8 to 11, 13, 14, 16, 18, 20 to 23, 26 - to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 a guanosine or corresponding to position 201 of any one of SEQ ID NOs: 6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292 a thymidine; (c) a nucleic acid molecule identical to the nucleic acid molecule of (a) or (b) wherein each thymidine is replaced by uridine. The invention also relates to methods for testing for a predisposition or presence of androgenetic alopecia comprising testing a sample obtained from a prospective patient or from a person suspected of carrying a predisposition for androgenetic alopecia. Further, the invention relates to a diagnostic composition for the detection of androgenetic alopecia or a predisposition therefore as well as to a kit.

## Description

The present invention relates to a genetic marker for androgenetic alopecia characterised in that the genetic marker is located in a chromosomal region of human chromosome 20p11 and wherein the genetic marker is selected from the group consisting of a single nucleotide polymorphism (SNP), variable number of tandem repeat (VNTR), microsatellites or short tandem repeats (STR). The invention also relates to a nucleic acid molecule selected from the group consisting of (a) a nucleic acid molecule consisting of at least 17 consecutive nucleotides of any one of SEQ ID NOs:1 to 292; wherein the at least 17 consecutive nucleotides contain an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292; (b) a nucleic acid molecule the complementary strand of which hybridises under stringent conditions to the nucleic acid molecule of (a), wherein said nucleic acid molecule has at a position corresponding to position 201 of any one of SEQ ID NOs:1 to 77 an adenosine, corresponding to position 201 of any one of SEQ ID NOs:78 to 149 a guanosine, corresponding to position 201 of any one of SEQ ID NOs:150 to 219 a thymidine or corresponding to position 201 of any one of SEQ ID NOs:220 to 292 a cytidine; (c) a nucleic acid molecule identical to the nucleic acid molecule of (a) or (b) wherein each thymidine is replaced by uridine. Further, the invention also relates to a nucleic acid molecule selected from the group consisting of (a) a nucleic acid molecule consisting of at least 17 consecutive nucleotides of any one of SEQ ID NOs:1 to 292; wherein the at least 17 consecutive nucleotides contain an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150, 152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; (b) a nucleic acid molecule the complementary strand of which hybridises under stringent conditions to the nucleic acid molecule of (a), wherein said nucleic acid molecule has at a position corresponding to position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264 an adenosine, corresponding to position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215 a cytidine, corresponding to position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 a guanosine or corresponding to position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292 a thymidine; (c) a nucleic acid molecule identical to the nucleic acid molecule of (a) or (b) wherein each thymidine is replaced by uridine. The invention also relates to methods for testing for a predisposition or presence of androgenetic alopecia comprising testing a sample obtained from a prospective patient or from a person suspected of carrying a predisposition for androgenetic alopecia. Further, the invention relates to a diagnostic composition for the detection of androgenetic alopecia or a predisposition therefore as well as to a kit.

Several documents are cited throughout the text of this specification. The disclosure content of the documents cited herein (including manufacturer's specifications, instructions, etc.) is herewith incorporated by reference.

Androgenetic alopecia (AGA, [MIM 109200]) is the most common form of hair loss in humans, occurring both in men and women. In men, this condition is commonly known as male pattern baldness (MPB), as hair is lost in a well-defined pattern, beginning above both temples. Over time, the hairline recedes and hair also thins at the crown (near the top of the head), often progressing to partial or complete baldness. In Caucasians, MBP is noticeable in about 20% of men aged 20, and increases steadily with age, so that a male in his 90s has a 90% chance of having some degree of MPB. In women, onset of androgenetic alopecia tends to occur later and in a milder form (Hanneken et al. (2003), Hautarzt 54:703-712). The pattern of hair loss in women also differs from male-pattern baldness. In women, the hair becomes thinner all over the head, and the hairline does not recede. Androgenetic alopecia in women rarely leads to total baldness.

The negative psychosocial effects associated with hair loss include decreased self-esteem, dissatisfaction with body image or appearance, self-consciousness, perception of aging, and often emotional stress. Thus, androgenetic alopecia has a considerable effect on the quality of life for many of those affected.

In addition to the psychosocial effects, recent studies indicate associations of male pattern baldness with: (1) benign prostatic hyperplasia (MIM 600082) (Hawk et al., (2000) B.I. Cancer Epidemiol Biomarkers Prev 9, 523-527); (2) coronary heart disease (Lotufo et al., (2000) J.E. Arch Intern Med 160, 165-171); (3) hyperinsulinemia; and (4) insulin-resistance-associated disorders, such as obesity (MIM 601665), hypertension (MIM 145500), and dyslipidemia (Matilainen et al, (2000) S. Lancet 356, 1165-1166). MBP is also a risk factor for clinical prostate cancer (MIM 176807) (Oh et al., (1998) Urology 51, 744-748).

The origin of androgenetic alopecia is genetic, with a high heritability of 81 % (Nyholt DR, et al. (2003), J Invest Dermatol 121:1561-1564). So far, the androgen receptor gene (*AR*) located on the X chromosome is the only risk gene identified (Ellis, J.A., et al. (2001), J Invest Dermatol 116, 452-455; Ellis, J.A. et al. (2007), Hum Genet 121, 451-457; Hillmer et al. (2005) Am J Hum Genet 77, 140-148). Variations in the *AR* gene are assumed to lead to increased activity of androgen receptors in hair follicles, which can lead to a shorter cycle of hair growth and the growth of shorter and thinner strands of hair. Additionally, there is a delay in the growth of new hair to replace hair shafts that are shed.

The investigation of a large number of genetic variants covering the AR locus demonstrated that genetic variation in the *AR* gene is one prerequisite for the development of early-onset androgenetic alopecia, with an etiologic fraction of 0.46 (Hillmer et al. (2005) Am J Hum Genet 77, 140-148). However, the genetic variation in *AR*, which is located on the X chromosome, cannot explain the resemblance of fathers and sons with respect to androgenetic alopecia (Küster W and Happle R (1984), J Am Acad Dermatol 11, 921-926; Ellis, JA et al. (1998), J Invest Dermatol 110, 849-853), since sons always inherit the X chromosome from their mothers.

Thus, androgen dependence is an important characteristic of androgenetic alopecia and genetic disposition, which is assumed to be polygenic, plays a substantial role in the development of androgenetic alopecia (Küster W and Happle R (1984), J Am Acad Dermatol 11, 921-926; Ellis, JA et al. (1998), J Invest Dermatol 110, 849-853; Nyholt, DR et al. (2003), J Invest Dermatol 121, 1561-1564).
The psychosocial effects as well as the association of androgenetic alopecia with a variety of diseases underlines the value of understanding the molecular basis of this common form of hair loss.

The technical problem underlying the present invention was the provision of means and methods useful in the diagnosis of androgenetic alopecia or a predisposition thereto.

The solution to this technical problem is achieved by providing the embodiments characterised in the claims.

Accordingly, the present invention relates to a genetic marker for androgenetic alopecia characterised in that the genetic marker is located in a chromosomal region of human chromosome 20p11 and wherein the genetic marker is selected from the group consisting of a single nucleotide polymorphism (SNP), variable number of tandem repeat (VNTR), microsatellites or short tandem repeats (STR).

The term "genetic marker" in accordance with the present invention refers to a DNA sequence at a genomic locus that varies due to mutation and that is associated with a disease. A genetic marker may be a short DNA sequence, such as a sequence surrounding a single base-pair change (single nucleotide polymorphism), or a long one, like for example microsatellites.

The term "androgenetic alopecia (AGA)" as used in accordance with the present invention refers to hair loss in humans.

The term single nucleotide polymorphism (SNP) in accordance with the present invention refers to a DNA sequence variation occurring when a single nucleotide - A, T, C, or G - in the genome differs between paired chromosomes in an individual or between members of a species. Almost all common SNPs have only two alleles. For a variation to be considered a SNP, it must occur in at least 1% of the population. Within a population, SNPs can be assigned a minor allele frequency, which is the lowest allele frequency at a locus that is observed in a particular population. Single nucleotide polymorphisms may fall within coding sequences of genes, non-coding regions of genes, or in the intergenic regions between genes. SNPs within a coding sequence will not necessarily change the amino acid sequence of the protein that is produced, due to degeneracy of the genetic code. A SNP in which both forms lead to the same polypeptide sequence is termed *synonymous* or a silent mutation. If a different polypeptide sequence is produced they are *non-synonymous.* SNPs that are not in protein-coding regions nonetheless can affect gene splicing, transcription factor binding, or the sequence of non-coding RNA.

The term "variable number of tandem repeats (VNTR)" refers to short nucleotide sequences generally ranging from 14 to 100 nucleotides organised into clusters of tandem repeats that are usually repeated in the range of between 4 and 40 times per occurrence. Clusters of such repeats are scattered on chromosomes and each variant constitutes an allele.

The term "microsatellites" or "short tandem repeats" (STR) relates to polymorphic loci present in nuclear and organellar DNA consisting typically of repeating units of 1 to 10 base pairs in length. The repeated sequences are directly adjacent to each other and in the same orientation. Microsatellites and STRs are characterised by a high allelic variability due to an increased rate of mutation compared to other neutral regions of DNA.

Using a genome-wide association study (GWAS) in 296 individuals with androgenetic alopecia (male pattern baldness) and 347 controls, the genetic basis of androgenetic alopecia was investigated in a genome-wide context. The chromosome 20p11 locus was found to have a strong effect on the development of early onset androgenetic alopecia. Analysis of two of the associated SNPs on chromosome 20p11 (rs2180439 and rs6113491) for interaction with the primary associated SNP at the *AR* locus (rs1041668) did not provide evidence for an interaction with the *AR* locus (see Example 5). The chromosome 20p11 locus, therefore, shows no evident connection to the androgen pathway. Thus, the present inventors identified a chromosomal region associated with androgenetic alopecia. Within this region, 52 SNPs have been shown to be associated with androgenetic alopecia (P < 0.05). These SNPs are located at position 201 of any one of SEQ ID NOs: 1, 7, 23, 27, 30, 36, 39, 41, 42, 43, 51, 53, 60, 61, 62, 74, 77, 86, 92, 103, 104, 105, 106, 111, 116, 117, 118, 121, 132, 136, 141, 145, 153, 156, 157, 166, 175, 183, 198, 200, 208, 215, 255, 257, 258, 259, 270, 278, 286, 289, 290, and 292. The human genome has been analyzed in detail by the Human HapMap Project and the correlation between the alleles of two SNPs (at high correlation two SNPs are in linkage disequilibrium) in a genomic region is well known and the information is publicly available at www.hapmap.org. By combining the association data with the publicly available linkage disequilibrium data the inventors defined 240 additionally SNPs which are in linkage disequilibrium with at least one of the 52 associated SNPs. The risk alleles of the 240 SNPs are clearly defined by the risk alleles of the 52 associated SNPs and can therefore be used as a substitute of these. Any of the genetic markers within the chromosomal region can thus be used by the skilled person to determine the presence or predisposition for androgenetic alopecia.

In a preferred embodiment of the genetic marker of the invention, the chromosomal region of human chromosome 20p11 consists of the region having the sequence of SEQ ID NO:1 at the 5'end and the sequence of SEQ ID NO:292 at the 3' end.

Thus, the chromosomal region is enclosed between the sequence as defined by SEQ ID NO:1 at the 5' end and the sequence as defined by SEQ ID NO:292 at the 3' end. In other terms, the chromosomal region spans from position chr20:21692914 to position chr20:22200475 (the genomic positions are based on NCBI build 36).

In a more preferred embodiment, the chromosomal region of human chromosome 20p11 comprises at least one of SEQ ID NOs:1 to 292.

In a further preferred embodiment, the genetic marker is a SNP selected from the group consisting of the SNPs comprised in any one of SEQ ID NOs:1 to 292.

SEQ ID NOs:1 to 292 and the respective SNPs have been summarised in table 1. Table 1 further details the position of the respective SNPs in the chromosomal region 20p11 and the corresponding alleles.

**Table 1: SNPs associated with androgenetic alopecia on chromosome 20p11**

| SEQ ID NO: | Name of SNP | Position of SNP | Position of sequence | Risk allele | Non-risk allele |
|---|---|---|---|---|---|
| 1 | rs2424407 | chr20:21693114 | chr20:21692914-21693314 | A | G |
| 2 | rs6035945 | chr20:21702301 | chr20:21702101-21702501 | A | G |
| 3 | rs6113363 | chr20:21705933 | chr20:21705733-21706133 | A | C |
| 4 | rs2208309 | chr20:21719761 | chr20:21719561-21719961 | A | G |
| 5 | rs12625990 | chr20:21726684 | chr20:21726484-21726884 | A | G |
| 6 | rs6035955 | chr20:21728253 | chr20:21728053-21728453 | A | T |
| 7 | rs6047633 | chr20:21729363 | chr20:21729163-21729563 | A | C |
| 8 | rs6082514 | chr20:21730156 | chr20:21729956-21730356 | A | G |
| 9 | rs6047636 | chr20:21732876 | chr20:21732676-21733076 | A | G |
| 10 | rs6047645 | chr20:21748445 | chr20:21748245-21748645 | A | G |
| 11 | rs6035959 | chr20:21749469 | chr20:21749269-21749669 | A | G |
| 12 | rs6082519 | chr20:21749501 | chr20:21749301-21749701 | A | T |
| 13 | rs2224367 | chr20:21749974 | chr20:21749774-21750174 | A | G |
| 14 | rs4281974 | chr20:21751581 | chr20:21751381-21751781 | A | G |
| 15 | rs6047649 | chr20:21755292 | chr20:21755092-21755492 | A | T |
| 16 | rs1535199 | chr20:21766308 | chr20:21766108-21766508 | A | G |
| 17 | rs4405823 | chr20:21772164 | chr20:21771964-21772364 | A | T |
| 18 | rs12479719 | chr20:21798786 | chr20:21798586-21798986 | A | G |
| 19 | rs997078 | chr20:21798850 | chr20:21798650-21799050 | A | T |
| 20 | rs6047684 | chr20:21801764 | chr20:21801564-21801964 | A | G |
| 21 | rs6075854 | chr20:21814377 | chr20:21814177-21814577 | A | G |
| 22 | rs4815081 | chr20:21825020 | chr20:21824820-21825220 | A | G |
| 23 | rs6137473 | chr20:21832693 | chr20:21832493-21832893 | A | G |
| 24 | rs6113418 | chr20:21837318 | chr20:21837118-21837518 | A | T |
| 25 | rs1884593 | chr20:21838817 | chr20:21838617-21839017 | A | C |
| 26 | rs6082558 | chr20:21839163 | chr20:21838963-21839363 | A | G |
| 27 | rs6047731 | chr20:21847141 | chr20:21846941-21847341 | A | G |
| 28 | rs6082560 | chr20:21850100 | chr20:21849900-21850300 | A | G |
| 29 | rs6515191 | chr20:21853732 | chr20:21853532-21853932 | A | T |
| 30 | rs6113424 | chr20:21854780 | chr20:21854580-21854980 | A | G |
| 31 | rs6113425 | chr20:21855562 | chr20:21855362-21855762 | A | G |
| 32 | rs2328682 | chr20:21866288 | chr20:21866088-21866488 | A | G |
| 33 | rs2208050 | chr20:21866427 | chr20:21866227-21866627 | A | G |
| 34 | rs6047745 | chr20:21867054 | chr20:21866854-21867254 | A | C |
| 35 | rs2424410 | chr20:21877057 | chr20:21876857-21877257 | A | C |
| 36 | rs6035995 | chr20:21879683 | chr20:21879483-21879883 | A | G |
| 37 | rs1080275 | chr20:21884485 | chr20:21884285-21884685 | A | G |
| 38 | rs6047761 | chr20:21886282 | chr20:21886082-21886482 | A | C |
| 39 | rs4815086 | chr20:21886530 | chr20:21886330-21886730 | A | C |
| 40 | rs969921 | chr20:21895184 | chr20:21894984-21895384 | A | T |
| 41 | rs2207878 | chr20:21899847 | chr20:21899647-21900047 | A | G |
| 42 | rs6047768 | chr20:21901649 | chr20:21901449-21901849 | A | G |
| 43 | rs1555264 | chr20:21909410 | chr20:21909210-21909610 | A | G |
| 44 | rs2424427 | chr20:21931464 | chr20:21931264-21931664 | A | G |
| 45 | rs1980551 | chr20:21933781 | chr20:21933581-21933981 | A | G |
| 46 | rs201587 | chr20:21975674 | chr20:21975474-21975874 | A | G |
| 47 | rs201593 | chr20:21981819 | chr20:21981619-21982019 | A | G |
| 48 | rs913063 | chr20:21990418 | chr20:21990218-21990618 | A | C |
| 49 | rs1160312 | chr20:21998503 | chr20:21998303-21998703 | A | G |
| 50 | rs1887056 | chr20:21999811 | chr20:21999611-22000011 | A | C |
| 51 | rs6113491 | chr20:22005415 | chr20:22005215-22005615 | A | C |
| 52 | rs6113495 | chr20:22009487 | chr20:22009287-22009687 | A | G |
| 53 | rs11698943 | chr20:22013466 | chr20:22013266-22013666 | A | G |
| 54 | rs6036037 | chr20:22018525 | chr20:22018325-22018725 | A | C |
| 55 | rs6113508 | chr20:22024189 | chr20:22023989-22024389 | A | G |
| 56 | rs6036041 | chr20:22030448 | chr20:22030248-22030648 | A | G |
| 57 | rs201146 | chr20:22036650 | chr20:22036450-22036850 | A | C |
| 58 | rs201149 | chr20:22038387 | chr20:22038187-22038587 | A | T |
| 59 | rs201150 | chr20:22038581 | chr20:22038381-22038781 | A | T |
| 60 | rs6137547 | chr20:22057895 | chr20:22057695-22058095 | A | G |
| 61 | rs804531 | chr20:22076554 | chr20:22076354-22076754 | A | G |
| 62 | rs6113543 | chr20:22077323 | chr20:22077123-22077523 | A | G |
| 63 | rs804533 | chr20:22077932 | chr20:22077732-22078132 | A | G |
| 64 | rs6137561 | chr20:22092267 | chr20:22092067-22092467 | A | G |
| 65 | rs721353 | chr20:22096401 | chr20:22096201-22096601 | A | G |
| 66 | rs804571 | chr20:22100012 | chr20:22099812-22100212 | A | G |
| 67 | rs804580 | chr20:22108981 | chr20:22108781-22109181 | A | C |
| 68 | rs6137580 | chr20:22135693 | chr20:22135493-22135893 | A | G |
| 69 | rs804600 | chr20:22137917 | chr20:22137717-22138117 | A | G |
| 70 | rs6132492 | chr20:22141192 | chr20:22140992-22141392 | A | G |
| 71 | rs6137582 | chr20:22145211 | chr20:22145011-22145411 | A | G |
| 72 | rs804618 | chr20:22152465 | chr20:22152265-22152665 | A | G |
| 73 | rs1090293 | chr20:22155156 | chr20:22154956-22155356 | A | G |
| 74 | rs6106481 | chr20:22171640 | chr20:22171440-22171840 | A | G |
| 75 | rs6113592 | chr20:22177505 | chr20:22177305-22177705 | A | G |
| 76 | rs6047978 | chr20:22187448 | chr20:22187248-22187648 | A | C |
| 77 | rs804688 | chr20:22190336 | chr20:22190136-22190536 | A | G |
| 78 | rs6137432 | chr20:21701799 | chr20:21701599-21701999 | G | A |
| 79 | rs2328672 | chr20:21718398 | chr20:21718198-21718598 | G | A |
| 80 | rs1476396 | chr20:21718657 | chr20:21718457-21718857 | G | C |
| 81 | rs2208310 | chr20:21719845 | chr20:21719645-21720045 | G | T |
| 82 | rs2208311 | chr20:21721249 | chr20:21721049-21721449 | G | T |
| 83 | rs1476398 | chr20:21723783 | chr20:21723583-21723983 | G | A |
| 84 | rs2208313 | chr20:21724826 | chr20:21724626-21725026 | G | T |
| 85 | rs11698973 | chr20:21724952 | chr20:21724752-21725152 | G | T |
| 86 | rs2876622 | chr20:21725915 | chr20:21725715-21726115 | G | A |
| 87 | rs6035954 | chr20:21726074 | chr20:21725874-21726274 | G | A |
| 88 | rs6047635 | chr20:21729625 | chr20:21729425-21729825 | G | T |
| 89 | rs1540930 | chr20:21736175 | chr20:21735975-21736375 | G | C |
| 90 | rs6137445 | chr20:21737803 | chr20:21737603-21738003 | G | A |
| 91 | rs6137446 | chr20:21738861 | chr20:21738661-21739061 | G | A |
| 92 | rs6047641 | chr20:21739854 | chr20:21739654-21740054 | G | T |
| 93 | rs1540932 | chr20:21745520 | chr20:21745320-21745720 | G | A |
| 94 | rs2328640 | chr20:21751728 | chr20:21751528-21751928 | G | A |
| 95 | rs17738464 | chr20:21753589 | chr20:21753389-21753789 | G | C |
| 96 | rs2224260 | chr20:21756259 | chr20:21756059-21756459 | G | A |
| 97 | rs6113394 | chr20:21769149 | chr20:21768949-21769349 | G | T |
| 98 | rs6113395 | chr20:21769262 | chr20:21769062-21769462 | G | A |
| 99 | rs8122229 | chr20:21772282 | chr20:21772082-21772482 | G | A |
| 100 | rs2328647 | chr20:21778507 | chr20:21778307-21778707 | G | A |
| 101 | rs2064773 | chr20:21781737 | chr20:21781537-21781937 | G | A |
| 102 | rs1014883 | chr20:21792630 | chr20:21792430-21792830 | G | A |
| 103 | rs4815080 | chr20:21811397 | chr20:21811197-21811597 | G | A |
| 104 | rs6075852 | chr20:21814151 | chr20:21813951-21814351 | G | A |
| 105 | rs1998076 | chr20:21828045 | chr20:21827845-21828245 | G | A |
| 106 | rs6137476 | chr20:21845656 | chr20:21845456-21845856 | G | A |
| 107 | rs2208054 | chr20:21847708 | chr20:21847508-21847908 | G | A |
| 108 | rs2208055 | chr20:21847864 | chr20:21847664-21848064 | G | A |
| 109 | rs4390829 | chr20:21848117 | chr20:21847917-21848317 | G | A |
| 110 | rs2281526 | chr20:21856332 | chr20:21856132-21856532 | G | C |
| 111 | rs2024885 | chr20:21865393 | chr20:21865193-21865593 | G | A |
| 112 | rs2208051 | chr20:21866471 | chr20:21866271-21866671 | G | A |
| 113 | rs2328684 | chr20:21866642 | chr20:21866442-21866842 | G | T |
| 114 | rs6113435 | chr20:21868688 | chr20:21868488-21868888 | G | C |
| 115 | rs4815087 | chr20:21886603 | chr20:21886403-21886803 | G | C |
| 116 | rs201543 | chr20:21894928 | chr20:21894728-21895128 | G | A |
| 117 | rs6106434 | chr20:21897593 | chr20:21897393-21897793 | G | A |
| 118 | rs6047769 | chr20:21906049 | chr20:21905849-21906249 | G | A |
| 119 | rs6036003 | chr20:21909964 | chr20:21909764-21910164 | G | A |
| 120 | rs2424425 | chr20:21927109 | chr20:21926909-21927309 | G | A |
| 121 | rs6047798 | chr20:21933498 | chr20:21933298-21933698 | G | A |
| 122 | rs127747 | chr20:21936830 | chr20:21936630-21937030 | G | A |
| 123 | rs201564 | chr20:21955602 | chr20:21955402-21955802 | G | A |
| 124 | rs4815090 | chr20:21983416 | chr20:21983216-21983616 | G | T |
| 125 | rs6113483 | chr20:21984524 | chr20:21984324-21984724 | G | C |
| 126 | rs4813445 | chr20:21995177 | chr20:21994977-21995377 | G | A |
| 127 | rs6113496 | chr20:22009632 | chr20:22009432-22009832 | G | T |
| 128 | rs6113501 | chr20:22012973 | chr20:22012773-22013173 | G | A |
| 129 | rs6113509 | chr20:22025352 | chr20:22025152-22025552 | G | A |
| 130 | rs1883751 | chr20:22028540 | chr20:22028340-22028740 | G | A |
| 131 | rs201145 | chr20:22035670 | chr20:22035470-22035870 | G | A |
| 132 | rs6047917 | chr20:22052421 | chr20:22052221-22052621 | G | A |
| 133 | rs11698068 | chr20:22054230 | chr20:22054030-22054430 | G | A |
| 134 | rs804516 | chr20:22063729 | chr20:22063529-22063929 | G | A |
| 135 | rs804520 | chr20:22067141 | chr20:22066941-22067341 | G | A |
| 136 | rs973106 | chr20:22067832 | chr20:22067632-22068032 | G | A |
| 137 | rs6113539 | chr20:22071129 | chr20:22070929-22071329 | G | A |
| 138 | rs6047929 | chr20:22084786 | chr20:22084586-22084986 | G | A |
| 139 | rs804573 | chr20:22100230 | chr20:22100030-22100430 | G | A |
| 140 | rs6137568 | chr20:22111056 | chr20:22110856-22111256 | G | A |
| 141 | rs804596 | chr20:22134593 | chr20:22134393-22134793 | G | A |
| 142 | rs804598 | chr20:22136311 | chr20:22136111-22136511 | G | A |
| 143 | rs12329414 | chr20:22139825 | chr20:22139625-22140025 | G | T |
| 144 | rs961157 | chr20:22141523 | chr20:22141323-22141723 | G | A |
| 145 | rs804610 | chr20:22142770 | chr20:22142570-22142970 | G | A |
| 146 | rs804614 | chr20:22148414 | chr20:22148214-22148614 | G | A |
| 147 | rs709007 | chr20:22151787 | chr20:22151587-22151987 | G | A |
| 148 | rs4815098 | chr20:22157538 | chr20:22157338-22157738 | G | A |
| 149 | rs1337907 | chr20:22162901 | chr20:22162701-22163101 | G | A |
| 150 | rs6137433 | chr20:21703750 | chr20:21703550-21703950 | T | C |
| 151 | rs6035946 | chr20:21706674 | chr20:21706474-21706874 | T | A |
| 152 | rs975165 | chr20:21712736 | chr20:21712536-21712936 | T | C |
| 153 | rs2208308 | chr20:21718908 | chr20:21718708-21719108 | T | G |
| 154 | rs6047632 | chr20:21728197 | chr20:21727997-21728397 | T | C |
| 155 | rs11906825 | chr20:21732105 | chr20:21731905-21732305 | T | A |
| 156 | rs6047637 | chr20:21733073 | chr20:21732873-21733273 | T | C |
| 157 | rs6137444 | chr20:21733639 | chr20:21733439-21733839 | T | C |
| 158 | rs6075840 | chr20:21736459 | chr20:21736259-21736659 | T | A |
| 159 | rs6137448 | chr20:21739008 | chr20:21738808-21739208 | T | A |
| 160 | rs6047643 | chr20:21741400 | chr20:21741200-21741600 | T | A |
| 161 | rs2180700 | chr20:21744855 | chr20:21744655-21745055 | T | G |
| 162 | rs2328673 | chr20:21747692 | chr20:21747492-21747892 | T | A |
| 163 | rs6047646 | chr20:21748510 | chr20:21748310-21748710 | T | G |
| 164 | rs6035957 | chr20:21748585 | chr20:21748385-21748785 | T | C |
| 165 | rs16983003 | chr20:21751314 | chr20:21751114-21751514 | T | A |
| 166 | rs17665823 | chr20:21752341 | chr20:21752141-21752541 | T | C |
| 167 | rs12624315 | chr20:21753128 | chr20:21752928-21753328 | T | G |
| 168 | rs6082520 | chr20:21760118 | chr20:21759918-21760318 | T | C |
| 169 | rs6113393 | chr20:21763192 | chr20:21762992-21763392 | T | C |
| 170 | rs6106421 | chr20:21769217 | chr20:21769017-21769417 | T | C |
| 171 | rs6047663 | chr20:21769910 | chr20:21769710-21770110 | T | C |
| 172 | rs6047666 | chr20:21774762 | chr20:21774562-21774962 | T | C |
| 173 | rs6047676 | chr20:21790889 | chr20:21790689-21791089 | T | C |
| 174 | rs1014885 | chr20:21792827 | chr20:21792627-21793027 | T | C |
| 175 | rs2180439 | chr20:21801100 | chr20:21800900-21801300 | T | C |
| 176 | rs6047685 | chr20:21803904 | chr20:21803704-21804104 | T | A |
| 177 | rs6035971 | chr20:21804148 | chr20:21803948-21804348 | T | A |
| 178 | rs6035979 | chr20:21823172 | chr20:21822972-21823372 | T | G |
| 179 | rs4544515 | chr20:21833311 | chr20:21833111-21833511 | T | C |
| 180 | rs1884588 | chr20:21833500 | chr20:21833300-21833700 | T | C |
| 181 | rs6113416 | chr20:21834362 | chr20:21834162-21834562 | T | C |
| 182 | rs6047715 | chr20:21835266 | chr20:21835066-21835466 | T | A |
| 183 | rs1884592 | chr20:21838690 | chr20:21838490-21838890 | T | C |
| 184 | rs6113431 | chr20:21864356 | chr20:21864156-21864556 | T | A |
| 185 | rs2328680 | chr20:21866106 | chr20:21865906-21866306 | T | C |
| 186 | rs1007169 | chr20:21866310 | chr20:21866110-21866510 | T | G |
| 187 | rs6047744 | chr20:21866898 | chr20:21866698-21867098 | T | G |
| 188 | rs2009652 | chr20:21920581 | chr20:21920381-21920781 | T | C |
| 189 | rs6036007 | chr20:21922590 | chr20:21922390-21922790 | T | C |
| 190 | rs201548 | chr20:21922835 | chr20:21922635-21923035 | T | C |
| 191 | rs6106438 | chr20:21923026 | chr20:21922826-21923226 | T | C |
| 192 | rs6113456 | chr20:21925094 | chr20:21924894-21925294 | T | G |
| 193 | rs2424426 | chr20:21928725 | chr20:21928525-21928925 | T | C |
| 194 | rs201562 | chr20:21948174 | chr20:21947974-21948374 | T | G |
| 195 | rs201563 | chr20:21948281 | chr20:21948081-21948481 | T | C |
| 196 | rs201565 | chr20:21957335 | chr20:21957135-21957535 | T | C |
| 197 | rs201569 | chr20:21960862 | chr20:21960662-21961062 | T | C |
| 198 | rs201571 | chr20:21961514 | chr20:21961314-21961714 | T | C |
| 199 | rs201572 | chr20:21962754 | chr20:21962554-21962954 | T | C |
| 200 | rs201594 | chr20:21982671 | chr20:21982471-21982871 | T | C |
| 201 | rs6047844 | chr20:21985575 | chr20:21985375-21985775 | T | C |
| 202 | rs6036026 | chr20:21992205 | chr20:21992005-21992405 | T | C |
| 203 | rs4813446 | chr20:21997759 | chr20:21997559-21997959 | T | C |
| 204 | rs6113524 | chr20:22042192 | chr20:22041992-22042392 | T | A |
| 205 | rs1303872 | chr20:22059034 | chr20:22058834-22059234 | T | C |
| 206 | rs6113548 | chr20:22084839 | chr20:22084639-22085039 | T | C |
| 207 | rs6113551 | chr20:22087934 | chr20:22087734-22088134 | T | A |
| 208 | rs6137567 | chr20:22110856 | chr20:22110656-22111056 | T | C |
| 209 | rs767457 | chr20:22133332 | chr20:22133132-22133532 | T | C |
| 210 | rs804609 | chr20:22142475 | chr20:22142275-22142675 | T | C |
| 211 | rs6113583 | chr20:22143065 | chr20:22142865-22143265 | T | C |
| 212 | rs2876633 | chr20:22145686 | chr20:22145486-22145886 | T | A |
| 213 | rs804616 | chr20:22149839 | chr20:22149639-22150039 | T | C |
| 214 | rs804617 | chr20:22151480 | chr20:22151280-22151680 | T | G |
| 215 | rs1090292 | chr20:22154969 | chr20:22154769-22155169 | T | C |
| 216 | rs1415801 | chr20:22159414 | chr20:22159214-22159614 | T | G |
| 217 | rs1337908 | chr20:22189466 | chr20:22189266-22189666 | T | A |
| 218 | rs10485630 | chr20:22193867 | chr20:22193667-22194067 | T | G |
| 219 | rs6047982 | chr20:22194450 | chr20:22194250-22194650 | T | G |
| 220 | rs6047613 | chr20:21694305 | chr20:21694105-21694505 | C | G |
| 221 | rs6082508 | chr20:21717042 | chr20:21716842-21717242 | C | A |
| 222 | rs2224366 | chr20:21719909 | chr20:21719709-21720109 | C | G |
| 223 | rs6047628 | chr20:21722338 | chr20:21722138-21722538 | C | G |
| 224 | rs6047630 | chr20:21722842 | chr20:21722642-21723042 | C | G |
| 225 | rs2208312 | chr20:21724735 | chr20:21724535-21724935 | C | T |
| 226 | rs2208314 | chr20:21724875 | chr20:21724675-21725075 | C | A |
| 227 | rs6082512 | chr20:21728905 | chr20:21728705-21729105 | C | T |
| 228 | rs6082513 | chr20:21728972 | chr20:21728772-21729172 | C | A |
| 229 | rs6113378 | chr20:21729032 | chr20:21728832-21729232 | C | T |
| 230 | rs873137 | chr20:21732131 | chr20:21731931-21732331 | C | A |
| 231 | rs6113381 | chr20:21732950 | chr20:21732750-21733150 | C | T |
| 232 | rs6113382 | chr20:21735568 | chr20:21735368-21735768 | C | A |
| 233 | rs4815066 | chr20:21738075 | chr20:21737875-21738275 | C | A |
| 234 | rs16982990 | chr20:21738982 | chr20:21738782-21739182 | C | T |
| 235 | rs1540931 | chr20:21745349 | chr20:21745149-21745549 | C | . A |
| 236 | rs2328642 | chr20:21754161 | chr20:21753961-21754361 | C | G |
| 237 | rs11696327 | chr20:21757409 | chr20:21757209-21757609 | C | T |
| 238 | rs2224261 | chr20:21762035 | chr20:21761835-21762235 | C | T |
| 239 | rs6047657 | chr20:21762130 | chr20:21761930-21762330 | C | A |
| 240 | rs1014884 | chr20:21792694 | chr20:21792494-21792894 | C | T |
| 241 | rs6047677 | chr20:21795479 | chr20:21795279-21795679 | C | G |
| 242 | rs987050 | chr20:21800049 | chr20:21799849-21800249 | C | A |
| 243 | rs6047683 | chr20:21801193 | chr20:21800993-21801393 | C | A |
| 244 | rs1012184 | chr20:21804914 | chr20:21804714-21805114 | C | T |
| 245 | rs6047688 | chr20:21807207 | chr20:21807007-21807407 | C | G |
| 246 | rs4815077 | chr20:21807643 | chr20:21807443-21807843 | C | T |
| 247 | rs6113404 | chr20:21811825 | chr20:21811625-21812025 | C | T |
| 248 | rs6075855 | chr20:21814547 | chr20:21814347-21814747 | C | T |
| 249 | rs6035978 | chr20:21823139 | chr20:21822939-21823339 | C | T |
| 250 | rs6047705 | chr20:21823655 | chr20:21823455-21823855 | C | T |
| 251 | rs1884589 | chr20:21833619 | chr20:21833419-21833819 | C | A |
| 252 | rs6047732 | chr20:21847248 | chr20:21847048-21847448 | C | G |
| 253 | rs3818179 | chr20:21849123 | chr20:21848923-21849323 | C | A |
| 254 | rs3818182 | chr20:21849398 | chr20:21849198-21849598 | C | A |
| 255 | rs927059 | chr20:21862194 | chr20:21861994-21862394 | C | T |
| 256 | rs2328681 | chr20:21866213 | chr20:21866013-21866413 | C | T |
| 257 | rs2328683 | chr20:21866447 | chr20:21866247-21866647 | C | T |
| 258 | rs4813442 | chr20:21887656 | chr20:21887456-21887856 | C | T |
| 259 | rs970616 | chr20:21900054 | chr20:21899854-21900254 | C | A |
| 260 | rs1555265 | chr20:21909472 | chr20:21909272-21909672 | C | T |
| 261 | rs169311 | chr20:21910333 | chr20:21910133-21910533 | C | A |
| 262 | rs6047790 | chr20:21926495 | chr20:21926295-21926695 | C | T |
| 263 | rs1980552 | chr20:21934005 | chr20:21933805-21934205 | C | T |
| 264 | rs6047799 | chr20:21934312 | chr20:21934112-21934512 | C | A |
| 265 | rs201559 | chr20:21945115 | chr20:21944915-21945315 | C | T |
| 266 | rs201561 | chr20:21947213 | chr20:21947013-21947413 | C | G |
| 267 | rs201574 | chr20:21963564 | chr20:21963364-21963764 | C | T |
| 268 | rs6137526 | chr20:21986726 | chr20:21986526-21986926 | C | T |
| 269 | rs6132477 | chr20:21988422 | chr20:21988222-21988622 | C | T |
| 270 | rs6137527 | chr20:21991921 | chr20:21991721-21992121 | C | T |
| 271 | rs6036029 | chr20:21997177 | chr20:21996977-21997377 | C | T |
| 272 | rs6137533 | chr20:22018663 | chr20:22018463-22018863 | C | T |
| 273 | rs4815092 | chr20:22029333 | chr20:22029133-22029533 | C | G |
| 274 | rs6113534 | chr20:22065778 | chr20:22065578-22065978 | C | G |
| 275 | rs708996 | chr20:22070158 | chr20:22069958-22070358 | C | G |
| 276 | rs804524 | chr20:22070810 | chr20:22070610-22071010 | C | T |
| 277 | rs1415798 | chr20:22078529 | chr20:22078329-22078729 | C | G |
| 278 | rs1415799 | chr20:22079194 | chr20:22078994-22079394 | C | T |
| 279 | rs2328701 | chr20:22084001 | chr20:22083801-22084201 | C | G |
| 280 | rs804553 | chr20:22092775 | chr20:22092575-22092975 | C | T |
| 281 | rs6106474 | chr20:22136798 | chr20:22136598-22136998 | C | T |
| 282 | rs804605 | chr20:22140057 | chr20:22139857-22140257 | C | T |
| 283 | rs804606 | chr20:22140446 | chr20:22140246-22140646 | C | T |
| 284 | rs6082642 | chr20:22144420 | chr20:22144220-22144620 | C | G |
| 285 | rs742655 | chr20:22147503 | chr20:22147303-22147703 | C | T |
| 286 | rs804615 | chr20:22149175 | chr20:22148975-22149375 | C | T |
| 287 | rs709006 | chr20:22151740 | chr20:22151540-22151940 | C | T |
| 288 | rs12625314 | chr20:22160946 | chr20:22160746-22161146 | C | T |
| 289 | rs12625315 | chr20:22160962 | chr20:22160762-22161162 | C | T |
| 290 | rs1337906 | chr20:22162821 | chr20:22162621-22163021 | C | T |
| 291 | rs804684 | chr20:22182736 | chr20:22182536-22182936 | C | T |
| 292 | rs6113604 | chr20:22200275 | chr20:22200075-22200475 | C | T |

Further, the invention relates to a nucleic acid molecule selected from the group consisting of (a) a nucleic acid molecule consisting of at least 17 consecutive nucleotides of any of SEQ ID NOs:1 to 292; wherein the at least 17 consecutive nucleotides contain an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292; (b) a nucleic acid molecule the complementary strand of which hybridises under stringent conditions to the nucleic acid molecule of (a), wherein said nucleic acid molecule has at a position corresponding to position 201 of any one of SEQ ID NOs:1 to 77 an adenosine, corresponding to position 201 of any one of SEQ ID NOs:78 to 149 a guanosine, corresponding to position 201 of any one of SEQ ID NOs:150 to 219 a thymidine or corresponding to position 201 of any one of SEQ ID NOs:220 to 292 a cytidine; (c) a nucleic acid molecule identical to the nucleic acid molecule of (a) or (b) wherein each thymidine is replaced by uridine.

In this embodiment, the nucleic acid molecules contain the respective risk alleles associated with the presence or predisposition for androgenetic alopecia.

Further, the invention relates to a nucleic acid molecule selected from the group consisting of (a) a nucleic acid molecule consisting of at least 17 consecutive nucleotides of any of SEQ ID NOs:1 to 292; wherein the at least 17 consecutive nucleotides contain an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; (b) a nucleic acid molecule the complementary strand of which hybridises under stringent conditions to the nucleic acid molecule of (a), wherein said nucleic acid molecule has at a position corresponding to position 201 of any one of SEQ ID NOs: SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264 an adenosine, corresponding to position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215 a cytidine, corresponding to position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 a guanosine or corresponding to position 201 of any one of SEQ ID NOs: 6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292 a thymidine; and (c) a nucleic acid molecule identical to any one of the nucleic acid molecules of (a) or (b) wherein each thymidine is replaced by uridine.

In this embodiment, the nucleic acid molecules contain the respective non-risk alleles associated with the absence of androgenetic alopecia or the absence of a predisposition for androgenetic alopecia.

The nucleic acid molecules of the present invention consist most preferably of at least 17 nucleotides, but may also consist of at least 19, 25, 50, 100, 150, 200 or more nucleotides. The present invention encompasses a large number of nucleic acid molecules, which all contain as a common denominator the nucleotide at position 201 of any one of SEQ ID NOs:1 to 292. For example, the 17-mer of the invention may contain as a common denominator the nucleotide at position 201 of any one of SEQ ID NOs:1 to 292, wherein the said nucleotide at position 201 may be located within the 17-mer at position 1, 2, 3, 4, 5, 6, 7, 8, 9, 10, 11, 12, 13, 14, 15, 16 or 17 of the 17-mer if viewed from its 5'end.

The nucleic acid molecules of the present invention are characterized as being associated with androgenetic alopecia. The sequences of any one of SEQ ID NOs:1 to 292 were uniformly chosen to contain the 200bp upstream and the 200bp downstream of the position of the respective risk allele or non-risk allele at the genetic locus, hence said alleles are uniformly located at position 201 of any of SEQ ID NOs:1 to 292.

The term "risk allele" in accordance with the present invention refers to the alleles with a higher frequency in the affected group than in the control group, whereas the term "non-risk allele" refers to the alleles with higher frequency in the non affected control groups. Both the risk alleles as well as the non-risk alleles can be unambiguously identified with information of position and the sequences contained in the SEQ ID NOs:1 to 292 as provided in the present application. Furthermore, table 1 details the individual risk alleles and non-risk alleles in the corresponding SEQ ID NOs as well as their position in the chromosomal region 20p11.

Performing a genome-wide association analysis the present inventors found that 52 SNPs at the chromosome 20p11 locus showed association with androgenetic alopecia (P < 0.05). These SNPs are located at position 201 of any one of SEQ ID NOs:1, 7, 23, 27, 30, 36, 39, 41, 42, 43, 51, 53, 60, 61, 62, 74, 77, 86, 92, 103, 104, 105, 106, 111, 116, 117, 118, 121, 132, 136, 141, 145, 153, 156, 157, 166, 175, 183, 198, 200, 208, 215, 255, 257, 258, 259, 270, 278, 286, 289, 290, and 292. The human genome has been analyzed in detail by the Human HapMap Project and the linkage disequilibrium between two SNPs in a genomic region is well known and the information is publicly available at www.hapmap.org. By combining the association data with the publicly available linkage disequilibrium data the inventors defined 240 additionally SNPs which are in linkage disequilibrium with at least one of the 52 associated SNPs. The risk alleles of the 240 SNPs are clearly defined by the risk alleles of the 52 associated SNPs and can therefore be used as a substitute of these. Any of these 292 genetic markers within the chromosomal region can thus be used by the skilled person to determine the presence or predisposition for androgenetic alopecia.

The term "hybridises/hybridising" as used herein refers to a pairing of a nucleic acid molecule to a (partially) complementary strand of this nucleic acid molecule which thereby form a hybrid.

It is well known in the art how to perform hybridisation experiments with nucleic acid molecules, i.e. the person skilled in the art knows what hybridisation conditions s/he has to use in accordance with the present invention. Such hybridisation conditions are referred to in standard text books such as Molecular Cloning A Laboratory Manual, Cold Spring Harbor Laboratory (1989) N.Y. Preferred in accordance with the present invention are nucleic acid molecules capable of hybridising under stringent hybridisation conditions to the nucleic acid molecules of the invention that are associated with androgenetic alopecia, i.e. that do not cross hybridise to unrelated nucleic acid molecules.
Nucleic acid hybridisation will be affected by such conditions as salt concentration, temperature, or organic solvents, in addition to the base composition, length of the complementary strands and the number of nucleotide base mismatches between the hybridising nucleic acids, as will be readily appreciated by those skilled in the art. Stringent temperature conditions will generally include temperatures in excess of 30°C, typically 37°C, and preferably in excess of 45°C. Stringent salt conditions will ordinarily be less than 1000 mM, typically less than 500 mM and preferably less than 200 mM. However, the combination of parameters is much more important than the measure of any single parameter; see, e.g., Wetmur and Davidson, 1968. Probe sequences may also hybridise specifically to duplex DNA under certain conditions to form triplex or higher order DNA complexes. The preparation of such probes and suitable hybridisation conditions are well known in the art. Nucleic acid molecules capable of hybridising to the nucleic acid molecules of the invention are preferably at least 70%, more preferably at least 75%, at least 80%, at least 85%, at least 90%, at least 95% or 100% identical to the nucleic acid sequences of the invention.

Generally, stringent hybridisation conditions refer to conditions which comprise, e.g. an overnight incubation at 65°C in 4x SSC (600 mM NaCl, 60 mM sodium citrate) followed by washing at 65°C in 0.1x SSC for one hour. Alternatively, hybridization conditions can comprise e.g. an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing the filters in 0.1 x SSC at about 65°C. Said conditions for hybridization are also known by a person skilled in the art as "highly stringent conditions for hybridization". Also contemplated are nucleic acid molecules that hybridize to the oligo- or polynucleotides of the invention at lower stringency hybridization conditions ("low stringency conditions for hybridization"). Changes in the stringency of hybridization and signal detection are primarily accomplished through the manipulation of formamide concentration (lower percentages of formamide result in lowered stringency), salt conditions, or temperature. For example, lower stringency conditions include an overnight incubation at 50°C in 4x SSC or an overnight incubation at 37°C in a solution comprising 6X SSPE (20X SSPE = 3M NaCl; 0.2M NaH₂PO4; 0.02M EDTA, pH 7.4), 0.5% SDS, 30% formamide, 100 µg/ml salmon sperm blocking DNA; followed by washes at 50°C with 1X SSPE, 0.1% SDS. In addition, to achieve an even lower stringency, washes performed following stringent hybridization can be done at higher salt concentrations (e.g. 5X SSC). It is of note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed). The embodiment recited herein above preferably refers to highly stringent conditions and alternatively to conditions of lower stringency.

In a preferred embodiment the genetic marker is a SNP selected from the group consisting of the SNPs defined by any one of SEQ ID NOs:1, 7, 23, 27, 30, 36, 39, 41, 42, 43, 51, 53, 60, 61, 62, 74, 77, 86, 92, 103, 104, 105, 106, 111, 116, 117, 118, 121, 132, 136, 141, 145, 153, -156, 157, 166, 175, 183, 198, 200, 208, 215, 255, 257, 258, 259, 270, 278, 286, 289, 290 or 292.

In a more preferred embodiment, the genetic marker is a SNP selected from the group consisting of the SNPs defined by any one of SEQ ID NOs: 51, 105, 157, 175 or 198.

In a preferred embodiment, the nucleic acid molecule is genomic DNA.

This preferred embodiment of the invention reflects the fact that usually the analysis would be carried out on the basis of genomic DNA from body fluid, cells or tissue isolated from the person under investigation.

The invention further relates to a nucleic acid molecule which is complementary to the nucleic acid molecule of the invention.

The term "complementary nucleic acid molecule" refers to a nucleic acid molecule the sequence of which is uniquely fitting to (e.g. hybridising to/complementary to preferably 100%) the sequence of the nucleic acid molecule of the invention. That is, the "complementary nucleic acid molecule" will be uniquely fitting to the nucleic acid molecule of the invention having the risk-allele, where said nucleic acid molecule of the invention has an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292, but will not be fitting to the sequences containing the non-risk allele as defined in SEQ ID NOs:1 to 292 at position 201. Alternatively, the "complementary nucleic acid molecule" will be uniquely fitting to the nucleic acid molecule of the invention having the non-risk allele, where said nucleic acid molecule of the invention has an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; but will not be fitting to the sequences containing the risk allele as defined in SEQ ID NOs:1 to 292 at position 201.

Complementary nucleic acid molecules are for example useful in the analysis of the genetic setup in the recited positions in hybridisation assays, such as in Southern blot analysis of DNA preparations, but also in PCRs or primer extension protocols respectively. Thus, for example, a 17mer exactly complementary either to a sequence having the non-risk allele or to a sequence having the risk allele associated with androgenetic alopecia may be used to differentiate between the polymorphic variants. This is because a nucleic acid molecule labelled with a detectable label not exactly complementary to the DNA in the analyzed sample will not give rise to a detectable signal, if appropriate hybridization and washing conditions are chosen.
In this regard, it is important to note that the nucleic acid molecule of the invention as well as the complementary nucleic acid molecule may be detectably labelled. Detectable labels include radioactive labels such as ³H, or ³²P or fluorescent labels. Labelling of nucleic acids is well understood in the art and described, for example, in Sambrook and Russel "Molecular Cloning, A Laboratory Manual", Cold Spring Harbor Laboratory, N.Y. (2001).

Furthermore, as complementary nucleic acid molecules are also associated with androgenetic alopecia, they may be directly analysed for the presence of the respective complementary nucleotide at position 201 of the nucleic acid molecule of the invention, which is indicative of the presence or predisposition for androgenetic alopecia. In other words, a thymidine at position 201 of a complementary nucleic acid molecule to any one of SEQ ID NOs:1 to 77, a cytidine at position 201 of a complementary nucleic acid molecule to any one of SEQ ID NOs:78 to 149, an adenosine at position 201 of a complementary nucleic acid molecule to any one of SEQ ID NOs:150 to 219 or a guanosine at position 201 of a complementary nucleic acid molecule to any one of SEQ ID NOs:220 to 292 would be indicative of the presence or predisposition for androgenetic alopecia.
The same applies to complementary nucleic acid molecules of the nucleic acid molecule of the invention being indicative of a reduced risk for androgenetic alopecia or the absence of androgenetic alopecia or a predisposition for this condition. Thus, a thymidine in a complementary nucleic acid molecule at a position corresponding to position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a guanosine in a complementary nucleic acid molecule at a position corresponding to position 201 of any one of SEQ ID NOs: 3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, an cytidine in a complementary nucleic acid molecule at a position corresponding to position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or an adenosine in a complementary nucleic acid molecule at a position corresponding to position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292 would be indicative for the absence of androgenetic alopecia or a predisposition therefore.

In a further embodiment, the invention also relates to a vector comprising the nucleic acid molecule of the invention as described above.

The term "vector" in accordance with the present invention refers to a vector that is a plasmid, cosmid, virus, bacteriophage or another vector conventionally used e.g. in genetic engineering. Incorporation of the nucleic acid into a vector offers the possibility of introducing the nucleic acid molecule efficiently into the cells and preferably the DNA of a recipient. The recipient may be a single cell such as cell from a cell line. Such a measure renders it possible to express, when expression vectors are chosen, the respective nucleic acid molecule in the recipient. Thus, incorporation of the nucleic acid molecule into an expression vector opens up the way to a permanently elevated level of the encoded protein in any cell or a subset of selected cells of the recipient. Furthermore, chromosomal loci may be modified when using specific targeting vectors comprising a nucleic acid molecule to be replaced for the homologous chromosomal sequence of the host cell. Typical targeting vectors contain a positive selectable marker and the replacement construct, with two regions of homology to the target sequence located on either side. When transfected into cells, a double crossover event will replace the target sequences with the replacement construct sequences.

The nucleic acid molecule may be inserted into several commercially available vectors. Non-limiting examples include vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen), pCINeo (Promega), Okayama-Berg cDNA expression vector pcDV1 (Pharmacia), pRc/CMV, pcDNA1, pSPORT1 (GIBCO BRL), pGEMHE (Promega), pSVL and pMSG (Pharmacia, Uppsala, Sweden), pRSVcat (ATCC 37152), pSV2dhfr (ATCC 37146) or pBC12MI (ATCC 67109).

The polynucleotide of the present invention referred to above may also be inserted into vectors such that a translational fusion with another polynucleotide is generated. The other polynucleotide may encode a protein which may e.g. increase the solubility and/or facilitate the purifcation of the fusion protein. Non-limiting examples include pET32, pET41, pET43. The vectors may also contain an additional expressible polynucleotide coding for one or more chaperones to facilitate correct protein folding. Suitable bacterial expression hosts comprise e. g. strains derived from BL21 (such as BL21 (DE3), BL21(DE3)PtysS, BL21(DE3)RIL, BL21 (DE3)PRARE) or Rosetta®.

For vector modification techniques, see Sambrook and Russel ("Molecular Cloning, A Laboratory Manual", Cold Spring Harbor Laboratory, N.Y. (2001)). Generally, vectors can contain one or more origin of replication (ori) and inheritance systems for cloning or expression, one or more markers for selection in the host, e. g., antibiotic resistance, and one or more expression cassettes.
The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in eukaryotic cells are well known to those skilled in the art. These elements comprise regulatory sequences ensuring the initiation of the transcription (e. g. translation initiation codon, promoters, enhancers, and/or insulators), internal ribosomal entry sites (IRES) (Owens Proc. Natl. Acad. Sci. USA 2001, 98: 1471) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule is operatively linked to such expression control sequences allowing expression in eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1a-promoter, CMV enhancer, CaM-kinase promoter, the Autographa californica multiple nuclear polyhedrosis virus (AcMNPV) polyhedral promoter or the SV40-enhancer. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide. Moreover, elements such as origin of replication, drug resistance gene, regulators (as part of an inducible promoter) may also be included. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter).

The co-transfection with a selectable marker such as dhfr, gpt, neomycin, hygromycin, ampicillin and kanamycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al., Biochem J. 1991, 227:277; Bebbington et al., Bio/Technology 1992, 10:169). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture.

Specifically-designed vectors allow the shuttling of DNA between different hosts, such as bacteria- fungal cells or bacteria-animal cells (e. g. the Gateway® system available at Invitrogen).

The nucleic acid molecules of the invention as described herein above may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as eukaryotic expression system for the nucleic acid molecules of the invention.

In a further embodiment, the present invention also relates to a host genetically engineered with the nucleic acid molecule of the invention or with the vector comprising the nucleic acid molecule of the invention as described above.

Preferably, introduction of the nucleic acid molecule of the invention or the vector comprising the nucleic acid molecule of the invention leads to integration of the nucleic acid molecule into the genome of the host. It is particularly envisaged that nucleic acid molecules of the invention may be introduced into the genome of the host by homologous recombination using techniques well known to the person skilled in the art. By integration of the nucleic acid molecules of the invention into the genome, regulatory effects of the particular SNPs, such as for example transcriptional regulation of neighbouring genes, may be investigated in suitable hosts.

The host cell may be any prokaryote or eukaryotic cell. Suitable prokaryotes/bacteria are those generally used for cloning like *E. coli* (e.g., *E coli* strains BL21(DE3), HB101, DH5α, XL1 Blue, Y1090 and JM101), *Salmonella typhimurium, Serratia marcescens, Pseudomonas putida, Pseudomonas fluorescens, Streptomyces lividans, Lactococcus lactis, Mycobacterium smegmatis* or *Bacillus subtilis.* A suitable eukaryotic host cell may be an animal cell such as a mammalian cell, an amphibian cell, a fish cell, an insect cell such as Drosophila S2 and Spodoptera Sf9 cells, a fungal cell or a plant cell. Mammalian host cells that could be used include, human Hela, HEK293, H9, Bowes melanoma cells and Jurkat cells, mouse NIH3T3 and C127 cells, Cos 1, Cos 7 and CV1, quail QC1-3 cells, mouse L cells, Chinese hamster ovary (CHO) cells or non-human embryonic stem cells. Also within the scope of the present invention are primary mammalian cells such as the immortalised keratinocyte cell line HaCaT, primary keratinocytes, primary hair folllicle cells or mouse embryonic fibroblasts (MEF). Appropriate culture mediums and conditions for the above-described host cells are well known in the art.

A method for the production of a transgenic non-human animal, for example a transgenic mouse, comprises introduction of the aforementioned nucleic acid molecule or targeting vector into a germ cell, an embryonic cell, stem cell or an egg or a cell derived thereof. The non-human animal can be used in accordance with the invention in a method for identifying potential roles of the risk alleles of the nucleic acid molecule of the invention. Production of transgenic embryos and screening of those can be performed, e.g., as described by A. L. Joyner Ed., Gene Targeting, A Practical Approach (1993), Oxford University Press. The DNA of the embryonic membranes of embryos can be analyzed using, e.g., PCR amplification and sequencing or Southern blots with an appropriate probe; see supra. A general method for making transgenic non-human animals is described in the art, see for example WO 94/24274. For making transgenic non-human organisms (which include homologously targeted non-human animals), embryonal stem cells' (ES cells) are preferred. Murine ES cells, such as AB-1 line grown on mitotically inactive SNL76/7 cell feeder layers (McMahon and Bradley, Cell 62:1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326:292-295 (1987)), the D3 line (Doetschman et al., J. Embryol. Exp. Morph. 87:27-45 (1985)), the CCE line (Robertson et al., Nature 323:445-448 (1986)), and the AK-7 line (Zhuang et al., Cell 77:875-884 (1994)). The success of generating a mouse line from ES cells bearing a specific targeted mutation depends on the pluripotence of the ES cells (i.e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant non-human females and are born, e.g. as chimeric mice. The resultant transgenic mice are backcrossed and screened for the presence of the correctly targeted transgene(s), usually by PCR or Southern blot analysis on tail biopsy DNA of offspring so as to identify transgenic mice.

The transgenic non-human animals may, for example, be transgenic mice, rats, hamsters, dogs, monkeys, rabbits, pigs, or cows. Preferably, said transgenic non-human animals are mice.

The invention further relates to a method for testing for a predisposition or presence of androgenetic alopecia comprising testing a sample obtained from a prospective patient or from a person suspected of carrying a predisposition for androgenetic alopecia, the method comprising determining (a) the presence of a risk allele of a genetic marker according to any one of claims 1 to 4; (b) the presence and quantity of a nucleic acid molecule of any one of claims 5 to 8; (c) the presence of a risk allele within the nucleic acid molecule of any one of claims 5 or 7 as compared to the non-risk allele, wherein said risk allele is characterised in that it is an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292; (d) the presence of a risk allele within the nucleic acid molecule of claim 8 as compared to the non-risk allele, wherein said risk allele is characterised in that it is a thymidine at position 201 of any one of SEQ ID NOs:1 to 77, a cytidine at position 201 of any one of SEQ ID NOs:78 to 149, an adenosine at position 201 of any one of SEQ ID NOs:150 to 219 or a guanosine at position 201 of any one of SEQ ID NOs:220 to 292; (e) the presence of a non-risk allele within the nucleic acid molecule of any one of claims 6 or 7 as compared to the risk allele, wherein said non-risk allele is characterised in that it is an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; or (f) the presence of a non-risk allele within the nucleic acid molecule of claim 8 as compared to the risk allele, wherein said non-risk allele is characterised in that it is an thymidine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a guanosine at position 201 of any one of SEQ ID NOs: 3, 7, 25, 34, 35, 38, 39, 48, 50, 51, 54, 57, 67, 76, 80, 89, 95, 110, 114, 115, 125, 150, 152, 154, 156, 157, 164, 166, 168 to 175, 179 to 181, 183, 185, 188 to 191, 193, 195 to 203, 205, 206, 208 to 211, 213 or 215, a cytidine at position 201 of any one of SEQ ID NOs: 1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a adenosine at position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; and wherein the presence of said genetic marker or said risk allele is indicative of androgenetic alopecia or a predisposition for androgenetic alopecia and wherein the presence of said non-risk allele is indicative of a reduced risk for androgenetic alopecia or the absence of androgenetic alopecia or a predisposition for androgenetic alopecia.

Due to the abundance of established methods for assessing the genotypes of SNPs, it is now possible to conveniently diagnose the presence or a genetic predisposition for androgenetic alopecia in a short amount of time, at low cost, with high accuracy and without significant trouble for the person under investigation. Detection of the non-risk allele may also conveniently be used to demonstrate that a person does not have androgenetic alopecia or a predisposition therefor.

Methods for testing a sample for the presence of the nucleic acid molecule of the invention include, but are not limited to, nucleic acid amplification and oligonucleotide extension, sequencing or hybridization assays.

Examples for nucleic acid amplification assays and means to perform such include without limitation PCR, (including nested PCR, RT-PCR, PCR extension assays, Nucleic Acid Sequence Base Amplification (NASBA), single-strand confirmation polymorphism (SSCP) PCR), amplification refractory mutation systems (ARMSTM) and amplification refractory mutation system linear extension (ALEXTM) assays. Details of such methods can be found in art, see, for example, Newton et al., Nucleic Acids Res. 17 (1989) 2503-2516; Agrawal (Ed.), "Protocols for Oligonucleotides and Analogs: Synthesis and Properties (Methods in Molecular Biology, 20)", Humana Press, 1993; Haque et al., Diagn. Mol. Pathol. 7 (1998) 248-252; Innis et al. (Ed.), "PCR Applications: Protocols for Functional Genomics", Academic Press, 1999; Chen and Janes (Ed.), "PCR Cloning Protocols: From Molecular Cloning to Genetic", 2nd edition, Humana Press, 2002; Pissard et al., Clin. Chem. 48 (2002) 769-772; Blondal et al., Nucleic Acids Res 31 (2003) e155; Steemers et al., Nature Meth. 3 (2006) 31-33; Kakavas et al., J. Clin. Lab. Anal. 20 (2006) 1-7.

Examples for sequencing assays comprise without limitation approaches of sequence analysis by direct sequencing, fluorescent SSCP in an automated DNA sequencer and Pyrosequencing. These procedures are common in the art, see e.g.
Adams et al. (Ed.), "Automated DNA Sequencing and Analysis", Academic Press, 1994; Alphey, "DNA Sequencing: From Experimental Methods to Bioinformatics", Springer Verlag Publishing, 1997; Ramon et al., J. Transl. Med. 1 (2003) 9; Meng et al., J. Clin. Endocrinol. Metab. 90 (2005) 3419-3422.

Examples for hybridization assays comprise without limitation Northern and Southern blot assays, heteroduplex analysis, detection of mutations by sequence specific oligonucleotide hybridization, allele-specific oligonucleotide hybridization on DNA chips, assays based on Illumina's^{®} technology, assays based on the BeadArray^{®} technology, see, for example, Barnes et al., Nucleic Acids Res. 33 (2005) 5914-5923; Fan et al., Biotechniques 39 (2005) 583-588; Shen et al., Mutat. Res.-Fund. Mol. M. 573 (2005) 70-82; Steemers and Gunderson, Pharmacogenomics, 6 (2005) 777-782.

In a preferred embodiment said determining of the presence of said genetic marker, said nucleic acid molecule or the presence of said risk allele comprises PCR based techniques, RFLP-or AFLP based techniques, DNA sequencing-based techniques, hybridization-based techniques, single-strand conformation polymorphism analysis (SSCA), denaturating gradient gel electrophoresis (DGGE), mismatch cleavage detection, heteroduplex analysis, primer extension-based techniques, and 5'-nuclease assay-based techniques. Said techniques are well known to the person skilled in the art.

In a preferred embodiment, the method further comprises detecting an alteration in the expression of a nucleic acid molecule encoding a polypeptide having PAX1 function, wherein said nucleic acid molecule comprises (a) a nucleic acid molecule encoding a polypeptide having the amino acid sequence of SEQ ID NO:293; (b) a nucleic acid molecule having the DNA sequence of SEQ ID NO:294; (c) a nucleic acid molecule having the sequence of SEQ ID NO:294, wherein each thymidine is replaced by uridine; wherein the alteration of expression as compared to the amount of nucleic acid molecule expression in the control sample is indicative of androgenetic alopecia or a predisposition for androgenetic alopecia.

The transcriptional activator PAX1 (paired box 1) is a member of the paired box-containing class of developmental control genes and is located in the chromosomal region 20p11.2. PAX1 is involved in the generation of the vertebral column. The term "a polypeptide having PAX1 function" in accordance with the present invention refers to a polypeptide having or essentially having PAX1 function, wherein said function can be determined e.g. by its trans-activating capacity in reporter gene assays or by its activation of NKX3-2 (BAPX1), a downstream gene of PAX1.

The expression levels of a nucleic acid molecule encoding a polypeptide having PAX1 function can be measured by any method known that can provide quantitative information regarding the levels to be measured. The methods preferably are highly sensitive and provide reproducible results. In particular, methods based upon the polymerase chain reaction (real-time PCR) and related amplification technologies, such as NASBA and other isothermal amplification technologies, may be used. A suitable approach may be, for example, real-time PCR employing the relative quantification approach using GAPDH or β-actin as housekeeping gene.

Amounts of purified protein in solution can be determined by physical methods, e.g. photometry. Methods of quantifying a particular protein in a mixture rely on specific binding, e.g of antibodies. Specific detection and quantitation methods exploiting the specificity of antibodies comprise immunohistochemistry (in situ) and surface plasmon resonance. Western blotting combines separation of a mixture of proteins by electrophoresis and specific detection with antibodies.

The present inventors found that *PAX1* is located in proximity to the locus identified to be associated with androgenetic alopecia. Furthermore, as shown in Example 6, *PAX1* was found to be expressed at considerable levels in the scalp. These expression data are compatible with a role of this gene in the development of androgenetic alopecia.

In a further preferred embodiment, the sample is blood, serum, plasma, fetal tissue, saliva, urine, mucosal tissue, mucus, vaginal tissue, fetal tissue obtained from the vagina, skin, hair, hair follicle or another human tissue.

In another embodiment, the invention relates to a diagnostic composition for the detection of androgenetic alopecia or a predisposition for androgenetic alopecia comprising the nucleic acid molecules, the vector or the host cell of the invention.

The term "diagnostic composition" relates to compositions for diagnosing a predisposition of individual patients for androgenetic alopecia. The diagnostic composition of the invention comprises at least one of the nucleic acid molecules, the vector or the host cell recited above. Said composition may further comprise appropriate buffer(s), and enzymes such as reverse transcriptase, thermostable polymerases etc. The diagnostic composition of the invention may be used to test for the presence of the nucleic acid molecule of the invention using methods well known in the art. Such methods have been described above and are also described, e. g. in Sambrook and Russel "Molecular Cloning, A Laboratory Manual", Cold Spring Harbor Laboratory, N.Y. (2001).

The invention also relates to a Kit comprising the nucleic acid molecules of the invention, the complementary nucleic acid molecules, the vector containing the nucleic acid molecule or the host of the invention.

The various components of the kit may be packaged in one or more containers such as one or more vials. The vials may, in addition to the components, comprise preservatives or buffers for storage.

The figures show:

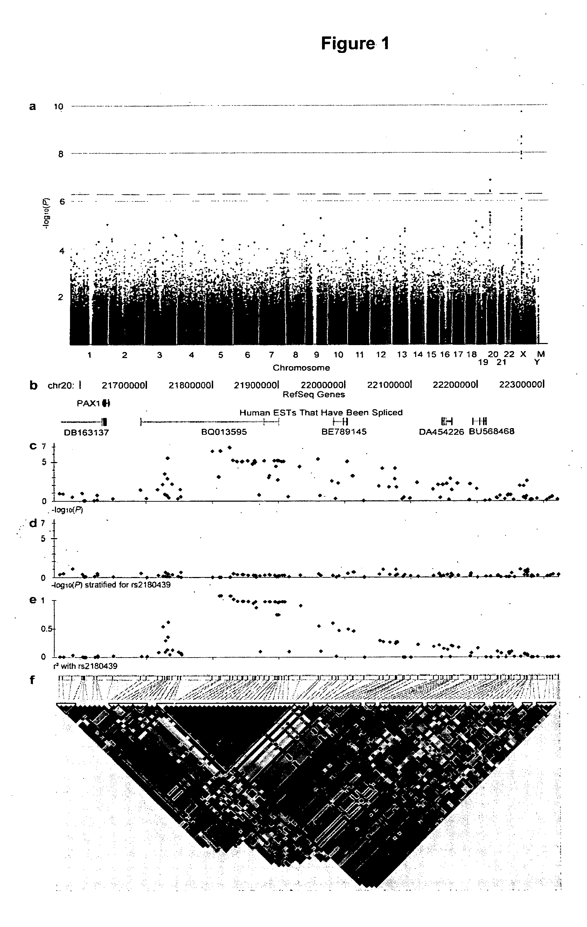

**Figure 1****: Genome-wide scan for association with AGA and details of the region 21,571,171 - 22,321,597 on chromosome 20p11.** (a) Negative log₁₀ of the Cochran-Armitage trend test P for the autosomes and of the allele frequency difference test P of chromosomes X, Y and the mitochondrion for quality-control-positive SNPs. Chromosomes are shown in alternating colours, with SNPs selected for the replication step highlighted in red. Genome-wide significance level is indicated by dashed red line. (b) Transcript information of the chromosome 20 locus (UCSC genome browser). (c) Negative log₁₀ association P are displayed for the GWAS (with rs2180439 indicated in red) and for an analysis stratified for rs2180439 (d). (e) The LD measured by r² between each SNP genotyped in the region and rs2180439 is shown. (f) LD displayed by GOLD heatmap and haplotype blocks indicated by black triangles were analyzed using Haploview software. High D' is indicated in red, low D' is indicated in blue.

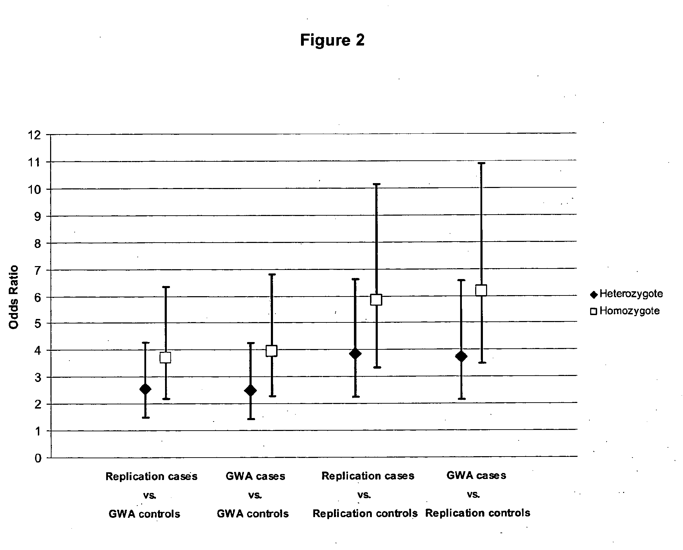

**Figure 2****: Genotype odds ratios of rs2180439 with increasing phenotypic divergence between cases and controls.** All cases had AGA that was representative of the most severely affected 10% of the distribution for the respective age class. Thereof, cases used in the GWAS were the most severely affecteds and cases used in the replication step were less extreme. Controls of the GWAS were population based where males with extreme AGA had been excluded. Controls of the replication step represented the least-affected 20% of the distribution for their age class. ORs for heterozygotes (C/T) are indicated by filled diamond, ORs for homozygotes (T/T) are indicated by open squares, 95% Cls are indicated by bars.

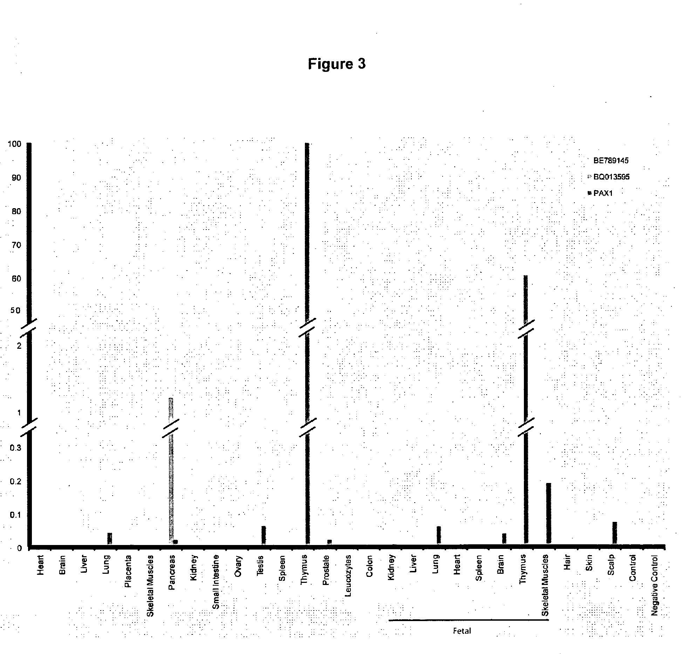

**Figure 3****: Expression of BQ013595, BE789145, and *PAX1* in human tissues.** Expression levels were determined by real time PCR, normalized by cyclophilin and displayed relative to the strongest expression *(PAX1* in adult thymus). *PAX1* expression is detectable in scalp.

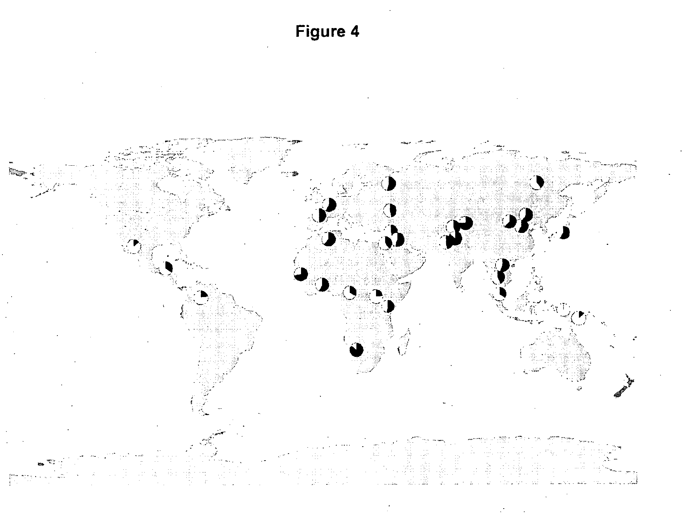

**Figure 4****: Global distribution of the rs2180439 risk allele.** Genotype data of the HGDP-CEPH Diversity Panel was extracted from the HGDP-CEPH Diversity Panel Database Supplement 2 at http://www.cephb.fr/hgdp-cephdb/. Worldwide distribution of the AGA risk allele T is indicated by the proportion of black shading in pie charts. Lower risk allele frequencies were observed in Oceanians and Native Americans

The examples illustrate the invention:

### Example 1: Materials and Methods

### German sample

Families and unrelated AGA and non-AGA individuals were recruited through various sources including press reports, and advertisements in magazines, newspapers and placards. Blood samples were drawn from 754 affected males who were <30 years of age having AGA of grades IV - VII, based on the classification of Hamilton JB (1951), Ann N Y Acad Sci 53:708-728 and modified by Norwood OT (1975), South Med J 68:1359-1365, or <40 years having AGA of grades V - VII. Based on these criteria, the AGA sample was representative of the most severely affected 10% of the distribution for the respective age class (Hamilton JB (1951), Ann N Y Acad Sci 53:708-728; Norwood OT (1975), South Med J 68:1359-1365). Of the affecteds, the 296 unrelated individuals with the strongest hair loss (mean±SD age [in years] 33.3±4.9, Hamilton/Norwood grades: median VI, lower quartile V, upper quartile VII) were selected for GWAS and 319 independent unrelated affecteds (mean±SD age [in years] 33.4±5.1, Hamilton/Norwood grades: median V, lower quartile V, upper quartile VI) were selected for the replication step. 234 non-affected males were >60 years of age and without AGA (mean±SD age [in years] 67.6±5.9), representing the least affected 20% of the distribution for this age class. 491 of the 754 affecteds were part of 352 nuclear families comprising of parents with one to four affected sons. As controls, we investigated 383 individuals (mean±SD age [in years] 61.1±5.3) from the population-based Heinz Nixdorf RECALL cohort (Schmermund A, et al. (2002), Am Heart J 144:212-218) consisting of 182 male and 201 female probands (mean±SD age [in years] 61.1±5.3) who were randomly selected from the general population. Of 129 male controls, hair status information was available: 16 individuals with grade I (Hamilton/Norwood classification), 21 individuals with grade II, one individual with grade IIa, eight individuals with grade III, 21 individuals with grade III vertex, four individuals with grade IIIa, ten individuals with grade IV, one individual with grade IVa, eleven individuals with grade V, 27 individuals with grade VI, and nine individuals with grade VII. Thirty six males with AGA grade VI and VII, respectively, were excluded from GWAS to increase phenotypic stratification between cases and controls. Females were included in the GWAS control sample as they were considered to show the representative AGA risk allele frequencies for population controls. The study was approved by the Ethics Committees of the Universities of Bonn, Düsseldorf and Essen, and informed consent was obtained from all participating individuals. All participants were of German descent.

### Australian sample

As part of a population based twin study (Nyholt DR, et al. (2003), J Invest Dermatol 121:1561-1564), hair status was documented in 291 unrelated male individuals for whom DNA was available. Measures of hair loss were obtained in the course of an extensive semi-structured telephone interview with respondent booklet, designed to assess physical, psychological and social manifestations of alcoholism and related disorders, conducted with 6265 twins born 1964-71 from the volunteer based Australian Twin Registry. All males (45% of the sample) were asked to rate their degree of hair loss, if any, using the Hamilton-Norwood Baldness scale (Hamilton JB (1951), Ann N Y Acad Sci 53:708-728; Norwood OT (1975), South Med J 68:1359-1365), which was printed in the respondent booklet. This data collection scheme was validated in a study by Ellis JA, et al. (1998), J Invest Dermatol 110:849-853. Individuals with hair loss Hamilton/Norwood grade ≥ IIa were classified as AGA affecteds and individuals with Hamilton/Norwood grade I were classified as controls. The study was approved by the Human Research Ethics Committee of the Queensland Institute of Medical Research, and informed consent was obtained from all participating individuals.

### DNA extraction

EDTA anticoagulated venous blood samples were collected from all individuals. Lymphocyte DNA from German AGA affecteds, non-affecteds, families, and Australian samples was isolated by salting out with saturated NaCl solution (Miller SA, et al. (1988), Nucleic Acids Res 16:1215) and DNA from German population-based controls was isolated using a Chemagic Magnetic Separation Module I (Chemagen, Baesweiler, Germany) according to the manufacturer recommendations. Genomic DNAs were diluted to working concentrations of 50 ng/µl for genome-wide genotyping and 2.5 ng/µl for the replication step.

### Genotyping

Genome-wide genotyping of 317,503 SNPs in 150 AGA affecteds using the IIIumina HumanHap300 BeadChip and of 561,466 SNPs in 146 affecteds and 383 population based controls using the IIIumina HumanHap550 BeadChip was conducted according to the Infinium II protocol from Illumina (Illumina, San Diego, USA). A DNA sample was deemed to have failed if it generated genotypes at fewer than 95% of loci of the respective BeadChip. A SNP was deemed to have failed if fewer than 95% of case or control samples generated a genotype at the locus. SNPs were excluded with a minor allele frequency (MAF) < 0.01 in controls and a Hardy-Weinberg-Equilibrium (HWE) P < 10⁻⁷ in controls. 531,695 SNPs passed quality criteria and were used for association analysis.
For the replication study, the best SNPs were selected based on the P values of the Cochran-Armitage trend test for the autosomes and the pseudoautosomal region and based on the allele frequency difference test for chromosomes X, Y, and the mitochondrion. To maximize efficiency in the replication step, four tagging SNPs of chromosome 20p11 were selected using the pairwise tagging algorithm of Haploview software (Barrett JC, et al. (2005), Bioinformatics 21:263-265) (parameters: r² ≥ 0.8 and LOD ≥ 3) and CEU HapMap data (Frazer KA, et al. (2007), Nature 449:851-861). The genome-wide best SNP, rs19998076 (Supplementary Table 1), was forced to be included and rs2180439 which was in perfect LD with rs1998076 was added for additional confidence in the most significant SNP. The genome-wide next best 30 SNPs excluding the *AR* locus (chromosome X 65.5 - 67.2 Mb, build 36) were selected for replication analysis. Visual inspection of genotype clusters of selected SNPs revealed for rs4976846 a shifted genotype cluster for a subset of samples and the SNP was excluded from the genome-wide data set. Of the 34 selected SNPs four had to be excluded: rs11655206 and rs4805229 could not be included in the two plexes for MassArray genotyping, rs13079866 was a genotyping failure, and rs1139488 showed fuzzy genotype clusters, significant deviation from HWE, and 19 Mendel errors, respectively, in the family data.
Genotyping for the replication step in the German and Australian samples was performed using the MassArray system on a Sequenom Compact MALDI-TOF device (Sequenom Inc., San Diego, CA, USA). Primer sequences and PCR/assay conditions can be obtained from the authors upon request. SNP call rates were >95.0% and samples with call rates <95% were excluded from the analysis.

### Statistical analysis

Genome-wide association analysis at single marker level was performed by Cochran-Armitage trend test for the autosomes and the pseudoautosomal region and by the allele frequency difference test for chromosomes X, Y, and the mitochondrion. P < 5 x 10⁻⁷ were assumed to be genome-wide significant as suggested by The Wellcome Trust Case Control Consortium (2007), Nature 447:661-678. Haplotype and family based analysis of the chromosome 20 locus was conducted with FAMHAP software (Becker T, et al. (2005), Ann Hum Genet 69:747-756). For case-control haplotype analysis, P values were calculated from the _{X}² distribution with n-1 degrees of freedom of the respective likelihood-ratio test (n = number of different haplotypes). The family data were analyzed with the permutation-based association test for nuclear families (Zhao H, et al. (2000), Am J Hum Genet 67:936-946; Knapp M, Becker T (2003), Hum Hered 56:2-9). For each marker (single locus analysis) and each marker combination (haplotype analysis), we used 10¹⁰ permutation replicates. As rs2180439 was the most associated marker in the German combined sample, we carried out further statistical analysis based on that marker in the compound data set. We used logistic regression as suggested by Cordell HJ, Clayton DG (2002), Am J Hum Genet 70:124-141. First, we tested for deviation from a multiplicative model on the OR scale by comparing a 2-degree of freedom (DF) model with parameter β₁ for a multiplicative SNP-effect and parameter β₂ for a dominance effect versus a 1-DF model with just β₁. Secondly, we tested for independent effects of the remaining four SNPs of the region. Therefore, we tested a 3-DF model with parameters β₁, β₂ and β₃ (parameter for multiplicative effect of additional SNP) versus the 2-DF model for rs2180439 (β₁, β₂). If this test was significant, we further tested for additional dominance at the new SNP (parameter β₄) by comparing the 4-DF model to the 3-DF model. In order to test for interaction with the X chromosome, we considered the initial GWAS case sample (n = 296) versus the male controls (n = 146). For this data set, the model with β₁, β₃ and β₄ had a P value of 5.89 x 10⁻⁶. Introduction of the X-chromosomal SNP rs1041668 improved the model-fit significantly (P = 1.32 x 10⁻⁸), resulting in a 4-DF model with P = 1.17 x 10⁻¹³. On top of this model, neither the interaction terms with rs2180439 (P = 0.975) nor with rs6113491 (P = 0.984) yielded significant improvement of the model fit.

### Expression analysis

To test for tissue specific expression of BQ013595, BE789145 and *PAX1,* we used the Human Multiple-Tissue cDNA (MTC) Panels I, II and the Human Fetal MTC Panel (Takara Bio Europe/Clontech, Saint-Germain-en-Laye, France). In addition, total RNA from hair follicles, skin and scalp was extracted (RNeasy Micro Kit, Qiagen). Quality and quantity of the RNA was analyzed on a NanoDrop ND-1000 spectrophotometer (Peqlab Biotechnologie, Erlangen, Germany) and reverse transcription was performed using SuperScript First Strand Synthesis System for RT-PCR (Invitrogen, Karlsruhe, Germany) with oligo dT primers. cDNAs were adjusted in an initial real-time PCR experiment using the TaqMan Endogenous Controls Human Cyc (Cyclophilin, 4326316E; Applied Biosystems, Darmstadt, Germany) in which 5 µl of the MTC-cDNAs were set as reference. Relative quantifications in real-time experiments were performed in 384-well plates with the adjusted amounts of cDNAs per well in a total volume of 20 µl on the ABI Prism 7900HT Fast Real-Time PCR System (Applied Biosystems, Darmstadt, Germany) using Custom TaqMan Gene Expression Assays for BQ013595 (forward primer AGACATACTTTTCTATTCACCTTCCAATGATG, reverse primer GGTTCTGACCCTTATTATTGGAACTCA, probe AAGTCCCAACAAACTCCA), BE789145 (forward primer ACCTGACATCAACGTACAAAGATTCA, reverse primer GCGGCTTCTCCTCTGCAT, probe CATGGCCACAAGAGTTA), and *PAX1* (forward primer GGCTAGAGAAACCTGCCTTAGAG, reverse primer CGCCCACGGCAGAGA, probe ACACTCAGTCGGCCTCC). Each sample was normalized by TaqMan Endogenous Controls Human Cyc, and CT values were transformed by 100,000,000/(2^{CT}). Values were calculated relative to the expression of *PAX1* in thymus which was set as 100%.

### Example 2: Genome-wide association study

We conducted a genome-wide association study (GWAS) to identify predisposing genetic factors in AGA. The analysis was performed on 296 males with early-onset AGA (Hamilton/Norwood hair loss grades: median VI, lower quartile V, upper quartile VII ) and 383 population based controls (201 females and 182 males), all of German descent, were genotyped with IIIumina BeadChips (HumanHap300/HumanHap550). For the analysis, 36 male controls with strong AGA were excluded resulting in 347 controls. Following quality control, we performed association analysis on 531,695 single nucleotide polymorphisms (SNPs) with a minor allele frequency (MAF) of > 1% in controls. The SNP call rate was 99.78%.
We found highly significant association in a 1.7 Mb region flanking the previously implicated *AR* locus (Fig. 1). The best SNP (rs1998076, P = 1.3 x 10⁻⁷) located outside the *AR* locus was on chromosome 20, a locus which was supported by 17 additional SNPs with P < 10⁻⁵.

**Table 2: Genome-wide association results for AGA (excluding AR locus).**

| SNP | Chr. | Position [bp]^{a} | MAF cases | MAF controls | *P* | OR (95% Cl) |
|---|---|---|---|---|---|---|
| rs1998076 | 20 | 21,828,045 | 0.282 (A) | 0.427 (A) | 1.30 x 10⁻⁷ | 1.90 (1.50 - 2.41) |
| rs6075852 | 20 | 21,814,151 | 0.292 (A) | 0.429 (A) | 3.50 x 10⁻⁷ | 1.83 (1.45 - 2.31) |
| rs2180439 | 20 | 21,801,100 | 0.292 (C) | 0.429 (C) | 3.85 x 10⁻⁷ | 1.82 (1.45-2.30) |
| rs6137444 | 20 | 21,733,639 | 0.264 (C) | 0.383 (C) | 3.11 x 10⁻⁶ | 1.74 (1.37 - 2.21) |
| rs201571 | 20 | 21,961,514 | 0.289 (C) | 0.411 (C) | 4.31 x 10⁻⁶ | 1.72 (1.36 - 2.17) |
| rs10992241 | 9 | 93,931,599 | 0.156 (C) | 0.262 (C) | 5.31 x 10⁻⁶ | 1.92 (1.44 - 2.54) |
| rs6137473 | 20 | 21,832,693 | 0.321 (G) | 0.445 (G) | 5.58 x 10⁻⁶ | 1.70 (1.35 - 2.13) |
| rs6047768 | 20 | 21,901,649 | 0.331 (G) | 0.457 (G) | 6.09 x 10⁻⁶ | 1.70 (1.35 - 2.13) |
| rs2207878 | 20 | 21,899,847 | 0.331 (G) | 0.457 (G) | 6.19 x 10⁻⁶ | 1.70 (1.35 - 2.13) |
| rs6035995 | 20 | 21,879,683 | 0.331 (G) | 0.455 (G) | 6.97 x 10⁻⁶ | 1.69 (1.35 - 2.13) |
| rs6113424 | 20 | 21,854,780 | 0.331 (G) | 0.455 (G) | 7.05 x 10⁻⁶ | 1.69 (1.35 - 2.12) |
| rs2024885 | 20 | 21,865,393 | 0.331 (A) | 0.455 (A) | 7.05 x 10⁻⁶ | 1.69 (1.35 - 2.12) |
| rs201543 | 20 | 21,894,928 | 0.331 (A) | 0.455 (A) | 7.05 x 10⁻⁶ | 1.69 (1.35 - 2.12) |
| rs17817969 | 18 | 70,473,226 | 0.223 (T) | 0.117 (T) | 7.16 x 10⁻⁶ | 2.17 (1.51 - 3.11) |
| rs6047769 | 20 | 21,906,049 | 0.333 (A) | 0.457 (A) | 8.47 x 10⁻⁶ | 1.68 (1.34 - 2.12) |
| rs1884592 | 20 | 21,838,690 | 0.331 (C) | 0.454 (C) | 8.58 x 10⁻⁶ | 1.68 (1.34 - 2.11) |
| rs6137476 | 20 | 21,845,656 | 0.331 (A) | 0.454 (A) | 8.58 x 10⁻⁶ | 1.68 (1.34 - 2.11) |
| rs6047731 | 20 | 21,847,141 | 0.331 (G) | 0.454 (G) | 8.58 x 10⁻⁶ | 1.68 (1.34 - 2.11) |
| rs6113491 | 20 | 22,005,415 | 0.359 (C) | 0.483 (C) | 8.63 x 10⁻⁶ | 1.66 (1.33 - 2.08) |
| rs1555264 | 20 | 21,909,410 | 0.332 (G) | 0.455 (G) | 8.64 x 10⁻⁶ | 1.68 (1.34 - 2.11) |
| rs4658627 | 1 | 242,577,799 | 0.172 (A) | 0.308 (A) | 9.60 x 10⁻⁶ | 2.14 (1.51 - 3.02) |
| rs11975187 | 7 | 153,863,909 | 0.490 (A) | 0.359 (G) | 1.02 x 10⁻⁵ | 1.86 (1.41 - 2.46) |
| rs4805229 | 19 | 33,692,043 | 0.380 (T) | 0.268 (T) | 1.34 x 10⁻⁵ | 1.67 (1.32 - 2.12) |
| rs9300398 | 13 | 96,690,316 | 0.363 (T) | 0.252 (T) | 1.46 x 10⁻⁵ | 1.69 (1.33 - 2.15) |
| rs4771987 | 13 | 96,699,391 | 0.429 (G) | 0.315 (G) | 1.88 x 10⁻⁵ | 1.63 (1.30 - 2.05) |
| rs3786343 | 18 | 59,798,875 | 0.458 (T) | 0.340 (T) | 2.27 x 10⁻⁵ | 1.64 (1.31 - 2.05) |
| rs6986303 | 8 | 134,547,711 | 0.309 (A) | 0.205 (A) | 2.33x 10⁻⁵ | 1.74 (1.35 - 2.24) |
| rs9856948 | 3 | 189,240,095 | 0.419 (C) | 0.304 (C) | 2.51 x 10⁻⁵ | 1.65 (1.31 - 2.08) |
| rs1139488 | 9 | 115,193,721 | 0.306 (C) | 0.418 (C) | 2.77 x 10⁻⁵ | 1.63 (1.29 - 2.05) |
| rs13079866 | 3 | 196,641,541 | 0.061 (G) | 0.131 (G) | 2.84 x 10⁻⁵ | 2.33 (1.56 - 3.49) |
| rs7319347 | 13 | 66,977,210 | 0.072 (C) | 0.173 (C) | 3.00 x 10⁻⁵ | 2.70 (1.66 - 4.38) |
| rs17818946 | 18 | 70,519,734 | 0.170 (G) | 0.082 (G) | 3.06 x 10⁻⁵ | 2.30 (1.52 - 3.47) |
| rs6804475 | 3 | 113,152,321 | 0.246 (A) | 0.352 (A) | 3.22 x 10⁻⁵ | 1.66 (1.30 - 2.12) |
| rs500629 | 11 | 113,550,770 | 0.346 (G) | 0.241 (G) | 3.62 x 10⁻⁵ | 1.66 (1.30 - 2.12) |
| rs10495747 | 2 | 24,368,500 | 0.149 (C) | 0.075 (C) | 3.78 x 10⁻⁵ | 2.16 (1.50 - 3.10) |
| rs1464766 | 5 | 111,641,241 | 0.243 (A) | 0.372 (A) | 4.14 x 10⁻⁵ | 1.84 (1.35-2.51) |
| rs2551182 | 2 | 24,293,921 | 0.128 (T) | 0.061 (T) | 4.75 x 10⁻⁵ | 2.29 (1.54 - 3.39) |
| rs300639 | 9 | 32,776,553 | 0.067 (T) | 0.021 (T) | 4.83 x 10⁻⁵ | 3.45 (1.85 - 6.42) |
| rs10001582 | 4 | 124,578,020 | 0.364 (A) | 0.478 (A) | 4.91 x 10⁻⁵ | 1.60 (1.28 - 2.01) |
| rs11584662 | 1 | 184,847,447 | 0.417 (T) | 0.467 (G) | 4.92 x 10⁻⁵ | 1.60 (1.28 - 1.99) |
| rs1009070 | 11 | 29,484,237 | 0.106 (G) | 0.038 (G) | 5.20 x 10⁻⁵ | 3.05 (1.78 - 5.24) |
| rs880013 | 9 | 1,425,496 | 0.474 (T) | 0.415 (C) | 5.28 x 10⁻⁵ | 1.56 (1.25 - 1.95) |
| rs4896028 | 6 | 134,580,872 | 0.309 (G) | 0.209 (G) | 5.35 x 10⁻⁵ | 1.69 (1.32 - 2.18) |
| rs11725633 | 4 | 37,868,359 | 0.368 (T) | 0.483 (T) | 5.57 x 10⁻⁵ | 1.60 (1.28 - 2.00) |
| rs11655206 | 17 | 72,501,171 | 0.497 (T) | 0.361 (T) | 5.60 x 10⁻⁵ | 1.74 (1.32 - 2.30) |
| rs6550859 | 3 | 24,385,419 | 0.390 (G) | 0.468 (A) | 5.72 x 10⁻⁵ | 1.77 (1.34 - 2.34) |
| rs3786939 | 19 | 44,888,022 | 0.301 (A) | 0.184 (A) | 5.79 x 10⁻⁵ | 1.91 (1.39 - 2.61) |

| | | | | | | |
|---|---|---|---|---|---|---|
| (^{a} Build 36) | | | | | | |

Three SNPs at this locus reached genome-wide significance based on criteria suggested elsewhere (Consortium TWTCC (2007), Nature 447:661-678).

### Example 3: Replication study

To replicate the association findings, four tagging SNPs of the chromosome 20 region and the next best 29 SNPs (excluding the *AR* locus) were selected. In addition, rs2180439, which was in perfect LD with rs1998076, was included for the purpose of providing additional confidence in the most significant SNP. Selected markers were analyzed in an independent set of 319 affected and 234 unaffected individuals (males >60 years of age without AGA). The association with all five SNPs of the chromosome 20 locus was replicated (*P* = 8.13 x 10⁻¹⁰ for rs6113491).

**Table 3 Replication analysis in 319 German cases and 234 German controls**

| SNP | Chr. | Position [bp]^{a} | MAF cases | MAF controls | *P* | OR (95% CI) |
|---|---|---|---|---|---|---|
| rs6113491 | 20 | 22,005,415 | 0.364 (C) | 0.447 (A) | 8.13x10⁻¹⁰ | 2.17 (1.7 - 2.77) |
| rs2180439 | 20 | 21,801,100 , | 0.303 (C) | 0.485 (C) | 1.37 x 10⁻⁰⁹ | 2.17 (1.7 - 2.78) |
| rs1998076 | 20 | 21,828,045 | 0.301 (A) | 0.479 (A) | 3.69 x 10⁻⁰⁹ | 2.13 (1.66 - 2.73) |
| rs201571 | 20 | 21,961,514 | 0.313 (C) | 0.483 (C) | 2.21 x 10⁻⁸ | 2.05 (1.6 - 2.62) |
| rs6137444 | 20 | 21,733,639 | 0.277 (C) | 0.404 (C) | 1.57 x 10⁻⁰⁵. | 1.76 (1.37 - 2.27) |
| rs10992241 | 9 | 91,971,333 | 0.289 (C) | 0.201 (C) | 8.68 x 10⁻⁰⁴ | 1.62 (1.22 - 2.15) |
| rs11725633 | 4 | 38,014,530 | 0.422 (T) | 0.479 (T) | 0.058 | 1.26 (0.99 - 1.6) |
| rs4771987 | 13 | 96,699,391 | 0.417 (G) | 0.363 (G) | 0.075 | 1.25 (0.98 - 1.6) |
| rs6804475 | 3 | 113,152,321 | 0.292 (A) | 0.340 (A) | 0.081 | 1.25 (0.97 - 1.62) |
| rs3786939 | 19 | 44,888,022 | 0.202 (A) | 0.162 (A) | 0.085 | 1.31 (0.96 - 1.79) |
| rs880013 | 9 | 1,425,496 | 0.431 (C) | 0.479 (C) | 0.123 | 1.21 (0.95 - 1.54) |
| rs500629 | 11 | 113,550,770 | 0.307 (G) | 0.276 (G) | 0.249 | 1.17 (0.9 - 1.52) |
| rs9300398 | 13 | 96,690,316 | 0.335 (T) | 0.303 (T) | 0.252 | 1.16 (0.9 - 1.5) |
| rs1009070 | 11 | 29,484,237 | 0.062 (G) | 0.076 (G) | 0.35 | 1.25 (0.78 - 2) |
| rs4896028 | 6 | 134,580,872 | 0.268 (G) | 0.244 (G) | 0.368 | 1.14 (0.86 - 1.5) |
| rs10495747 | 2 | 24,426,647 | 0.130 (C) | 0.115 (C) | 0.458 | 1.15 (0.8 - 1.65) |
| rs17817969 | 18 | 70,473,226 | 0.147 (T) | 0.132 (T) | 0.483 | 1.13 (0.8 - 1.6) |
| rs6986303 | 8 | 134,547,711 | 0.288 (A) | 0.269 (A) | 0.495 | 1.1 (0.84 - 1.43) |
| rs10001582 | 4 | 124,716,175 | 0.429 (A) | 0.409 (A) | 0.51 | 1.09 (0.85 - 1.38) |
| rs3786343 | 18 | 59,798,875 | 0.395 (T) | 0.376 (T) | 0.527 | 1.08 (0.85 - 1.38) |
| rs1464766 | 5 | 111,641,241 | 0.324 (A) | 0.342 (A) | 0.545 | 1.08 (0.84 - 1.39) |
| rs11975187 | 7 | 153,670,624 | 0.346 (G) | 0.363 (G) | 0.559 | 1.08 (0.84 - 1.38) |
| rs11584662 | 1 | 183,312,481 | 0.436 (T) | 0.453 (T) | 0.573 | 1.07 (0.84 - 1.36) |
| rs17818946 | 18 | 70,519,734 | 0.108 (G) | 0.098 (G) | 0.588 | 1.11 (0.75 - 1.65) |
| rs6550859 | 3 | 24,385,419 | 0.498 (A) | 0.483 (A) | 0.608 | 1.06 (0.84 - 1.35) |
| rs9856948 | 3 | 189,240,103 | 0.350 (C) | 0.340 (C) | 0.735 | 1.04 (0.81 - 1.34) |
| rs2551182 | 2 | 24,352,068 | 0.099 (T) | 0.096 (T) | 0.886 | 1.03 (0.69 - 1.54) |
| rs7319347 | 13 | 66,977,210 | 0.160 (C) | 0.162 (C) | 0.91 | 1.02 (0.74 - 1.41) |
| rs4658627 | 1 | 240,837,217 | 0.270 (A) | 0.274 (A) | 0.911 | 1.02 (0.78 - 1.33) |
| rs300639 | 9 | 32,776,553 | 0.023 (T) | 0.023 (T) | 1 | 1 (0.44 - 2.27) |

| | | | | | | |
|---|---|---|---|---|---|---|
| (^{a} Build 36) | | | | | | |

In addition, rs10992241 on chromosome 9 showed significance. However, the putative risk allele (C) in the replication study differed from that in the GWAS (T), and thus rs10992241 was not considered to be truly associated. After combining the samples from the genome-wide and replication analyses, rs2180439 showed the highest significance (*P* = 2.67 x 10⁻¹⁵, Table 4). Haplotype analysis did not significantly improve the association findings.

**Table 4 Association between the chromosome 20 locus and AGA in the German sample**

| | | | | MAF | | Genotypes | | | |
|---|---|---|---|---|---|---|---|---|---|
| SNP (position)^{a} | Sample | Cases^{b} | Controls^{c} | Cases^{b} | Controls^{c} | Cases^{b} | Controls^{c} | *P* | OR(95% CI)^{d} |
| rs6137444 | GWAS | 296 | 347 | 0.264(C) | 0.383(C) | 14/128/154 | 49/168/130 | 3,11x10⁻⁶ | 1.74(1.37-2.21) |
| (21,733,639bp) | Replication | 319 | 234 | 0.277(C) | 0.404(C) | 21/135/163 | 45/99/90 | 1.57x10⁻⁵ | 1.76(1.37-2.27) |
| | Combined^{e} | 605 | 579 | 0.269(C) | 0.391(C) | 35/255/315 | 93/267/219 | 2.20x10⁻¹⁰ | |
| rs2180439 | GWAS | 296 | 347 | 0.292(C) | 0.429(C) | 21/131/144 | 66/166/115 | 3.85x10⁻⁷ | 1.82(1.45-2.30) |
| (21,801,100bp) | Replication | 319 | 234 | 0.303(C) | 0.485(C) | 23/147/149 | 62/103/69 | 1.37x10⁻⁹ | 2.17(1.70-2.78) |
| | Combined^{e} | 605 | 579 | 0.293(C) | 0.452(C) | 43/268/294 | 127/269/183 | 2.67x10⁻¹⁵ | |
| rs1998076 | GWAS | 296 | 347 | 0.282(A) | 0.427(A) | 20/120/144 | 65/163/115 | 1.30x10⁻⁷ | 1.90(1.50-2.41) |
| (21,828,045 bp) | Replication | 319 | 234 | 0.301(A) | 0.479(A) | 23/146/150 | 61/102/71 | 3.69x10⁻⁹ | 2.13(1.66-2.73) |
| | Combined^{e} | 605 | 579 | 0.292(A) | 0.448(A) | 43/267/295 | 126/267/186 | 7.73x10⁻¹⁵ | |
| rs201571 | GWAS | 296 | 347 | 0.289(C) | 0.411(C) | 17/137/142 | 61/163/123 | 4.31x10⁻⁶ | 1.72(1.36-2.17) |
| (21,961,514 bp) | Replication | 319 | 234 | 0.314(C) | 0.483(C) | 30/140/149 | 58/110/66 | 2.21x10⁻⁸ | 2.05(1.60-2.62) |
| | Combined^{e} | 605 | 579 | 0.298(C) | 0.44(C) | 46/269/290 | 119/272/188 | 1.21x10⁻¹² | |
| rs6113491 | GWAS | 296 | 347 | 0.359(C) | 0.483(C) | 29/154/112 | 88/159/100 | 8.63x10⁻⁶ | 1.66(1.33-2.08) |
| (22,005,415 bp) | Replication | 319 | 234 | 0.364(C) | 0.447(A) | 38/156/125 | 77/105/52 | 8.13x10⁻¹⁰ | 2.17(1.70-2.77) |
| | Combined^{e} | 605 | 579 | 0.359(C) | 0.488(A) | 66/302/237 | 165/263/151 | 1.13x10⁻¹³ | |

| | | | | | | | | | |
|---|---|---|---|---|---|---|---|---|---|
| ^{a} NCBI build 36. ^{b} GWAS cases were the most extremely affected. ^{c} GWAS controls are population based, the most extreme AGA affected having been excluded; replication controls are males >60 years of age without AGA and representing the least affected 20%. ^{d} ORs were not calculated for the combined samples since the composition of the two control samples differed as regards the exclusion of AGA cases. ^{e} Twelve GWAS samples were excluded in the combined analysis due to a call rate <95% for the reduced SNP set. | | | | | | | | | |

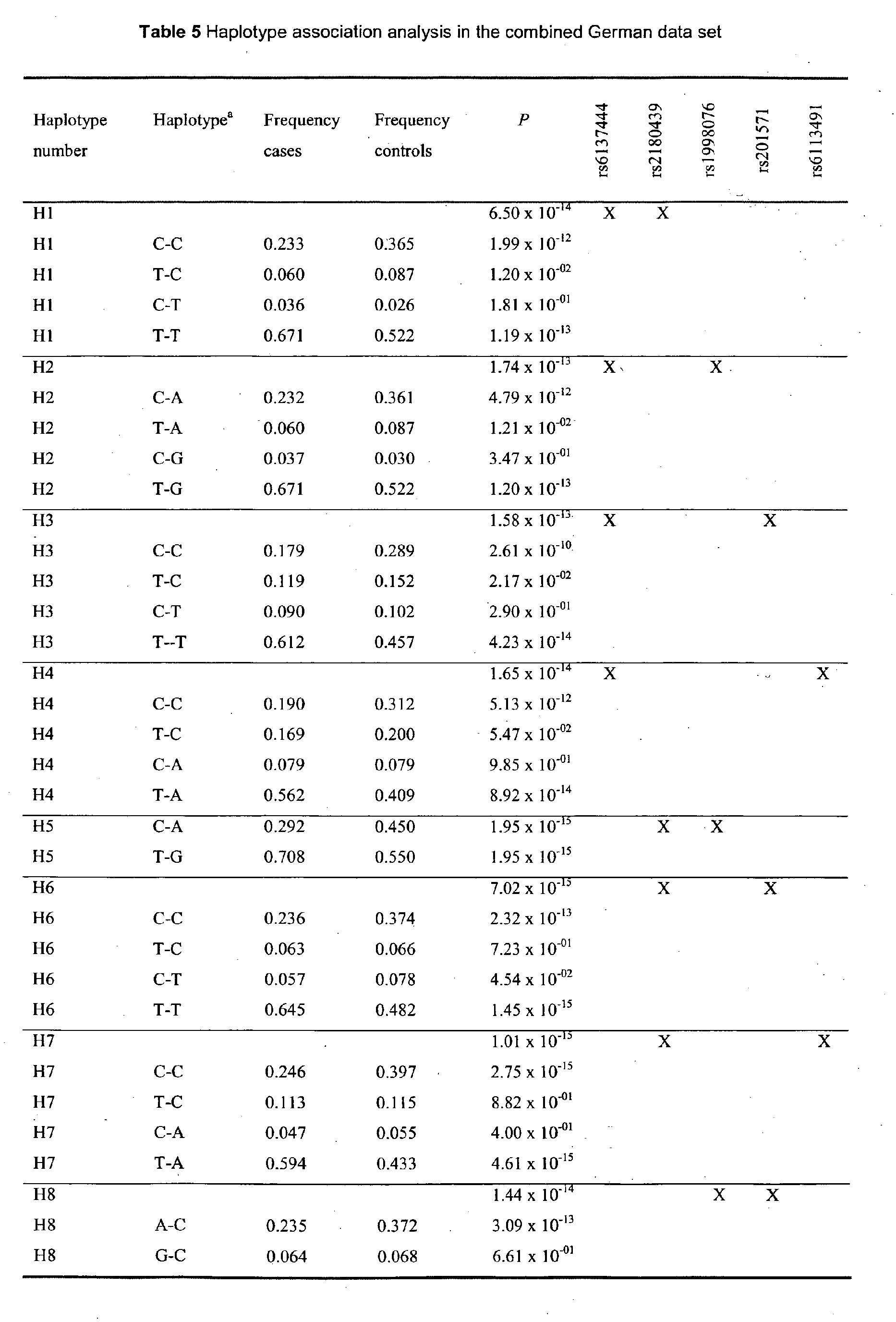

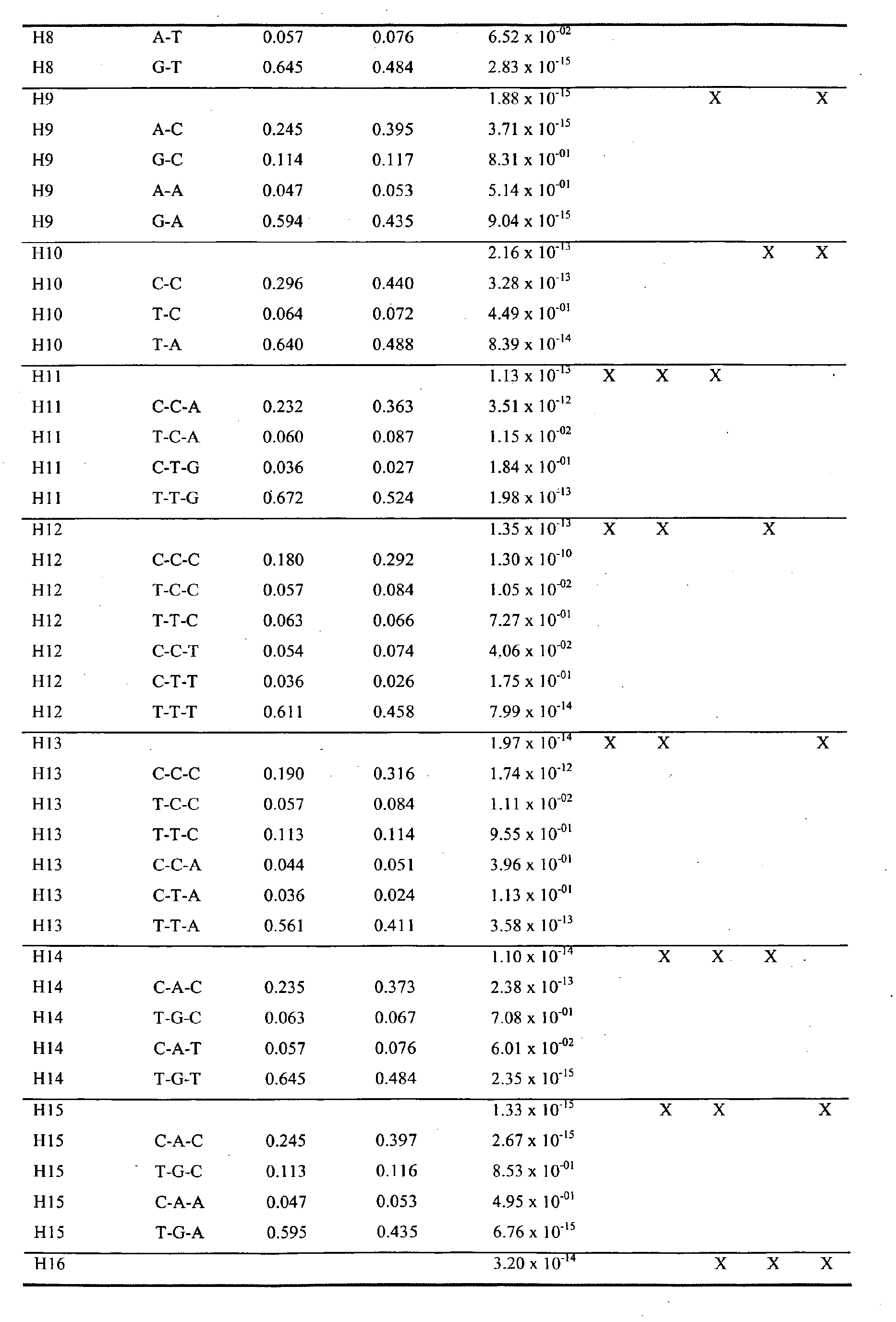

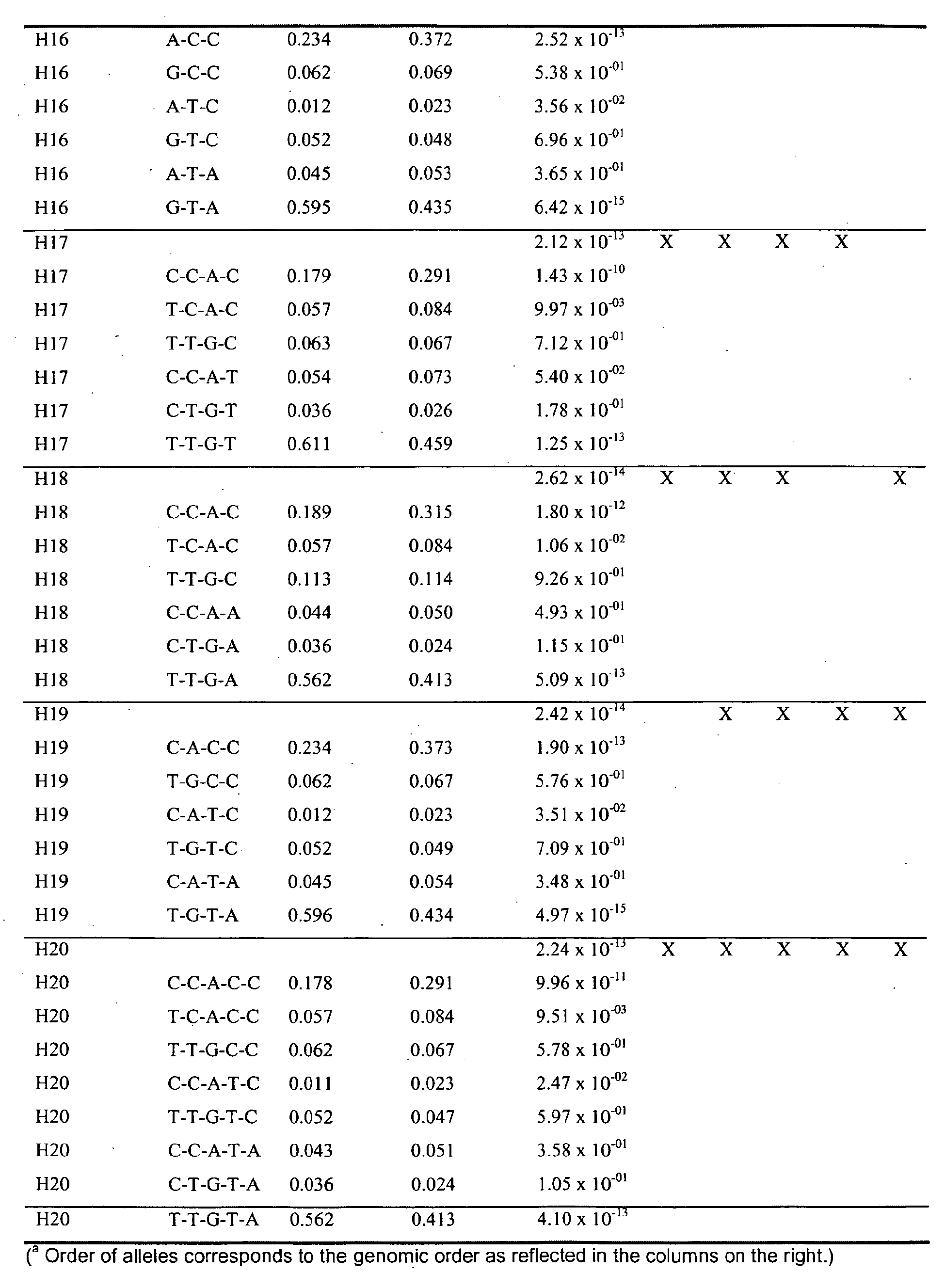

A subset of the affected sample were members of 352 nuclear families, and the results of a transmission disequilibrium test (TDT) were in accordance with the case-control findings which had shown association for the five chromosome 20 SNPs and the highest significance for rs2180439 (TDT *P* = 7.22 x 10⁻⁷).

**Table 6 Transmission disequilibrium test in 352 German nuclear families**

| SNP | Chr. | Position [bp]^{a} | Transmitted | Untransmitted | *P*^{b} | OR (95% CI) |
|---|---|---|---|---|---|---|
| rs6137444 | 20 | 21,733,639 | 182 (C) | 255 (C) | 4.79x10⁻⁴ | 1.40 (1.16 - 1.69) |
| rs2180439 | 20 | 21,801,100 | 172 (C) | 277 (C) | 7.22 x 10⁻⁷ | 1.61 (1.33 - 1.95) |
| rs1998076 | 20 | 21,828,045 | 170 (A) | 270 (A) | 1.87 x 10⁻⁶ | 1.59 (1.31 - 1.92) |
| rs201571 | 20 | 21,961,514 | 168 (C) | 258 (C) | 1.30 x 10⁻⁵ | 1.54 (1.26 - 1.86) |
| rs6113491 | 20 | 22,005,415 | 193 (C) | 288 (C) | 1.48 x 10⁻⁵ | 1.49 (1.24 - 1.79) |

| | | | | | | |
|---|---|---|---|---|---|---|
| (^{a} Build 36; ^{b} Association *P* value) | | | | | | |

We also performed an initial step to investigate the importance of this locus in non-German populations by analyzing a modestly sized Australian sample for the five chromosome 20 SNPs. Again, rs2180439 showed significant association.

**Table 7 Replication analysis in Australian AGA sample**

| | | 141 cases^{a} vs. 150 controls | | | | 60 cases^{b} vs. 150 controls | | | |
|---|---|---|---|---|---|---|---|---|---|
| SNP | Minor allele | MAF cases | MAF controls | *P* | OR (95% CI) | MAF cases | MAF controls | *P* | OR (95% CI) |
| rs6137444 | C | 0.298 | 0.343 | 0.247 | 1.23 (0.86-1.74) | 0.275 | 0.343 | 0.171 | 1.37 (0.86 - 2.19) |
| rs2180439 | C | 0.340 | 0.420 | 0.041 | 1.4 (1-1.96) | 0.317 | 0.420 | 0.045 | 1.56 (1 - 2.44) |
| rs1998076 | A | 0.344 | 0.417 | 0.064 | 1.36 (0.97-1.91) | 0.317 | 0.417 | 0.053 | 1.54 (0.99 - 2.41) |
| rs201571 | C | 0.309 | 0.417 | 0.0069 | 1.6 (1.14-2.25) | 0.292 | 0.417 | 0.018 | 1.73 (1.1 - 2.73) |
| rs6113491 | C | 0.401 | 0.477 | 0.066 | 1.36 (0.98-1.89) | 0.392 | 0.477 | 0.111 | 1.41 (0.92 - 2.17) |

| | | | | | | | | | |
|---|---|---|---|---|---|---|---|---|---|
| (^{a} AGA grade ≥ IIa; ^{b} AGA grade ≥ IV) | | | | | | | | | |

### Example 4: Phenotype

As regards phenotype, German GWAS cases (extreme) and controls (containing affecteds) differed from the replication cases (less extreme) and controls (unaffecteds). The genotype based ORs of rs2180439 in the four possible case-control comparisons showed an increase from the least extreme (replication cases/GWAS controls, OR = 3.72, 95% CI 2.18-6.34) to the most extreme (GWAS cases/replication controls, OR = 6.16, 95% CI 3.48-10.92) for the homozygotes (Fig. 2). Correlation of effect size and phenotypic difference supports an association between rs2180439 and AGA. However, this relation was less clear for the heterozygotes. We used logistic regression (Cordell HJ, Clayton DG (2002), Am J Hum Genet 70:124-141) and tested for deviation from a multiplicative model on the OR scale in the combined data set. The test showed a tendency toward a dominant model for rs2180439 (*P* = 0.023)

**Table 8 Test for deviation from a multiplicative model with two degrees of freedom versus one degree of freedom in the combined German data set**

| SNP | *P* |
|---|---|
| rs6137444 | 0.0608 |
| rs2180439 | 0.0225 |
| rs1998076 | 0.0203 |
| rs201571 | 0.0583 |
| rs6113491 | 0.0025 |

We also found evidence that rs2180439 and rs6113491 contribute independently to the risk of AGA, and that rs6113491 showed a dominant effect.

**Table 9 Tests for additional effects of further SNPs at the chromosome 20 locus in the combined German data set**

| | LD measure | *P* | |
|---|---|---|---|
| | r² with rs2180439 | Test for independence^{a} | Test for dominance^{b} |
| rs6137444 | 0.6 | 0.865 | n.d. |
| rs1998076 | 0.99 | 0.2634 | n.d. |
| rs201571 | 0.5 | 0.0367 | 0.3677 |
| rs6113491 | 0.44 | 0.0033 | 0.0192 |

| | | | |
|---|---|---|---|
| (^{a} 3 degrees of freedom versus 2 degrees of freedom; ^{b} 4 degrees of freedom versus 3 degrees of freedom) | | | |

To observe this effect, however, use of the combined sample was necessary since rs6113491 did not remain significant after stratifying the GWAS data for rs2180439 (Fig. 1).

### Example 5: Interaction

We tested chromosome 20p11 for interaction with the primary associated SNP at the AR locus (rs1041668). Introduction of rs1041668 improved the model-fit significantly. However, neither the interaction terms with rs2180439 (*P* = 0.975) nor with rs6113491 (P = 0.984) yielded significant improvement of the model-fit. Thus, our data provide no evidence for interaction effects between the two regions.

### Example 6: Gene contributing to the development of AGA

Two expressed sequence tags, BQ013595 and BE789145, map to the associated region on 20p11 (Fig. 1). Since a gene contributing to the development of AGA would be expected to be expressed in the human scalp, we quantified the expression of BQ013595 and BE789145 in various human tissues including skin, hair and scalp. In addition, we included the closest reference sequence gene, *PAX1* (paired box 1). BE789145 was not detectable in skin, hair or scalp and BQ013595 showed very low expression in hair and scalp. *PAX1* was found to be expressed at very low levels in hair and skin, but showed considerable expression in the scalp (Fig. 3). Although *PAX1* is located outside of the associated LD block, the expression data might suggest that *PAX1* confers the AGA relevant effect at this locus and that a regulatory variant within the associated LD block may modulate its expression.

### Example 7: Etiological fraction

The AGA risk allele T of rs2180439 has a frequency of 0.57 in our population based control sample of 383 individuals (before exclusion of the AGA affected controls for the GWAS step). The etiological fraction for rs2180439 is estimated at 0.32, underlining the importance of this locus in the development of AGA. The frequency of the risk allele varies world-wide from 0.03 to 0.86 (Fig. 4). This may account in part for population specific differences in AGA prevalence, but it does not explain the lower prevalence reported in South-East Asian populations (Paik, J.H., et al. (2001) N.I. Br J Dermatol 145, 95-99; Norwood OT (1975), South Med J 68, 1359-1365). It is of note that the chromosome 20 locus did not show linkage to AGA in a recent study (Hillmer, A.M. et al. (2008), Am J Hum Genet 82, 737-743). This supports the value of the association approach in detecting susceptibility genes for complex diseases (Risch, N., Merikangas, K. (1996), Science 273, 1516-1517).

## Claims

1. A genetic marker for androgenetic alopecia **characterised in that** the genetic marker is located in a chromosomal region of human chromosome 20p11 and wherein the genetic marker is selected from the group consisting of a single nucleotide polymorphism (SNP), variable number of tandem repeat (VNTR), microsatellites or short tandem repeats (STR).

2. The genetic marker of claim 1, wherein the chromosomal region of human chromosome 20p11 consists of the region having the sequence of SEQ ID NO:1 at the 5'end and the sequence of SEQ ID NO:292 at the 3' end.

3. The genetic marker of claim 1 or 2, wherein the chromosomal region of human chromosome 20p11 comprises at least one of SEQ ID NOs:1 to 292.

4. The genetic marker according to any one of claims 1 to 3, wherein the genetic marker is a SNP selected from the group consisting of the SNPs comprised in any one of SEQ ID NOs:1 to 292.

5. A nucleic acid molecule selected from the group consisting of:
(a) a nucleic acid molecule consisting of at least 17 consecutive nucleotides of any of SEQ ID NOs:1 to 292; wherein the at least 17 consecutive nucleotides contain an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292;
(b) a nucleic acid molecule the complementary strand of which hybridises under stringent conditions to the nucleic acid molecule of (a), wherein said nucleic acid molecule has at a position corresponding to position 201 of any one of SEQ ID NOs:1 to 77 an adenosine, corresponding to position 201 of any one of SEQ ID NOs:78 to 149 a guanosine, corresponding to position 201 of any one of SEQ ID NOs:150 to 219 a thymidine or corresponding to position 201 of any one of SEQ ID NOs:220 to 292 a cytidine;
(c) a nucleic acid molecule identical to any one of the nucleic acid molecules of (a) or (b) wherein each thymidine is replaced by uridine.

6. A nucleic acid molecule selected from the group consisting of:
(a) a nucleic acid molecule consisting of at least 17 consecutive nucleotides of any of SEQ ID NOs:1 to 292; wherein the at least 17 consecutive nucleotides contain an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292;
(b) a nucleic acid molecule the complementary strand of which hybridises under stringent conditions to the nucleic acid molecule of (a), wherein said nucleic acid molecule has at a position corresponding to position 201 of any one of SEQ ID NOs: SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264 an adenosine, corresponding to position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215 a cytidine, corresponding to position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 a guanosine or corresponding to position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292 a thymidine;
(c) a nucleic acid molecule identical to any one of the nucleic acid molecules of (a) or (b) wherein each thymidine is replaced by uridine.

7. The nucleic acid molecule of claim 5 or 6 which is genomic DNA.

8. A nucleic acid molecule which is complementary to the nucleic acid molecule of claim 5 or 6.

9. A vector comprising the nucleic acid molecule of any one of claims 5 to 8.

10. A host cell genetically engineered with the nucleic acid molecule of any one of claims 5 to 8 or the vector of claim 9.

11. A method for testing for a predisposition or presence of androgenetic alopecia comprising testing a sample obtained from a prospective patient or from a person suspected of carrying a predisposition for androgenetic alopecia, the method comprising determining
(a) the presence of a risk allele of a genetic marker according to any one of claims 1 to 4;
(b) the presence and quantity of a nucleic acid molecule of any one of claims 5 to 8;
(c) the presence of a risk allele within the nucleic acid molecule of any one of claims 5 or 7 as compared to the non-risk allele, wherein said risk allele is **characterised in that** it is an adenosine at position 201 of any one of SEQ ID NOs:1 to 77, a guanosine at position 201 of any one of SEQ ID NOs:78 to 149, a thymidine at position 201 of any one of SEQ ID NOs:150 to 219 or a cytidine at position 201 of any one of SEQ ID NOs:220 to 292;
(d) the presence of a risk allele within the nucleic acid molecule of claim 8 as compared to the non-risk allele, wherein said risk allele is **characterised in that** it is an thymidine at position 201 of any one of SEQ ID NOs:1 to 77, a cytidine at position 201 of any one of SEQ ID NOs:78 to 149, a adenosine at position 201 of any one of SEQ ID NOs:150 to 219 or a guanosine at position 201 of any one of SEQ ID NOs:220 to 292;
(e) the presence of a non-risk allele within the nucleic acid molecule of any one of claims 6 or 7 as compared to the risk allele, wherein said non-risk allele is **characterised in that** it is an adenosine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a cytidine at position 201 of any one of SEQ ID NOs:3, 7, 25, 34, 35, 38, 39, 48, 50, 51, 54, 57, 67, 76, 80, 89, 95, 110, 114, 115, 125, 150,152, 154, 156, 157, 164, 166, 168 to 175, 179 to 181, 183, 185, 188 to 191, 193, 195 to 203, 205, 206, 208 to 211, 213 or 215, a guanosine at position 201 of any one of SEQ ID NOs:1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a thymidine at position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292; or
(f) the presence of a non-risk allele within the nucleic acid molecule of claim 8 as compared to the risk allele, wherein said non-risk allele is **characterised in that** it is an thymidine at position 201 of any one of SEQ ID NOs:78, 79, 83, 86, 87, 90, 91, 93, 94, 96, 98 to 109, 111, 112, 116 to 123, 126, 128 to 142, 144 to 149, 151, 155, 158 to 160, 162, 165, 176, 177, 182, 184, 204, 207, 212, 217, 221, 226, 228, 230, 232, 233, 235, 239, 242, 243, 251, 253, 254, 259, 261 or 264, a guanosine at position 201 of any one of SEQ ID NOs: 3, 7, 25, 34, 35, 38, 39, 48, 50, 51, 54, 57, 67, 76, 80, 89, 95, 110, 114, 115, 125, 150, 152, 154, 156, 157, 164, 166, 168 to 175, 179 to 181, 183, 185, 188 to 191, 193, 195 to 203, 205, 206, 208 to 211, 213 or 215, a cytidine at position 201 of any one of SEQ ID NOs: 1, 2, 4, 5, 8 to 11, 13, 14, 16, 18, 20 to 23, 26 to 28, 30 to 33, 36, 37, 41 to 47, 49, 52, 53, 55, 56, 60 to 66, 68 to 75, 77, 153, 161, 163, 167, 178, 186, 187, 192, 194, 214, 216, 218 to 220, 222 to 224, 236, 241, 245, 252, 266, 273 to 275, 277, 279 or 284 or a adenosine at position 201 of any one of SEQ ID NOs:6, 12, 15, 17, 19, 24, 29, 40, 58, 59, 81, 82, 84, 85, 88, 92, 97, 113, 142, 127, 143, 225, 227, 229, 231, 234, 237, 238, 240, 244, 246 to 250, 255, 256 to 258, 260, 262, 263, 265, 267 to 272, 276, 278, 280 to 283, 285 to 292;
and wherein the presence of said genetic marker, or said risk allele is indicative of androgenetic alopecia or a predisposition for androgenetic alopecia and wherein the presence of said non-risk allele is indicative of the absence of androgenetic alopecia or a predisposition for androgenetic alopecia.

12. The method of claim 11, wherein said determining of the presence of said genetic marker, said nucleic acid molecule or the presence of said risk allele comprises PCR based techniques, RFLP- or AFLP- based techniques, DNA sequencing-based techniques, hybridization-based techniques, single-strand conformation polymorphism analysis (SSCA), denaturating gradient gel electrophoresis (DGGE), mismatch cleavage detection, heteroduplex analysis, primer extension-based techniques, and 5'-nuclease assay-based techniques.

13. The method of claims 11 or 12, further comprising detecting an alteration in the expression of a nucleic acid molecule encoding a polypeptide having PAX1 function, wherein said nucleic acid molecule comprises:
(a) a nucleic acid molecule encoding a polypeptide having the amino acid sequence of SEQ ID NO:293;
(b) a nucleic acid molecule having the DNA sequence of SEQ ID NO:294;
(c) a nucleic acid molecule having the sequence of SEQ ID NO:294,
wherein each thymidine is replaced by uridine;
wherein the alteration of expression as compared to the amount of nucleic acid molecule expression in the control sample is indicative of androgenetic alopecia or a predisposition for androgenetic alopecia.

14. The method according to any one of claims 11 to 13, wherein the sample is blood, serum, plasma, fetal tissue, saliva, urine, mucosal tissue, mucus, vaginal tissue, fetal tissue obtained from the vagina, skin, hair, hair follicle or another human tissue.

15. A diagnostic composition for detection of androgenetic alopecia or a predisposition for androgenetic alopecia comprising the nucleic acid molecule of claims 5 to 8, the vector of claim 9 or the host cell of claim 10.

16. A Kit comprising the nucleic acid molecule of claims 5 to 8, the vector of claim 9 or the host cell of claim 10.


SEQUENCE LISTING

<110>  Life & Brain GmbH

<120>  Androgenetic alopecia

<130>  P1425 EP

<160>  294

<170>  PatentIn version 3.4

<210>  1
<211>  401
<212>  DNA
<213>  homo sapiens

<400>  1
tgttgctctt cccatagagt tcccgacaca ccaataacct cctacctctc tacccatggg      60

atttcggact gtttccagta ctccccatca ttgtagagtt tatttctaaa aagcccaccc     120

acgctataga tgaaagctca aacacagagg ctgagaagtc cagggaccta cagctcataa     180

atgacagggt caggacctca gcctggtcag ttgattcctg gccaaagttg tcgttggccc     240

tttttgaga agctaaatat aactttgctt acaagttcaa aggaaagtga ttcaaaactt     300

gctccgtcaa atgtcttgat tttaggattc acaggtagaa ataaaatgag tgaaaaagca     360

ctgaattcat gtgagaatgg gttccaaaaa atatagcaga t                         401

<210>  2
<211>  401
<212>  DNA
<213>  homo sapiens

<400>  2
tgaatattgg ataggcagtt agcatggttt gtcatgttcc gcaatttctt ttgctttggg      60

agtcaaattt ctgaacacag aagatatttc tagcaatgtt aatagtgttt caggaactta     120

tcatacttca tttttaaaa tgaaaatctc actttgacag tggttttgag aattggatga     180

ggcacatagg tgaaggacct gtaagacttc cctgacgtag tgactcaaat gatgtcaaac     240

attcatcatc atgacttctg aaaaactgta ccactttcca gcttttgttt ccacttttcc     300

cctttaaaaa atttgtagaa gtttaagagg tacaagtaca gttttgttcc atggctgtac     360

tgcatagtgg tgaagtctgg actgttagtg taccccccac t                         401

<210>  3
<211>  401
<212>  DNA
<213>  homo sapiens

<400>  3
gtgaaggacc tggccctggc tcagcaaggt tattgcattt aatcttcaca acaggtatta      60

ttatcatttt ataggtaagg cagctctatt tttcttaggg ctagctctac aaataatttt     120

ttttgtctta gaacaagctg atgtccaaaa tttgcttttt atggaaaatc tgtcgttgta     180

aaaatatgac tctggagaca cattttctga gagcagtaaa taccagaagc ccagccaaaa     240

aacttcagca ccctgttgag attgggaaaa ggcaacattg acttattttc acttaagttt 300

gtgacttaga aaaaaactaa cagtgatggt tttcttcttc tctgttaggc ttgtggctgc 360

aaattctttg catggccgtt tatttcactg aagcgaatga t 401

<210> 4
<211> 401
<212> DNA
<213> homo sapiens

<220>
<221> variation
<222> 31
<223> /replace="t"

<220>
<221> variation
<222> 285
<223> /replace="t"

<220>
<221> variation
<222> 349
<223> /replace="g"

<400> 4
tcttccagaa aactgcctgc aagcaatttc cttaatcggg ttgctaaggg caaccccaaa 60

gaaacaacat accaagctat ctgtctgtcc atccgaatcc caaaagcttt ccttgtgctt 120

cctgctctga ccaaagaggc atttgaggca catgtgagag aacagctttc cagaaaccac 180

acttgagtcc acagcagagc ggaaggggca ggaggcatca ggaccatttc cagatttctc 240

tctggccctg attgtctaga tttgcatcca atcactgcaa gagtgtatac aaaagttgtt 300

agaaagattt gggtttcaca cagagttatg aaaacaaggt agaagcctca tgtcttcatt 360

tcccatgaaa tcttgtgggt gtaaatgaaa catgttttag t 401

<210> 5
<211> 401
<212> DNA
<213> homo sapiens

<400> 5
cagaactggg aaaaagcaaa caataaatgt ccattgttta agccctccag tctgtggtat 60

tttgttacgg cagcctgcgc taagacacca gccaattttg tttctgtttt ccctccctga 120

agtcagtgag gatgggagct ctctaggtag cagtgtgagg gtctgaggga gaccaccgag 180

ggacatgggt ggagagggac ggtcatgggt ctgagaggcc agcaggggcc agatcatgcc 240

tgctttgtag gacacagtga ggggctgata cttcaagatc tgtgggaatg ggagcgggag 300

cccagggagg gctgcaggga aggacttggc tctggctgct gtggagggag agcaggtggt 360

gggaaggcag ggcaaaaagt gaggagtcca caagagacac t 401

<210> 6
<211> 401
<212> DNA
<213> homo sapiens

<220>
<221> variation
<222> 145
<223> /replace="t"

<400> 6
ttaggggagt tttgtgaact tacttcatgt gccattgaat ttctaatagg tgatatctaa 60

ttttaaattg tagtttagag aaaaaatgtt gctattctat tgctgaaaca taattgatat 120

ataaggagag tttggctttg aatacatttc tttctctgta tatatttatt ttagatttca 180

tgagcgagta tttgattctt taaatattga ggacattgca aaacatgatt ttactggatt 240

tttcagaatc gtaaagaaga ataattcttt tatagtttag taactatcta aaataattta 300

gacacttttg accttatttt cttgatgttt ctttgagatg aggaggtagt aattgcaatt 360

tcactttttc tattgaataa aaaactaaat gaagtgtatg a 401

<210> 7
<211> 401
<212> DNA
<213> homo sapiens

<220>
<221> variation
<222> 340
<223> /replace="c"

<400> 7
tttttcttga gcaaggtatg tataagtaag taaataaggg cataatgtca ggtaaactga 60

atggctggaa agtggcaacc aggatttagt tatttggcat tatcacaggt ttttcagaaa 120

cattcaattt ttagcaaaaa acaagtcccc tccaaaattc aatctactgc aattgatttg 180

cacgtggttt aatgctgtta ctagtctata aataatggac tcaaatattt gaagttgtag 240

atgctttcat actgttttaa acaaacataa cttatatgca aatggttaag aataccagaa 300

acattgcaag aatgctcttt ttaaatcctg aagatattgg tttattttaa agcatcaagg 360

atgatttaga tttttggaaa aacagaggtg gcatgaagat t 401

<210> 8
<211> 401
<212> DNA
<213> homo sapiens

<400> 8
taggcgctga tttttgaaaa tgcaatcttt atccagagta tttgacaaat gctcagaatg 60

ctcagaaatg tattaatata cagccagaca gctgtcaaag cagcaggctt ctcccctgga 120

gagttcatta acctcatttc cacttgctga gtaaggagct ctgtcttgcc tgagagggac 180

cacggtacca aaatgtacgg gtttcaagtg aggagtgaga aaaataactg aagtatgaga 240

aaataaagat ttcttgataa ggcccaaaga ctagctcatt tcctagtatg tgttttaaat 300

gcgcgttttc tgtttatttt acttacctat gcatttatct attcatctct tttcaaaatg 360

ctttgaaggt gcaatgatga tatgctattt tatgaactaa t 401

<210>  9
<211>  401
<212>  DNA
<213>  homo sapiens

<220>
<221>  variation
<222>  275
<223>  /replace="t"

<220>
<221>  variation
<222>  398
<223>  /replace="t"

<400>  9
tgaatgtgtg tgtgtatgtg taagtgcacg tgcacatgaa tgactacgta tgtttatgca      60

cttgtgcatg catatacatg tacgtgtgct tgtatgcatg ggtgtgcatt atgtgcgtgt     120

ttgcctgtgt gtgcatgcat gcgtgtgtgt gtgtgtgtgt tctctgtgga gggaggagac     180

tggagattct cttccccaac gttcgaggcc agtttgccaa aaatcttggt gctaactgta     240

tgtccaggtt acagatccac acgcaaatgc agacccaaca acactgaggc tcatcttttg     300

ggtttctccc catcttcttc accatgactc agttctccaa gaaaaatgtt gttgctactt     360

tcaggaacag tccaggatga gagcttaagg tagagtccgg a                         401


<210>  10
<211>  401
<212>  DNA
<213>  homo sapiens

<220>
<221>  variation
<222>  266
<223>  /replace="t"

<220>
<221>  variation
<222>  341
<223>  /replace="t"

<400>  10
gttcacaagg caaataaata acagatgttt gagattatgg atatgctaat tacccttacc      60

tgatcactat gcattatatg tatcgaaaca tcactatgta ccccatgaat atgtcaatta     120

aaaaaatagt ttcaccaaga cccactaaat caacagaatt ttgtggcaac attatgcaga     180

ccccactgtt gagagtaaac gctatttgat atgatatcaa gttccatcag tttgcatcag     240

atcatcagat gagagggcca taagcgtctt ctgttctcat aattatgcag atttattgtt     300

ttgaaacaaa atcaacttct ctctaaaata agcaaggaca ctggggcaac caactattct     360

cagagaagga gtgtttttaa tttttttcag gattcttagg a                          401


<210>  11
<211>  401
<212>  DNA
<213>  homo sapiens

<220>
<221>  variation
<222>  50
<223>  /replace="t"

<220>
<221>  variation
<222>  105
<223>  /replace="g"

<220>
<221>  variation
<222>  233
<223>  /replace="t"

<400>  11
taatacacac acactagaaa cctaattggt tttgaatctt gagcaattgc ttagaaatgt      60

tcatatgatg aaataaaatg ctttgtattg caaactttta tttaagaatg agagcttctt     120

aattccgggt cgttggacaa tatccaaagt caaatggtag agacatatga aagatggtta     180

gtaattaatc acgcctcacc ggtgtgccag gaaagtaaac tcaccaggag ccactctcag     240

tgccttatca aattactaaa acttgacttg ctttcctgga ttagtaaagt gatacactgt     300

tgattctgga aattcgtaac ttaagatgat tcctctgcat agtattcagc aaaccataag     360

atggtatttt gataattttg agtttcatca aaatacagat t                         401

<210>  12
<211>  401
<212>  DNA
<213>  homo sapiens

<220>
<221>  variation
<222>  18
<223>  /replace="t"

<220>
<221>  variation
<222>  73
<223>  /replace="g"

<220>
<221>  variation
<222>  169
<223>  /replace="g"

<400>  12
tgaatcttga gcaattgctt agaaatgttc atatgatgaa ataaaatgct ttgtattgca      60

aacttttatt taagaatgag agcttcttaa ttccgggtcg ttggacaata tccaaagtca     120

aatggtagag acatatgaaa gatggttagt aattaatcac gcctcaccag tgtgccagga     180

aagtaaactc accaggagcc tctctcagtg ccttatcaaa ttactaaaac ttgacttgct     240

ttcctggatt agtaaagtga tacactgttg attctggaaa ttcgtaactt aagatgattc     300

ctctgcatag tattcagcaa accataagat ggtattttga taattttgag tttcatcaaa     360

atacagatta ttatcctgcg tgagtccact gtaatcttag c                         401

<210>  13

<211>  401
<212>  DNA
<213>  homo sapiens

<400>  13
aggaatgagt tcttgatgac tccatgtgtg tcaacaagca gtttacgact tctcctttaa      60

aacagaaaac gaacaagagt gtagttttga aaagtaaatt tatttattta tttattttta     120

cttaggctta tataattttc tgttctgtaa gagagatctt ttagggccca gggaattatt     180

ccataccttg gggacccggg gaaagactgc gtacctggcc aagactgatg atcaaccaag     240

tgggatgctt tatttcctct ttagttagaa gtttcaggat caaagtgagc ctttgagcta     300

tttgtcattt tccctctaaa tatttttgtc acacacacac aatttgtata gtggcttctt     360

cagaagcctg tggcaatcct tcctggctgt gaaaacagat g                         401

<210>  14
<211>  401
<212>  DNA
<213>  homo sapiens

<220>
<221>  variation
<222>  348
<223>  /replace="g"

<400>  14
aacaacttct ggatatggat aatgttaatt cttgtccatg atacaattgg agaaattgag      60

gcttaaagag atgaagtcac ttttcaagtg ctacatagat gctaagtgat aaagaagaaa     120

ttcaaaccca ggactcttga ttgcaggccc ccagctctta tcacagacac tatacaaaca     180

ggaatacttt gtgttaccct gtgttaatgg ttctcttatt ttgctgtgca tcagaatacc     240

ctggaaggat gactaacata cagattgcca ggctcagcac ctgctgtgtc taagccagga     300

tatctagggg catttctagt aagttccagg taatgctgat gctgctgatt tagggacaac     360

actttgagaa atatggctgt gtattatggg tctatttgaa c                         401

<210>  15
<211>  401
<212>  DNA
<213>  homo sapiens

<400>  15
ccctgaggcc ctcagagtca tacacagagg aagagcttgg gtcagacttt ggccttttct      60

gcttgtttca tcaaaacaga aaccacaagc tttatcacag cctcaaaatg cattttctct     120

cttctttggg gttaaaaaca aatttttctt caaagaaagc atattgagat ttttaagtca     180

agttcacaat taaaaaaaaa ttttcttgaa agatgtgtgt attgtcttaa catgggttcc     240

ctttgttaac acctcccagt ggatttattt ggcatgaggg atctaggggc aggaccctgg     300

acttcttctt agacaaccta acctctttgg tccagaacta tctcaaggtt ctgctaccaa     360

catgttagaa gcaaaaggcc caggaaacaa cccagtcact t                         401

<210>  16

<211>  401
<212>  DNA
<213>  homo sapiens

<220>
<221>  variation
<222>  378
<223>  /replace="c"

<400>  16
gaattgaata accatcataa ggaaacaata aaacatgaag tgactgagac cagatgatca      60

ccactgggca cacgtggtct ctgtagcagg ctcagggagc ccagggttgg ctttcagttt     120

cctgcaacac agagcagtcc cttcagcact ctgccaataa ggatcccatc ggttaatgag     180

acgagcttca actaactggc ggaggctcgg agggtggggc acccacgtta cattttctcc     240

ttgcccacaa ctctttgaat tttagtctgg ttgtcaattg ttctagcatc tcttccccct     300

agtgtgctgt ttctcccctc ataaaaacag ctacagagct gaggtctgaa atatctggag     360

tatataaaga tggtgaggat tggaggggtt catggtgctc t                         401

<210>  17
<211>  401
<212>  DNA
<213>  homo sapiens

<220>
<221>  variation
<222>  319
<223>  /replace="g"

<400>  17
cttgagtttt aaaagttgtc agaagaattc tgagaagatg atagagtagg aaataccaga      60

aatccctctc cccatctaga caataatagc attggcaata tctgtctgac atgattattt     120

tgggattctg gagtctattg aatatttgca acttctaggg gaaggcttgg atggtaaatt     180

gtttaatttt gatcattttc tgctcctagc acagtagctg ctacctatct ttcacctctg     240

gaccctcctc tcttgagcag gcagcatgca tgtgttcctg gagaagtttg cacacagctt     300

gttggaggca gggtgggcaa aaggatcctg ccctccaaat atcagggatt tgtactctga     360

ctgctgatgg ctgcttctga tcacaggggt gctggcaatg a                         401

<210>  18
<211>  401
<212>  DNA
<213>  homo sapiens

<220>
<221>  variation
<222>  265
<223>  /replace="t"

<220>
<221>  variation
<222>  361
<223>  /replace="g"

<220>
<221>  variation

<222>  366
<223>  /replace="t"

<400>  18
acagattaaa tccctatttg aaataaaagt agctgtttac tcatgtaaat gcattgtttt      60

tccttgacta cttaaaggca aacagattaa aggacatgga ttgtctaaag aggctttggt     120

gaagagtatg ttgaaattcc tttttttttc cctactgcaa tccttttcac caattcattt     180

aggctttcta tggcttttaa gagatgagag agaaaagttt ttgtttctaa ttaacaaggt     240

ttcacaagca ggcagaggca ttacagtttc catttaaaaa gaaagaaaac ctagaaaaat     300

ggtgacatgt tcctccaccg gcaggatggg ggtaagggac aactttgggc atgccaagat     360

actttcttca ctgagagatt tattttaaaa tattacttta t                         401

<210>  19
<211>  401
<212>  DNA
<213>  homo sapiens

<220>
<221>  variation
<222>  137
<223>  /replace="g"

<220>
<221>  variation
<222>  297
<223>  /replace="g"

<220>
<221>  variation
<222>  302
<223>  /replace="t"

<400>  19
tgactactta aaggcaaaca gattaaagga catggattgt ctaaagaggc tttggtgaag      60

agtatgttga aattcctttt tttttcccta ctgcaatcct tttcaccaat tcatttaggc     120

tttctatggc ttttaaaaga tgagagagaa aagtttttgt ttctaattaa caaggtttca     180

caagcaggca gaggcattac tgtttccatt taaaaagaaa gaaaacctag aaaaatggtg     240

acatgttcct ccaccggcag gatgggggta agggacaact ttgggcatgc caagatactt     300

tcttcactga gagatttatt ttaaaatatt actttataag caaaagggca atctatcatt     360

ggattttcca acaccatgtt ggtgttttaa ttaaaatagc a                         401

<210>  20
<211>  401
<212>  DNA
<213>  homo sapiens

<220>
<221>  variation
<222>  378
<223>  /replace="g"

<400>  20
ttgcctccaa gttctggcaa ttatgagtaa agctcctgta aacattaatg tgcaggtttt      60

tgtgtggact taagttttca actcctctgg gtaaatacca ggagcaccac tgctagattg 120

tatggtaaga ctatgtttac ctttgtaaga aactgccaaa ctctcttgca aagtgatggt 180

attattttac atttctataa gcaatagatg agggttcttg ttcctcttca tcttgccagc 240

atttggtgta gccagtgtct tggatttttt tccacagaat gatattttta catatccctg 300

aacaagggat gatgtttaaa attcttagca atgagtatca ccagggcagg agctcacaga 360

acagatgtgg atattcaagt gaagattggc tgatcactgg c 401

<210> 21
<211> 401
<212> DNA
<213> homo sapiens

<220>
<221> variation
<222> 23
<223> /replace="t"

<220>
<221> variation
<222> 27
<223> /replace="g"

<220>
<221> variation
<222> 282
<223> /replace="g"

<220>
<221> variation
<222> 371
<223> /replace="t"

<400> 21
agtgctgctt gatgcctatt acctttatgt aaagacacag ccagctcacg tgagctctta 60

ggaaggggga tagagaaata aataccctga cctcatcttc ctgccctctg ggctgctggc 120

ctccaaacca gaagcagggc actggagctt accaacatgg tacacaaagg ctggcatcct 180

ggagctcaga gcagaggaga ggaggtcaga cagtggatct agaggggcag acaaaaatat 240

ccagaacaag tttcactctc catgggcctg aactagtggt tacaattgct ggaaggtaga 300

aagtgttagg gggtgttcag gtatgggatc cagatgaagt gggagagaat tttagtatgc 360

tttgtgaaga cttcccatga tttgaaattg tgtaaggact g 401

<210> 22
<211> 401
<212> DNA
<213> homo sapiens

<400> 22
ccagagttcc catgattctg cacagaggaa ggttctcata gtaaggatct gatgctgtgt 60

ctgcaggctt ggcttggcca tgggagtatg ctctgtccat atgcagaagt cactcctgga 120

gcagctctca accagtgact gagagataaa taacctggct tcctcacccc tcaggtgagc 180

tgtgttccac acagtctacc ggattcctcc agggggatcg agcctcagct gctcaattat 240

tacctcagta ataacttact aattaatgca cctggtgttc actcctcttc tccatttcac 300

ctctccgttt acctgcgtgc ttcctgggat tgcatctcaa aatgaactaa ggaagtgaaa 360

tccttacctt agagcggaat tctgagggaa acccaaacta a 401

<210> 23
<211> 401
<212> DNA
<213> homo sapiens

<220>
<221> variation
<222> 173
<223> /replace="t"

<220>
<221> variation
<222> 240
<223> /replace="t"

<220>
<221> variation
<222> 280
<223> /replace="g"

<400> 23
tcctttgtaa gtcttttgct cattcatcac caaggttaga agtcatttct ctgtgaaccc 60

ttccctatca gagcaggtct gagcatgtgg tgtccgttca gtgggggtaa tgtgtgggca 120

gctgaagtct gattcgtacc ttccagacca aaagaaaggc actggttcca gcgtagtgtc 180

ccatagcaga aacatggctc ggagctcagt ttaaaggtgt tccttgaatg aggccaatcc 240

tttttggggg aggggaaggg gaaggagggt ctcccaaccc ccgccttttt tatttgagaa 300

agaatctcat tctgtcaccc aggctggagt gaagtggtgc gatctcaact cactgcaacc 360

tccacctcct gggttcaaga gattctcctg cctcagcctc c 401

<210> 24
<211> 401
<212> DNA
<213> homo sapiens

<400> 24
gtaatcactg tgatctggga gatgcgtttg gactggaccg gccagtgagg ggcctaccta 60

cctgcctaga tgaaaactat cacttgcaca gaaatacatg gaaagatgaa gaggcataaa 120

gttaatggag attaattgta ccagctttgt aggagcacag gaataataac catttatgga 180

acaaatattt ctatgtattt tttttcaaac tgtaattaag ggtaccattg tccccaattc 240

tttacccctc cctttatctc tatcctttgc caagtgactt gcagtccccc acctctatgg 300

gaggagtata tgtcctttat ccttgactct gggcgtgacc ttgtgatttg ctttggccaa 360

tgacaccaag gggacagtga cagtgttcca gttctgatcc t 401

<210> 25
<211> 401
<212> DNA
<213> homo sapiens

<220>
<221>  variation
<222>  74
<223>  /replace="t"

<220>
<221>  variation
<222>  232
<223>  /replace="g"

<400>  25
ttttatgtat caacaattta agttctactg ctttaagatt atatggacat taaatgtaca      60

gaaaaataga aaccaacaaa agataaataa attaaataca tttttaaaaa agtctcgcga     120

gttgtggtgg cttaatattg ccgtgagcta atgccttgag ggctactctc tctggagtgg     180

gaatggagct ctcagtaatt cgacagtttt ggccagtggc ttgtcacacg gattgtgcat     240

tcacctggct ttaggaggaa gaggaggcat ggcagccctg ccttcatctt cctctttact     300

ttgccctcac tatcactgtt aacttcgatt catggatgct tttcagggat ttttttttct      360

aagccaccta gccaattcga ggggtttagt gtacttaaaa c                         401

<210>  26
<211>  401
<212>  DNA
<213>  homo sapiens

<220>
<221>  variation
<222>  237
<223>  /replace="c"

<400>  26
ggattttttt ttctaagcca cctagccaat tcgaggggtt tagtgtactt aaaaccagat      60

agtataatcc ataaacccac taaattgggc acatgtgagc tgcaatatgt cacaagatgc     120

agaaaaccag ccctgagtga gggcactgcc ttccgcagct gtcctctgga tctttcctgt     180

aatgtcagtg acacatgacc gctggcatgc agcctgctaa gggacggtgt ccatctgcat     240

atcaaccaag acaaaagcct ggctcatcat ttatttagag aaagcaaaca taaatggaag     300

aaattatatt ttcctcctat tccatagttt atcatgtctt gccccacctg ggtagtgtca     360

cttgtcctga agatggtagg agtgagggaa aagaggtgag c                         401

<210>  27
<211>  401
<212>  DNA
<213>  homo sapiens

<220>
<221>  variation
<222>  89
<223>  /replace="g"

<220>
<221>  variation
<222>  308
<223>  /replace="c"

<400> 27
aatggatgta tggagatgtt tctctccagc aaagataagg aaatggctgg aagactgcga 60

aagaatagat gtgccacttt ggaagttgac ataaaaatga atgatatttt tactttttgg 120

aaagccaagc aggtcccaga gtcgtggggc agtacttggc ctcatctctg aggccaccca 180

ttctgctgct ggcagccaca gtgatgtacc cttggcacta tgtcgctccc cctacttggc 240

atcctcagtt tttctaaact catgggtgag agacgaatga cgtcagaggc ccaaaccttt 300

ggatgctgat ctaaaagcat caaccaaaac ccaacacccg agcttcgtaa gttcccattt 360

gaagcaggag cagtttttat agcaacagac aaaggattgc t 401

<210> 28
<211> 401
<212> DNA
<213> homo sapiens

<220>
<221> variation
<222> 24
<223> /replace="c"
/replace="g"
/replace="t"

<220>
<221> variation
<222> 153
<223> /replace="g"

<400> 28
ctaagacagt cttttacaat ctaacaaaaa cctttctgct gtggaagttc tgtggaaagg 60

gtcacagatc atgtgcagtc acacaacact ctttggatta tcactgtcat gaaggagcaa 120

tgggggagac caactcctcc acgcagggct caagatgtgt gcatggtagg ccaagagggc 180

aagtgggtgg ggaggtggaa ggggtcagga aggaggcaca gacaccgtca aagcttaagg 240

agaacggacc gttgacacat ggaaatggat actgtgacac ttagatttat cgtcaggcaa 300

actgttcacc acaaacccaa agttcctcta aacaatgcct aggaaaatct tccagaagtt 360

ttgaggatct ggtgaccata ttttcagaat caaaagttgg g 401

<210> 29
<211> 401
<212> DNA
<213> homo sapiens

<220>
<221> variation
<222> 137
<223> /replace="c"

<400> 29
taggtaaata tttacaaact gaaggtcaaa cacaaatgtc tactttacaa ctactatact 60

gcattcataa tttctctcct cattttatac tctgatgaaa taccaacttg gtcatttctg 120

tgcattgtca agagggtcga agtcgaagca gaagcaagca gctggctggc ttagcttatg 180

gttcctctgg cacaggaaag tggtgagagt cattattctt attatccagg agttaacgga 240

tttttgcatg actgggctga gtgtatgttt attttctggc ccttgataat tactgtattc 300

ccattttcaa tatgaatttg tttctatcca atggatggtc aacagtagcc actgtaattc 360

tttttgaggg gctatatatg caccattatt tttcaaactt g 401

<210> 30
<211> 401
<212> DNA
<213> homo sapiens

<220>
<221> variation
<222> 93
<223> /replace="t"

<220>
<221> variation
<222> 260
<223> /replace="g"

<220>
<221> variation
<222> 274
<223> /replace="t"

<400> 30
ttacataaag tcaggctgac atgggttcaa attctggctc ttcacaataa ccctataagg 60

caaatactat tcccgtttta cagctgaaaa acctgaagtc aggaggtcag gctaagttaa 120

tatgtctatg gtcttatagc tagtgggtgg gagatgagaa tgaatccagt ctggctgaag 180

agtccatact tctggcctcc gtcatagcct gggcttccca aagcttgtgc ttttgttcct 240

tattggcaac aggccatcaa ctgcattcgt tacgtaaggt tattatgagg agtaagggag 300

ttattaaaag tgtctagcat ggttcctagg acatagtagg tactcaaata tttttccctt 360

tctgttacta ttaacacttt ctttataaaa taaaaattgg c 401

<210> 31
<211> 401
<212> DNA
<213> homo sapiens

<400> 31
ctaacactga gaaactccag acagatcctg aagttaacaa aacatgaatc tattatctac 60

ttaaaatgac tgcaataaag gcttcttgat ctataactct agttttgcat gaagatcaac 120

aagctttcac atggtaggtg gagtaaatgt gaaggtgaac agtgtcagat gtgaaagata 180

cagtgggatt cttgccatac gctcttcagt cttaaacctt ctgcaggtga tccaactaca 240

ccccaggaat taagaagcag attataaacc tcaggccttt aaagggcaaa aaggtctgaa 300

ttagtccaaa agttcagggc aaaactctaa atgactttta gttacaacgg aaacccactt 360

ggaaagttgc ttttcttctc caatagcttc ccaagaaagg g 401

<210> 32
<211> 401
<212> DNA

<213>  homo sapiens

<220>
<221>  variation
<222>  19
<223>  /replace="t"

<220>
<221>  variation
<222>  70
<223>  /replace="t"

<220>
<221>  variation
<222>  115
<223>  /replace="t"

<220>
<221>  variation
<222>  126
<223>  /replace="t"

<220>
<221>  variation
<222>  147
<223>  /replace="t"

<220>
<221>  variation
<222>  223
<223>  /replace="c"

<220>
<221>  variation
<222>  340
<223>  /replace="g"

<220>
<221>  variation
<222>  360
<223>  /replace="t"

<220>
<221>  variation
<222>  384
<223>  /replace="g"

<400>  32
taataataat agtgaaggcc tggactgggt agaggaagaa actgatccaa aacaaacgtg      60

caatgaggcc tcagccaatt cctggggggc agatctggct gtgtacagca gttcctgaat     120

aatgtcggtt cattcgatgt tctttcctta taacgttgat gagaaaacaa ttgatttgca     180

cctggggcca ccatctgtgt gtatttgcac aacctcccca taactgcatg ggttttctct     240

gagtactcta gtttgctctc tcattccaaa gtcatgcaca ttaggttaat tggcatgtct     300

acatggactc agtgtgagtg aatgtgagca tgggtgtgaa tgtgctctgc catgagagac     360

tgtcctgttc agggctggtt cccacctcac tccgtgagct g                         401

<210>  33
<211>  401
<212>  DNA
<213>  homo sapiens

<220>
<221> variation
<222> 8
<223> /replace="t"

<220>
<221> variation
<222> 62
<223> /replace="g"

<220>
<221> variation
<222> 84
<223> /replace="c"

<220>
<221> variation
<222> 221
<223> /replace="t"

<220>
<221> variation
<222> 245
<223> /replace="g"

<400> 33
ttctttcctt ataacgttga tgagaaaaca attgatttgc acctggggcc accatctgtg 60

tatatttgca caacctcccc ataactgcat gggttttctc tgagtactct agtttgctct 120

ctcattccaa agtcatgcac attaggttaa ttggcatgtc tacatggact cagtgtgagt 180

gaatgtgagc atgggtgtga gtgtgctctg ccatgagaga ctgtcctgtt cagggctggt 240

tcccacctca ctccgtgagc tgctgggatg ggctctggcc acccatgacc atgaactgga 300

aataagcagg taaagaatta tcttacttgg ttttattaat ctttcttaaa tgtatgtata 360

gttcacattt atttcaatgt ttaatattag aaatgttttg c 401

<210> 34
<211> 401
<212> DNA
<213> homo sapiens

<220>
<221> variation
<222> 45
<223> /replace="t"

<400> 34
gtgtcattag aggtacagaa acacactttt tttttctatc tgtggtgtct caaattgtta 60

tggtggtgtt attttcttct aaatgtcttt ttaaggaaag aaaaataaaa atttaaatta 120

ttagcaacga tttttgccat tcatctttat attatgcatt gctaatttta aacacaagtg 180

tgtttaatta acttgtatgg cgactcacca aaatatacat tttgtatttt attactcatg 240

cgtgtatagg tttttgttct aacaagaaga gtaaaaacac tgcacaaaac taactcaaat 300

gattttattt cacttattga tatgtgcata ttctgccaat gttctctacc tttggcatac 360

tggtgactaa cagagaactg aaaggaaatg taactatctg a 401

<210> 35

<211>  401
<212>  DNA
<213>  homo sapiens

<220>
<221>  variation
<222>  50
<223>  /replace="t"

<220>
<221>  variation
<222>  195
<223>  /replace="c"

<220>
<221>  variation
<222>  259
<223>  /replace="c"

<400>  35
aaaaaagttt caaaggcagt ttttaaaatg cttagttaaa ggctaacatc agtatagttt     60

aaaaggtaat tgatctatag atgaactaac aactaccaac agcatctctt ttgtcagtct    120

atagctgaca gatccctcct tcttatgcct acaagtctga catctactga agtttcatag    180

attggctgaa agcagtagtc cggaaaactt tagtcccagt tccagagatt ttcattctct    240

ataccatcag ctctttggaa actggtgcct tttaggctct gaattgacat gggttcaagg    300

gcatctcact ctgttgacct tgtaccttga atttcctcaa cattttcttt gtgatttgat    360

atcaatatta gttctctagc tacctgcatt acaatagtcc t                        401

<210>  36
<211>  401
<212>  DNA
<213>  homo sapiens

<400>  36
atatagggtc ctccatagta tttgcaaata attttaaaat ttccatattt gtaatttgcc     60

attttaattt ttctaatggt gttaacttat atcttctctt tttattttaa aatttgtatt    120

gatacataat atttatacat atttatggta ttcatgtgat attttgttac atgcttaaaa    180

tgtgtaatga tcaagtcatg gtttttaggg tatccattac ctcatgtatt tatcattttt    240

atgtattgtg aacatttcaa gtcctctttt ctggtatttt gaagtatgaa tatagtgttg    300

ttaactatag ttaccctact ctgatatcaa acattagaac ttatttcttc tatctaactg    360

tatgttcgtg cccattaacc aacctcccac cacacaccct t                        401

<210>  37
<211>  401
<212>  DNA
<213>  homo sapiens

<220>
<221>  variation
<222>  28
<223>  /replace="t"

<220>
<221>  variation

<222>  80
<223>  /replace="t"

<220>
<221>  variation
<222>  400
<223>  /replace="g"

<400>  37
cctggatgtc taagtctctt gctggacctg agaagttttc agctattatt ttgttaagta      60

gattttctat gcctttgacc ttctcttcat tttctgtaac attcaaaaat caaatattgg     120

tcactttatg gtatcccata tgctatctag gcattattcg ttctttttta tttagttttt     180

aattcatttt tgtctgattt gatgatttca aaagacctgt cttcaagttg tatgattttt     240

tcttctgttc gatcttatct cttgttgaag actttgtatt tttaatttga ttcaagaaat     300

tccttagttt caggattttt ctctggttct tttttataat acctatctct ttggtaaatt     360

ttcattaata tcttgtgttg ctttttctga cttctttgta t                         401

<210>  38
<211>  401
<212>  DNA
<213>  homo sapiens

<220>
<221>  variation
<222>  160
<223>  /replace="t"

<400>  38
atggtgtgct gtagctgctt aaagcccaca gcttgtgtgg gactcagcag gaacttctgt      60

tctgatgtaa tgctatcgtg tgatgcccag acatcttctt ctgttactct cagggcttga     120

gagggtagat gggctctctc atggctagga ttgctagagc tgcagtggaa atgtggacca     180

ctggggctct ctcacttata ctttctccac ttggggagct cctctgggat cccagcttat     240

cccagctgag caggctactt cactttgctc ctctaccttg ccttaggtgt tccctgtcac     300

ttctctgttg aattatagtg ttctttctta gatgatctat tcaaagtgtg gttatctact     360

cactattttt gtttttctct gtgcaggagg taccagatgc c                         401

<210>  39
<211>  401
<212>  DNA
<213>  homo sapiens

<220>
<221>  variation
<222>  274
<223>  /replace="c"

<400>  39
agcaggctac ttcactttgc tcctctacct tgccttaggt gttccctgtc acttctctgt      60

tgaattatag tgttctttct tagatgatct attcaaagtg tggttatcta ctcactattt     120

ttgtttttct ctgtgcagga ggtaccagat gcctctagtc agccgtctta tagcccctcc     180

tatatcgttt taatttttgt cttggtcaaa cttgcaaaag gttgtctact ttgttgatgt     240

taaaattttt tcgttttatc gaattttgat tttgtaattc acacttcagg cttttatatt 300

tatccattct gtccttgtag cttagttgga tttattttgt tgttacttcc tgtgcttctt 360

tgtttgacgt attagtttac ttatttacat ccccaccagc a 401

<210> 40
<211> 401
<212> DNA
<213> homo sapiens

<220>
<221> variation
<222> 247
<223> /replace="g"

<220>
<221> variation
<222> 290
<223> /replace="g"

<220>
<221> variation
<222> 339
<223> /replace="g"

<220>
<221> variation
<222> 400
<223> /replace="c"

<400> 40
ttgagttgtt gggtaccaga actgataggt gtcatgtatt taagcccatt tgaaaaatac 60

atttcaataa tggagtgaga ctctgtctca aaaaaatttc aaatgtcttt tctcaattga 120

aaaaacctat tatttctaga tagataagta aataaccttt gcaaactcga gccacacttt 180

tcccctttct tttaaaattc tgcttaatat aattgaaaac tgaagtctga ctctccaagc 240

ctataaagga taaaagagag acaggataga tagaaataac tggcaacgaa gagttactgg 300

gctttggctc catgggagac aatagtggtt tcatagaaag caatggtgga ctcttagtca 360

actgaagctt gggtggaact tagctgcata attaagaaga t 401

<210> 41
<211> 401
<212> DNA
<213> homo sapiens

<220>
<221> variation
<222> 121
<223> /replace="g"

<400> 41
gtttctggcc tgtgcttcta caaaacacac agcacatatt ttctaaccat aattgaaacc 60

attttaaaat agtgtttttg gaggagtgaa tctatgtgaa acttctgtaa gctatttgta 120

agacagccgt gaaaatcctc catgttctat gcattggatt ttttttcctc cttactcaca 180

aggatcatct tcaggttaat gtcacagaaa ttgggacaac ttagtgttcg agaaatagag 240

aaatagctca aggagctatt agataccaac ccataccatg ttgttttctg agggtgggag 300

aaactagaag aaagtttgac attactctca ttccatgaga caacctcaag aacacaaacc 360

caaactatcc aggtaggact tttagtttag ggagaccacc a 401

<210> 42
<211> 401
<212> DNA
<213> homo sapiens

<400> 42
ttaagatgac gtatcctgta ggatggacta aataatgctg cagcaaggaa tacttcccaa 60

attgtcatgg cttgcgataa gtcttatgtt tttctcatgt tttatgtcca ttatggatct 120

tgccctggta cccagcctga tgcagcagct gctctctgga tcatgcctca tcgctgtggc 180

agagcttatt gcaaatgagt gactctacct gggagtgaca cataactcca ctcacaattc 240

attgtccaaa ataagtcaat gaccacacct aacttcagtg ggacaaagaa acactatcct 300

actctgtgct tagaaggaag aaagatgaaa atatttggtg atggctctat gactaccatg 360

gatagctttt attttaatat tgtacattaa tagcgctctc t 401

<210> 43
<211> 401
<212> DNA
<213> homo sapiens

<220>
<221> variation
<222> 103
<223> /replace="g"

<220>
<221> variation
<222> 263
<223> /replace="t"

<400> 43
gagtatgcaa gtacatgcat gtgtgtactt cgcacactgt tatctatttt aggctcaaat 60

agatgtatct atggttgaga aattcccatt aaatattggg gcaaggttca gatgctgtag 120

atatttgttt acattttatc aaatgctttc ttacatgtga acccctcaat ctctgagaaa 180

tcacctaagg gaagccatgg gtctgcctcc tccaggtttc attattgcat ggaatgtgag 240

gggatttctg tcttaattaa tgcataagcc agctcttctg agtttgggtg gatatccttg 300

aggagaaagg tagctaaaat tagcagcctc tactctcttc tgtgttcctt ataaaaaaaa 360

agagaaactg tctcatcttt aagaatggac cagttatgaa c 401

<210> 44
<211> 401
<212> DNA
<213> homo sapiens

<220>
<221> variation
<222> 90
<223> /replace="g"

<400> 44
ttgttctcac caaaactcat gttgaagttt gatttctcat tgtggcagtg ttgaaaggtg 60

gaagctgatg ggaggtgttt gggtcatgaa ggctctgtcc tcattgatgg attggtgcca 120

ttctcacagt agtgacttct ccctctggca agactaggta agttcctaca ggaatagatt 180

agtttctgaa agagtgtgtc gttataaagg cagcacgctc ctcagctttt ctctcttagc 240

acgcgtccat ttcccttttg accttctctg ccatattaga ggcaacatgg aaaccctcac 300

cagaagtcag ggctttgccc ttgaactttc caccctgcag aaccatgagc taaataaacc 360

cattttcttt ataaatcact cagttctggt attctgttat a 401

<210> 45
<211> 401
<212> DNA
<213> homo sapiens

<220>
<221> variation
<222> 45
<223> /replace="c"

<400> 45
tatcatgagt atatctaacc ctatgtttac tgtatttgtg ctatgatatg aatatatttt 60

gatttagtat ttgatcagtc tttactgatt gttgagaatc tactaactca gtgcttctca 120

aagtggtaaa gggctagttt ttcaaaattt cttatctctc ctggattggt gcatagtctt 180

accacatatg actcatctcc ggcttgcacc acttgctaat caccacgtaa gtgtggcagc 240

atctaaacaa atctgtcttc atttcaagga agagaacgct gataatgcac atggatgttg 300

caacaatgtc aaattgctat gaaaatttct aaatgaattt tctgctcaat tgcctacctt 360

gttgcaaatt aataacaggc agttaacata actggtagtg t 401

<210> 46
<211> 401
<212> DNA
<213> homo sapiens

<220>
<221> variation
<222> 314
<223> /replace="t"

<400> 46
acagggtcag gtgaggaatg tgaatacttt taggatgggg cctttcctcc tagatgtttc 60

taggtggtgg tttccttatg aaatgacgac accccagagt catgaccaaa ggctcatcac 120

agtcagcttg aaggatgccc tgtgtcagct cctacttctg ccccactgtt gggctggcac 180

ctacttcttc tgaaagacac gcctcgggtg tcccctctgc ggtggcacaa agtggctctc 240

agtgaacctt acaataataa attataaatt aataagtaat tgatagattg aataaatgaa 300

tgtttggaga gttcgtcttt ggttagatac attttcaata tgggtttatc tgttgatcat 360

tcagaaataa aagcgatatt taactgtgtc tcttatttgg a 401

<210> 47
<211> 401
<212> DNA
<213> homo sapiens

<220>
<221> variation
<222> 296
<223> /replace="g"

<220>
<221> variation
<222> 302
<223> /replace="g"

<400> 47
aggggaaaat gattaagtac tgggttattt gagaaaccat gaagaccaga aggaagtgac 60

acaagttttc aaatgctgaa agaaaagtct tatcaaacca ggattttata ttcaacaaaa 120

aattccttca ggaatgaagg attaacaaag atatagtcaa atgagggaaa actaagagag 180

tttattgcca tcagacctgt gctaaaagaa ttgcttaaaa aaattcttca gacagaaggg 240

aaatgataag aaatggaact tggaacttca aaaataaaag aagatcaaca gaaatagcca 300

cataaataga gtagactatt tttctccttt tgagttcttt tttccttttt tctatttgtg 360

taaatttagg gagtacaagc acagttttgt tacatggata c 401

<210> 48
<211> 401
<212> DNA
<213> homo sapiens

<400> 48
tagagtgtcc caatttgaat gtcagatctg gttacagata cattgagata atggtccctg 60

aaccctgggc aactgggcat gttctggggt tgctggagtc cttgttctgg tcacttccag 120

ccatataaac atactttttt ctttgctgag gaactgatct gtgctgagga atgccacatc 180

tgggctctgt tgaagaaggt cggcaaacag aagagagaga tggggcattt cttcctccac 240

tccctccctg ccttggggtc acaattccag cagcagttgt acccttccct tctggtgggc 300

acttcccctg cagccctctt ccttagctcc tagtatctcc tagggcagta atagctccaa 360

actgctccaa actgctgtta gtctttgggg gcctcccaac c 401

<210> 49
<211> 401
<212> DNA
<213> homo sapiens

<220>
<221> variation
<222> 124
<223> /replace="g"

<220>
<221> variation
<222> 153
<223> /replace="g"

<220>
<221>  variation
<222>  187
<223>  /replace="t"

<220>
<221>  variation
<222>  315
<223>  /replace="t"

<400>  49
ttcatctgtt caagaaacaa caatgaaaaa aaatggtgat taaatgcaac cacacctaaa      60

taaggtagcc aggaggtaga accagcttct ccatgaacag gccacatacc atctcccggg     120

gctacctcca gtgtccagcc tggctgtgct caagccctca gggcttgctg gactctcctg     180

gcagtggggg gtcaggactc gagtaggttg gatgctgata gcagagagtg tgattctccg     240

gcctgatgac ccagaatcac tcccaggact ggaatcccag ctctcccagg aggaaggagt     300

cgtccctggt ctcccgcatc ccccaaatgc tgattctgtg tctgcatgtg gaggggtaac     360

aaacaaacaa caaaggacca tctcaattaa cctgccacac a                         401

<210>  50
<211>  401
<212>  DNA
<213>  homo sapiens

<220>
<221>  variation
<222>  91
<223>  /replace="c"

<400>  50
ggatatcaga aaagtgttag aaagaggaaa gtagagtaaa tccaaacctc tctctctaca      60

ctctccaaac ctgtccccca cacccacccc acccccttgt tgccaagttg taagatcttg     120

ggctgacttc caagttccca aaattcaagt gtggaaaaag ccaactaatc tcctggaaaa     180

atctcctgat gtgtattttt ctttaacaat ctgggctgat tttaaactta gcccacagta     240

aactgtgctc aaccggcatt atcattatgg acacttgctg ctgtattttc caaggatctc     300

ccctcccccg ctggtattgc taccagatac agaatggaat ttgtcaggac aagccatgta     360

ttactactaa accattaatt cagttagtgc attaggagtg g                         401

<210>  51
<211>  401
<212>  DNA
<213>  homo sapiens

<400>  51
ctgcagacat gtatgcacta gcttcccttt ggacactctt tttttaaaaa aatgaggtga      60

catggacata atttaaaagt aaccatttta atgtgaacaa tttagtggca ttttgtacat     120

tcataatgtt gtgcaacaac cacccttact aattttaaaa cattttcctc accctaaaag     180

gaaaacctgt acccattaag caattgctcc ctatttctcc cacctctggc accaccaatt     240

ttatgttctg tatcctggat ttacctattc tggccacttc acataaatat aactacacaa     300

tacataacct gttgtgtctg gcttttttta cttagcataa tattttcaag actcatccac 360

attgtatcag gcatcagtac tttgactgga taatattcca t 401

<210> 52
<211> 401
<212> DNA
<213> homo sapiens

<220>
<221> variation
<222> 346
<223> /replace="t"

<400> 52
agaatatgta ttgggtggag tattctgtgt gttaggtcta gttgttcaag tcttctattt 60

tcttattcgt tttctgttta tattttatcc atgatttaaa gtggggtatt gaagtctcta 120

ttactgcaga actatctatt tctccttcaa ttttgtcagt gtttgcttca tatacttgag 180

gtgttctgtg aaagggctgt gtatacaaca tgatctccaa atgcagattg agccaagaaa 240

tgaaaaaatg agtcagacaa atccagtttg tcagtaaatg gtgatttatt agaggactta 300

cagacagaaa tgtggtcttg ggtggctgca agccaggaag aattcggcag tgcaaccctc 360

taggcccagg gcttatatat cttgggtggg gggtaagtac a 401

<210> 53
<211> 401
<212> DNA
<213> homo sapiens

<400> 53
agaaaaccca atgcaaggca aaagagcttg gttgagtcac cccaagtgac aaagtcatac 60

ttaagcttct ggtttcagtc agtcatcttt gctgagaggt tggagaacac atactagtta 120

agacaaataa atagcaattt aaactttgga catgctttct tctcttagct atgggaggga 180

cacatgtttg gagaaaaatc gaagcatgtc aggagggatg taaaaacatt tttatttcta 240

atcaacaagt ttataatgct ttaaatgctt ttggtttgta acaagttaaa aatgaatgtc 300

tatttgtttt ttttaaaatc tgtcttcttt gcagtatgat ttccttggtg ttagaagaaa 360

aaaaactgtg ccttatgaaa atatagatta aaaatgtcta a 401

<210> 54
<211> 401
<212> DNA
<213> homo sapiens

<220>
<221> variation
<222> 176
<223> /replace="c"

<220>
<221> variation
<222> 339
<223> /replace="t"

<400> 54

cagcattctt aatcaaaaaa ttcaaaattg aaaatgcccc agtgaagcat tcctttgagc 60

atcatgtcag tgttcaaaac actttggatt ttaaagcatt ttagatttca gattttcaga 120

ttaggtatac tcaacctgta ttatctttga tgcaatgtta tcattatact ttatgacttt 180

tatcatgctt tctcttagtt ctttgaacat aattataatg gctattttga agtctttttc 240

tcttaaatct gatgttttgt cattctcaca gacaaattta tgttttctgc tttttctggt 300

atatggctca tatttttctg tttcattgca tgcctcatca ctttcttttg gaagttggac 360

aatttagatc atatgtattg cagcaactgt gggtagtagc c 401

<210> 55
<211> 401
<212> DNA
<213> homo sapiens

<220>
<221> variation
<222> 258
<223> /replace="t"

<220>
<221> variation
<222> 297
<223> /replace="t"

<220>
<221> variation
<222> 344
<223> /replace="g"

<400> 55
attgcatcct tgtttttttc ctataataat gtgtctcaag ttcattttgt acaattcctt 60

tccagtcttg cattcatttt ccaaaggaga acttgttcct ttcaatggga aatgatattt 120

aaagatcaca gcttgggtga cattatttct agttcttttc actataaagt aaaatgcttt 180

tttcacttta cattttcacc gctaggaaat gattttttaa aaaagataaa atacatcatg 240

aggtcatact ggaaaacctt attcccacaa acgccaacat aacaacttgt tttatcctac 300

aaaacacaca cagcaccatt gttactacat ataataagat aatagaaaaa cctatataca 360

aatgaaaaag agccttaaac gttgttttac agttcttctt g 401

<210> 56
<211> 401
<212> DNA
<213> homo sapiens

<220>
<221> variation
<222> 23
<223> /replace="g"

<220>
<221> variation
<222> 121
<223> /replace="g"

<400> 56
gccttgaggt ctgtatagag acaaatagcc tgagagagga agaacctatt tgtgtaagca 60

tcaggggtaa tgatgtggag atgtctgtgt gtctgtgttt gtgtgtgggt gtgtgtaaca 120

agatagcgag agagtagaca gagagagaca gagagacaaa gagagactga gagagagagg 180

cagggagaaa cagaatgtgt gtgtgtaaga gagagagtca gagagaggag agagagggag 240

agagacagag gcagagagag acaaagtgtg catgtgtgtg tgtgtgtgag agagagagag 300

agagagaaag agggagagag acagagacaa agaccaagat aaagacagag acagagagag 360

aatggaaaga gagagcgaga gagaagatga tgaactggag g 401

<210> 57
<211> 401
<212> DNA
<213> homo sapiens

<220>
<221> variation
<222> 68
<223> /replace="g"

<220>
<221> variation
<222> 70
<223> /replace="c"

<220>
<221> variation
<222> 112
<223> /replace="t"

<220>
<221> variation
<222> 338
<223> /replace="g"

<400> 57
gtgaactgaa gttgcagaga aatacatgcc accttatgat gaggaataca acaaaatcta 60

acaattgaaa gtatccaagg atggtttagg atgatctgga gagggaagtt ggaggatagg 120

tatgtgtgac caaattatat gaccttaatg tcccatatat cttgagattc tatgatttaa 180

agatgccttt agtatttaat ccatggcttg acccaatcca tagtcaactt tttaatctat 240

aacattttaa aagataagca aataaagcca agttggttag attctcccac ccaaatccaa 300

gcctaaactt atgtaattga cattggattt attagtcaca aaaattatgt aacacatggg 360

aaatatatat ttgtaaaaaa aaatgagagt tttttaaggt c 401

<210> 58
<211> 401
<212> DNA
<213> homo sapiens

<220>
<221> variation
<222> 395
<223> /replace="t"

<400> 58
cattatgaaa tgaaaagaat ccacaatgga atgaaactgg taaagtattc aagctgcgta 60

agtcctggtt ttatcaggtc acaaaaatta attctcatat cattctgttg ttttgcaaac 120

aaactgcaag attgcagagg gtttatttta ttttagacag cctgaaaaca gcaaggtgta 180

caataacagg aataaccagg tcatttgaac aggatcgtga cagctattga tgctacctgt 240

ccagcctgga catgatttgg cctgaccaag cacccaagaa ggctcagtgg gttgtgagca 300

caccctgagc caaaagcgtt atgggctgct ccaaaccaag tgtactttta acgtggatta 360

ataaaaacct gatattcggg ttcagaaata tgacagttgc a 401

<210> 59
<211> 401
<212> DNA
<213> homo sapiens

<220>
<221> variation
<222> 7
<223> /replace="t"

<400> 59
accaggacat ttgaacagga tcgtgacagc tattgatgct acctgtccag cctggacatg 60

atttggcctg accaagcacc caagaaggct cagtgggttg tgagcacacc ctgagccaaa 120

agcgttatgg gctgctccaa accaagtgta cttttaacgt ggattaataa aaacctgata 180

ttcgggttca gaaatatgac tgttgcattg taaggctaca tctagagtag tgcctttctt 240

ctgggctcca catttagaaa ggacagggac aaagtaaaga atttcagatg aagaggaaca 300

aaatgggaaa ggccatgaaa ttgatttgtt atacatattt gttcagggaa agaaaggaca 360

gaggcagaat gataagagga gcaaggaaca caggacagag g 401

<210> 60
<211> 401
<212> DNA
<213> homo sapiens

<400> 60
aagggataaa aaaagatatc ctaggcaaat aaaagaagcc aaaaaacagc aggaatagct 60

atagttatat caggtaaaat aaacttcaag gtaaaaacca tgaaacaaga caaataaggt 120

cactatacaa tgaaaaatgt tcaattcatc aagaggatat aacaattgta aatgggtcca 180

acatcaaagc aactgaatat gtaaagaaaa tagtaataga tatgaaggga gagatagact 240

ataatacaat aatagtaggg gacttcaaaa ctcatcaatg gacagatcat ccagacagaa 300

aaccaataag gagacattga acttaaacta taatttagac caaatggact tgagtgacat 360

atacataaca ttctacctat cagcagcagc agcagcagat t 401

<210> 61
<211> 401
<212> DNA
<213> homo sapiens

<220>
<221> variation
<222> 117

<223>  /replace="t"

<220>
<221>  variation
<222>  275
<223>  /replace="c"

<220>
<221>  variation
<222>  349
<223>  /replace="g"

<400>  61
ctttgcccat gcttccttag taggaagaaa aagagaagga aaataatggc tagtaagtgg      60

ataatagcaa gttagaggat cctttgcaat cctggctggc tctgtcctgt gcgcctcctt     120

tccttatcct taaaggtttg tgaacttctt gtcaccagca gacaattgag attcaaacat     180

atgtcagtgt cattctgaac gaaaaatgga agtgtgatcc ttaagaaaaa tggctctgca     240

tattatcata gcttcttaga tttggaaaat aaccatggtg tctggaaaaa attgagagaa     300

tcttactttc aggttaaatg ccatatccct ttcaactcca aatacattag tatttctaaa     360

gtaagaataa atgattctga gactccgtta gtgaaaacta a                         401

<210>  62
<211>  401
<212>  DNA
<213>  homo sapiens

<220>
<221>  variation
<222>  98
<223>  /replace="t"

<400>  62
ctgccaggaa agaaaacgtg aactgacatg taactagccc tgtctggcac ccaccacttt      60

acttattagg tggcatctgg tccctatacc cacgtttcgg tgtcagtttc ttcatcttta     120

tgaatgatct gaggctggga gagggttggc cccaggtctc actcattgaa gagatttgaa     180

tccaggcgca tctcattcca gcacccatat ttatccagtg cattctctag ggcaagatat     240

ttcccttaca tgttttcctc ttaactgctt aaaaagttct tttggcacag tattcctgta     300

ttctagtaac aaactacaat gttatgtgtc agtattccta ttccataaat aagaacctgg     360

aggatgtgaa ataagtgatg tgtaaatgcg tgaaagatag c                         401

<210>  63
<211>  401
<212>  DNA
<213>  homo sapiens

<400>  63
tcttactccc catccctcct tgaacctggg cttgaccaat agaagacaat gggtttggtg      60

ctttgcaacc tctgctcctg gtcctgagag gtcttgcagc ctccttctgg gtgctggcaa     120

ctctttcttg gaaagttcac cctggtgcaa gccggcaact atgaacaaag gtaaacaatt     180

ctcagaccac cacgctgtga gaagcccaaa tgtagggaag ggtatcagac atgagagtga     240

agaaattacc ctggaagttg gtcctctagc cccagccacc ctggtggatc agaaagaaac 300

cgcccaaccc agaaaaccat aagaaaaata aatggttatc agaagtcact aacttctgag 360

gtggtttgtt acagggcaag aaattactgg gacatatatt c 401

<210> 64
<211> 401
<212> DNA
<213> homo sapiens

<220>
<221> variation
<222> 40
<223> /replace="t"

<220>
<221> variation
<222> 49
<223> /replace="t"

<220>
<221> variation
<222> 394
<223> /replace="t"

<400> 64
ttaaagtaat ccacacatga atatagagaa caaaaagaac gtaaaaatga aaatgtattt 60

tttaatttga gaaacatgtt ttatcaatac atatacacaa agatggcaat taaaaaggaa 120

aacagactta ctgtgaataa aacacaaatt ttgtctacat acactagaaa attttcctca 180

gatccttgga acccgttgat gtatttttat agaatagccg tgatctaatt tttggcaggg 240

gtgtgttcac atgtgtgcgt atccattctt agaaatgtgt aaaatagtat accaaagtta 300

aagtaaatag aatgaaacaa tttttctcta cttagtaaca tttttcagta actctcctga 360

aacaatttcc atctgcaact ttacctttaa aagctttaat t 401

<210> 65
<211> 401
<212> DNA
<213> homo sapiens

<220>
<221> variation
<222> 18
<223> /replace="t"

<220>
<221> variation
<222> 47
<223> /replace="t"

<220>
<221> variation
<222> 175
<223> /replace="g"

<220>
<221> variation
<222> 226
<223> /replace="t"

<220>
<221> variation
<222> 276
<223> /replace="t"

<400> 65
tttactcttg atcaaatcag cattttggca atgaaagtca gaagacgtgt gttttacaaa 60

gtcaaaatat tgttttattt tattataatt tgaaattaca ttttaaccag tctaatggct 120

atttctgagc cttaactgac atgaaactgt ctgtattggt gcaattataa tgcaagatat 180

ttgtagcttc atatgctgct gcagaggcta aacgcagagg atttagcctc ctttgacact 240

caatcattgt acatttatct tggaaaggga aaaatgtaag tggctgataa ctaattttaa 300

ttgacatcta gaaaaaataa ataaaccaag caaaccaaat gtgttttctt aaaactatgt 360

tcttcttagc tattagcctc tctttagcta cagaaagaaa t 401

<210> 66
<211> 401
<212> DNA
<213> homo sapiens

<220>
<221> variation
<222> 5
<223> /replace="g"

<220>
<221> variation
<222> 360
<223> /replace="t"

<400> 66
ataaagagta gagatcttct gctaactgta aatggaaata tggattagag agaaacagac 60

atgcactgtt ttaagccact aaacagtagc cactacccat tggttaatac agataagaaa 120

actctcacaa aataaaagtg agggggaaaa tagggtaaaa aatgtgtcct gaatgtatga 180

aatcaatttt gtgtgtgagg ggaaaataat gtaacaaata tacacacaag gaaaccccct 240

cccccaaacc caccagaata ttgtttttac ctctgggtgt tgagactata gagttttaat 300

ttctttatat attcttctgt ttttctagaa taaatgcata ttattttata attacaaagg 360

ctacttttta actcttcata atcataactt ataatggtaa a 401

<210> 67
<211> 401
<212> DNA
<213> homo sapiens

<220>
<221> variation
<222> 249
<223> /replace="t"

<400> 67
taggcaatga gaagaaaatc gtaattaagg ctgagagaat ggcattgcat cgttgcagag 60

gtaagaaaca gcactgggac tttaggaagt attttcacta ggatcaagta taatttgcac 120

aagggaaggg gaaacaggag tagaaaagta ggcaggagcc agagaataat atgccttgca 180

tgccttacta agaatatgga ctttactcca ttctttgggg agttatggac agataactga 240

tatttccagc cacaatcaat ccagaaacac ccctgttttg acaatacccc agcctcctct 300

agggggagcc acctttgatt ttcatggcag ggtgggatcc agaaagctga aagtctcttt 360

ccaaacttga tttggagaca tgaatttcac acaaatacaa c 401

<210> 68
<211> 401
<212> DNA
<213> homo sapiens

<400> 68
tgctgcaatt gtatgcacat gaccatgagg catgatttcc tgaactgggt gggagatagg 60

ggcacagaat ttttgactca gttccattgc tttaacctgg aaaaaaaatt ccaacatcct 120

tggaactctg attttctgct tgtaatacat ggagtgtaac agctgagcca caggctgttt 180

tgagaacaga acaaaaagct gtaaatatgt cagtcaataa aactctacca agcaaagagc 240

ctttataaat tcaaaacact attgatggtt ttaaattatt ttctctgaaa aaaacataac 300

atcagaggtt atttaacttt tctattattt ataatacagt caccaattta gagataggag 360

acttaatttt ctcttttgtc caaaatcaac tcaaattatg c 401

<210> 69
<211> 401
<212> DNA
<213> homo sapiens

<220>
<221> variation
<222> 152
<223> /replace="t"

<220>
<221> variation
<222> 223
<223> /replace="t"

<400> 69
acatcctctg caacatctgt tgtttcctga ctttttaatg attgccattc taactggtat 60

ggattttggt atctcattgt gcttttgatt tgcatttctc taatgaccag tgatgatgag 120

ctttttttca tatgtttgtt ggctgcataa acgtcttctt ttgaaaagtg tctgttcata 180

tccttcaccc actttttgac gagggtgttt gtttttctct tgaaaatttg tttaagttct 240

ttgtagattc tggatattag ccctttgtca gatgaagaga ttgcaaacat tttctcccat 300

tctgtaggtt gcctgttcac tctgatgatc ttgaaaatgg tcatattgcc caaagtaatt 360

tatagattca gtgctatccc catcaagcta ccattgactt t 401

<210> 70
<211> 401
<212> DNA
<213> homo sapiens

<220>

<221>  variation
<222>  63
<223>  /replace="c"

<400>  70
aacctttcag tggcttcttc cttttggtta ttacgaataa tgctgctatg aacatgggag      60

tgaaaatgtc tgttcaagtt cccgttttta attatttcag gtatatgcca agaagtggag     120

atgctggatc atctggtaat ttaacattta atttttgaag acccactata ctgttttccg     180

cagcgaccgt accatattac gtttcctacc agtaatatga gaaggttcta ctttctccac     240

atcctcacca ctatttgtct ttttatgctt ttgttttaat actagacatc ctaatgggtt     300

tgaggtggta tcttgtgatt ttaatttgca tttccctaat gattagtgac actgagcatc     360

ttttcatgtg cttattggcc atttgtatat ctgctttggg g                         401


<210>  71
<211>  401
<212>  DNA
<213>  homo sapiens

<400>  71
tgaaataatc caactgcagg aaccaactca atagggggag gattgcctaa ttttcaagca      60

tttctgattc attatgtcac cattagcata gtattcccaa aacgcacttc gtgacatgca     120

aagatcccaa gataaaaatt tcacctatgt gtacgtctgc aaaagagaaa aacaattgtc     180

agaatgagca ctaaaccttc gttgatggtt ataggaggga gtggaattat aggacagact     240

taattttatg ctttctcttc tccagtgatt tacttttgta cagaaaataa gccatattta     300

taattagaaa aagaggaaaa cgtaactgat tcaaaactac atgtacaata tgaattttag     360

cttacaaatt tatacataga ggtaaacatc aaaaacggtc a                         401


<210>  72
<211>  401
<212>  DNA
<213>  homo sapiens

<220>
<221>  variation
<222>  83
<223>  /replace="t"

<400>  72
agttggagaa tctactgact aaaagttgaa atggaaaatt ttaagactac aaataaagat      60

cagactaaag gcaaggcaga gccgccctgg gagtacagtg aatggggcat ctgtgtccca     120

ctcgggcgcc gatcgccttg tccagctact gaacagttac tttgagtgca tcaggaggga     180

gaaaattaca ggcacagctg gcatgaatcc actccccatc catggccagt gcaaaggaga     240

caagacactg tctcttcact tttatacact tacttcattt tgtgaaactt ctcttgccct     300

tactatatgt gcatattgtt caagtgaata cctgccagtt ttagaagaga aagggttagt     360

ctgttcttgg ctgctacatc aaaataccat aacctggggg c                         401


<210>  73

<211>  401
<212>  DNA
<213>  homo sapiens

<220>
<221>  variation
<222>  14
<223>  /replace="t"

<400>  73
ataataacac cagcaaatgt ttagctaaaa aatttagcca tacatgccac acattgtgct      60

acacattgag ctctttggtc ctcacatcaa cgctgaatta ggagctatga ttatccccat     120

tctttagatt agtaagctgg gtatcaccaa ggagaagtac ttgccccaaa gtcacatggc     180

tcatatgtgt tggaagcaag gtttgtgaca gatcttccat gttaaaccat tatgttatcc     240

tccttaggag agagtatggt tttatgaata tactgctcca attggctgag atgacaatac     300

tagtttttaa tcggcggctg tgacatattg agggcttata ttaagaatgc gagctgctcc     360

aaaatgtccc tgtcctgcag ttgtacaatc acatattttt a                         401


<210>  74
<211>  401
<212>  DNA
<213>  homo sapiens

<220>
<221>  variation
<222>  24
<223>  /replace="t"

<220>
<221>  variation
<222>  51
<223>  /replace="c"

<220>
<221>  variation
<222>  172
<223>  /replace="t"

<220>
<221>  variation
<222>  330
<223>  /replace="t"

<400>  74
caaaaacttt gaagttagat aagctgattc aaatcctaaa gccagccctt gagtgctgtg      60

tgaccttggg taagttatag aacctttctg ggcctcggtc tctttacctt ttaaatgagg     120

ccaagtgtgt acatgatgct aattaagtga gacagtccat gtgaaggctt agcacagtct     180

ctggcattca ataagaacac ggtgagtgag ttgtttttct tcctgtcatt ttaggggttg     240

tagcaatagt gtttttgatt gcagtgggtt gttgactgcc aagtgaatga ttatacatgg     300

acatggaagt tgagatatga aaaaagccac gtgggtcaga gtctcatggt cagctaccat     360

ggcaatcctg aaaagctcct ctacctgaat ggcctgtcct g                         401


<210>  75
<211>  401

<212>  DNA
<213>  homo sapiens

<220>
<221>  variation
<222>  12
<223>  /replace="g"

<220>
<221>  variation
<222>  237
<223>  /replace="t"

<400>  75
tgaaggggtg gatcaggcag agggtcttag gccactgctg gtaaatgcca aactaactct      60

gaggtcagaa gtccttgatg ctacatcccc aggttcaaaa gcctaagaag ccctcatttg     120

gactgggcag cgtccctcca cctgagagaa attaataaca gcaagcttct gaataattgc     180

tgaaatccac acctgaaccc ggatggacca gagcagccac aaagtcctca aagcacctga     240

ccagccccct cagtgggcta tttttgtaga aaaagaatga gtcctggaaa acaaccaaat     300

acccattgtt tggctgcgtt tattttccat caagagtgat gcagatagct tgcccagtag     360

ggacccactc agcgtctaaa gactaggcaa atggaggtgg g                         401

<210>  76
<211>  401
<212>  DNA
<213>  homo sapiens

<220>
<221>  variation
<222>  57
<223>  /replace="c"

<400>  76
cattcaaata caggaataag aacttcattt tgtaacaaaa acaataatta tataacactt      60

cttaatatta gctatcactt cctaatattt tcactgtcac actgagctaa attttaatat     120

gaattatctc actcaatcct taaaccaatc tgtataaggc aggcaacata acttttccaa     180

agttgtacag ggaacaagaa ccaaaactag gacccaaatc aaggattctc tgatgatctc     240

catgcttgta atgttctctt tctttttccc ccaccaatat gtggaccacg gtcttcttaa     300

caaaatagct gcaatatgtg gtattaatca ttatgtacac acacagacac atacacaaat     360

acaaaggaga tttgctatta acttagattg tacaattaaa a                         401

<210>  77
<211>  401
<212>  DNA
<213>  homo sapiens

<220>
<221>  variation
<222>  240
<223>  /replace="c"

<400>  77
cttgattcaa aacggaatgt catgttggtc agcaatatat gcttccacta aataaccaaa      60

tacacaaatt gaaataaagt gaaattcttt ttccagagtt aatgaaaggt tcctgtttgt 120

gaatttgggg ctttgaagtc caaggcttcc aaacagcacc aggaatttac tggtgattta 180

ttactatttg gacaagtggc gaacagagca ttactctacg gaaagtccac attccctaca 240

tgtcaaggga gccttcctga aatggggaca ccagctcccc ttgctgcaac tcctcttagg 300

gctgtgcctc aggtggggtc cacatagtct ggtgtggtca tctctcctgg gccagcgccc 360

tccccctctc tctgctctag cccctgccct atcctgtgtc g 401

<210> 78
<211> 401
<212> DNA
<213> homo sapiens

<400> 78
atggaaatat aagctgtaaa ctgaaataat atattttcta taataattat gaattcagct 60

ggatggaaca gaaaacttaa atgacagcag ctattctttc ccatacagca agaaggtgag 120

tggtggatgg ctgagggcca gcgcagggct cctgatgtta taaatgtcca ggttgcctcc 180

ctcttcaggc tcggcaccct aagttggaga cttttgctct cagtatcaca ggatgggttt 240

ccaggcgttg agactgcatt ccaggtgggg aaaagaggaa agagcaaagg taaaaattca 300

gctgaatcta cactgcttat ctggagaaaa atacttcccc agaagctcca tttggtacat 360

ttctgcttat gtctaattgg acagaactga gtcacatggc c 401

<210> 79
<211> 401
<212> DNA
<213> homo sapiens

<220>
<221> variation
<222> 274
<223> /replace="t"

<400> 79
ttaatgcttc cttttgtggg aagaacccct gaggtaatca gtggggttca ctgaggtccc 60

ctcctctttt cctctacacc actgttttat gcgtcctgtc tagcctcgtg ttgaaagtgc 120

ctatttctgg gctccaatta ggctcattct ccctcttttc tttgaattat catgaactga 180

attgatctgt cttttaaaaa attatccttg taaattgggt ctatctttta agtattaacc 240

ttatttccct caattcaact tatttccaaa taactattag gcctgcccat taggtgataa 300

gtttttttaa aaataatttg tcactgtaga aatattcaaa catatacaaa aatagagagt 360

atagcctgtg aactcctaca tccctagcac ccagctttgt c 401

<210> 80
<211> 401
<212> DNA
<213> homo sapiens

<220>
<221> variation
<222> 15

<223>  /replace="t"

<400>  80
ttatttccaa ataactatta ggcctgccca ttaggtgata agttttttta aaaataattt      60

gtcactgtag aaatattcaa acatatacaa aaatagagag tatagcctgt gaactcctac     120

atccctagca cccagctttg tcactcctcc atattatgtt gaagtaaatc tcagatatct     180

ttctactata aatatttcac catattcttt taacagctaa ggaaacttaa aaaataacca     240

tcatacaatt tattacttaa atttattaat acatttttaa tatcagatgg taagttacag     300

gatggagctg tgacaccctt gtcatgaaca agcctacagt tgtagactta tgctcacaaa     360

gggtgctcag ctccctatta atgaattgat tgatgaatca a                         401

<210>  81
<211>  401
<212>  DNA
<213>  homo sapiens

<220>
<221>  variation
<222>  117
<223>  /replace="g"

<220>
<221>  variation
<222>  265
<223>  /replace="c"

<400>  81
ctgtccatcc gaatcccaaa agctttcctt gtgcttcctg ctctgaccaa agaggcattt      60

gaggcacatg tgagagaaca gctttccaga aaccacactt gagtccacag cagagcagaa     120

ggggcaggag gcatcaggac catttccaga tttctctctg gccctgattg tctagatttg     180

catccaatca ctgcaagagt ttatacaaaa gttgttagaa agatttgggt ttcacacaga     240

gttatgaaaa caaggtagaa gcctgatgtc ttcatttccc atgaaatctt gtgggtgtaa     300

atgaaacatg ttttagtctc aaatcctgga gctgtggggt tcacaatgga gaaatatcca     360

ggtctcttga acaagactgg acgataacca gggacagttc c                         401

<210>  82
<211>  401
<212>  DNA
<213>  homo sapiens

<400>  82
tctcactcca tgtcaagggc tattcttctt ctctctctct gtctctaaca atattgcaac      60

gatattctga atttagttgt aaatttagtt ggaatttgat ataagctatt tgattttcct     120

tcacaaattt gtttacagca atttcctccc agaacacact gataaagaga agtagatgac     180

agttattgat gaaccctgct tctggcagtt attaatcagc cattgacttg atgaagcagc     240

catggatgcc acacctgatg gtgaggcatg tgacctgggg tccagctgcc atgggtcatc     300

catgtggctt tgggagtgtt gggaagctac tgcttcagca tctccaagga acaagcgtcc     360

tgtgagagtg gaagtgagag gtcagtaaag acagacccgt t                         401

<210>  83
<211>  401
<212>  DNA
<213>  homo sapiens

<220>
<221>  variation
<222>  101
<223>  /replace="g"

<220>
<221>  variation
<222>  153
<223>  /replace="t"

<220>
<221>  variation
<222>  189
<223>  /replace="g"

<220>
<221>  variation
<222>  224
<223>  /replace="g"

<220>
<221>  variation
<222>  308
<223>  /replace="g"

<400>  83
catatgtcaa tgcatagaaa ttatttcaaa taaagtatgt ttctgtgaac ataaaacatt      60

tacttgttat gacactgtaa tcagggcgtc acagattcta agatatgaaa tgctaagcac     120

cccatctcta ccaaaataaa aaaaatttag cccggcgtgg tggaacacac tgtagtctca     180

gcttctcaag aagcaggggt aggaggacaa cttgagcctg ggaagctgca gtgagctgtg     240

gttgtgtcac tgcactccag tctgggtgac agagggagac tatgtctcaa caaacaaaca     300

aacaaacaaa caataaataa atagaaagaa atactaagta gacatttgat tatctcatgc     360

ccaggttgta tctggagata agggtatgct ggaaagaatt c                         401

<210>  84
<211>  401
<212>  DNA
<213>  homo sapiens

<220>
<221>  variation
<222>  110
<223>  /replace="t"

<220>
<221>  variation
<222>  250
<223>  /replace="c"

<220>
<221>  variation
<222>  327
<223>  /replace="t"

<400> 84
agaatcttag tggtgactgt gcaaagccat ttggtctaat gattttacca agagcatctc 60

agaacactag tattggtagt atgtagcctt gttaataaat atatacaagc acatgttgaa 120

atgacttgtt attatatggc tagagcaaac ccacgtcatc ctcatgatgt cttgctgtca 180

caatctcatt ctctcttggg ttctgacctg actcctcaag caagagaaca agggttggca 240

gagctctcca ggaatgcaca gccccaggtg cccagcagag ggcatcaagt atgacagata 300

ttggaatgac atctgttgtg tgtgcagagc agactgactg agtggaacca tgaccaaaga 360

gaactcaggt agactcaagt gcttctggga aatttgagaa a 401

<210> 85
<211> 401
<212> DNA
<213> homo sapiens

<220>
<221> variation
<222> 75
<223> /replace="t"

<220>
<221> variation
<222> 124
<223> /replace="c"

<220>
<221> variation
<222> 376
<223> /replace="t"

<400> 85
tgttattata tggctagagc aaacccacgt catcctcatg atgtcttgct gtcacaatct 60

cattctctct tggggtctga cctgactcct caagcaagag aacaagggtt ggcagagctc 120

tccaggaatg cacagcccca ggtgcccagc agagggcatc aagtatgaca gatattggaa 180

tgacatctgt tgtgtgtgca tagcagactg actgagtgga accatgacca aagagaactc 240

aggtagactc aagtgcttct gggaaatttg agaaacttcc ctgacagaga atttacttca 300

agtcagatac ctccccaata attgaaaatc ctgccaataa tgaggaaaga gaagtggacc 360

caaattccta tttccctgaa tctgctcttc agtttgttca t 401

<210> 86
<211> 401
<212> DNA
<213> homo sapiens

<220>
<221> variation
<222> 91
<223> /replace="g"

<220>
<221> variation
<222> 284
<223> /replace="g"

<220>

<221>  variation
<222>  360
<223>  /replace="g"

<400>  86
agggtagggg agtgtagagg ttcaggagtg gtgttttagt ttggtgttgt gcccagggca      60

gaccctcaga taaaatgcct ttaagacaag atcctgtgga atggagggag gacctaagga     120

ggcaatgcag atgtctggta acggagggtc ccaattagaa aacacaaaag atcagaaaac     180

ctaaggcctg agtcagggtc agtacctgaa gtggggctga gcagggtgtt ggggacgagt     240

tgaggacaga gagggcctga aggtgagatc agttatcaat cagattaggc cttgtgaaga     300

ctttggctct tactctggat gggaagtaga ggtctggcat tctctgcctt tcatttggaa     360

gggaaccctc aggagtggaa ggggagcaaa gctgggaaaa g                         401

<210>  87
<211>  401
<212>  DNA
<213>  homo sapiens

<220>
<221>  variation
<222>  42
<223>  /replace="g"

<220>
<221>  variation
<222>  125
<223>  /replace="g"

<220>
<221>  variation
<222>  272
<223>  /replace="g"

<400>  87
aaacacaaaa gatcagaaaa cctaaggcct gagtcagggt cagtacctga agtggggctg      60

agcagggtgt tggggacgag ttgaggacag agagggcctg aaggtgagat cagttatcaa     120

tcagattagg ccttgtgaag actttggctc ttactctgga tgggaagtag aggtctggca     180

ttctctgcct ttcatttgga agggaaccct caggagtgga aggggagcaa agctgggaaa     240

agtagcaata aagtacctgc aaatgtttta tatagatttt gttcacatct ccaaaaaaag     300

tgcaaaaact cccctagctc tgcattttcc agccactgtg gactgaattg tgcccctccc     360

caccaagtta atatgcagaa gcccgaactc ccaatgtgac a                         401

<210>  88
<211>  401
<212>  DNA
<213>  homo sapiens

<220>
<221>  variation
<222>  78
<223>  /replace="c"

<400>  88
aaacataact tatatgcaaa tggttaagaa taccagaaac attgcaagaa tgctcttttt      60

aaatcctgaa gatattggtt tattttaaag catcaaggat gatttagatt tttggaaaaa 120

cagaggtggc atgaagattt ctgaactcgc tcacacacaa acccgtcaca gccgctgtga 180

tctataagcc aaatttgtgt tagaactttt cttttttgaa taacaacaaa acaacaaaaa 240

cgctcctaac tgaaactggg aaaggactag aaactttgtt ttcttactaa tgtgaggttg 300

gaaacatatt ggctttcaag tgaattggcc gtgaccacag cagcacttgg atctgaggga 360

gccgagattg caggcagatg tgagtttggc acttgcatgc c 401

<210> 89
<211> 401
<212> DNA
<213> homo sapiens

<220>
<221> variation
<222> 352
<223> /replace="t"

<400> 89
taacatagta acagcttaca tttttatcct ttggttttat tgaaggatcc tgtaaatgaa 60

taaaaccata agaaaacata tggaagaaca gtcaaatagg ttataaatat aatcaacact 120

gttttaacag catcacagag caagcatgga gggaatgata ttttattgat tatttagagc 180

ctaagccagg ccttgtgcaa cgcatttctc atgtataatc ttatctgacc tcatgaagga 240

ggattttcct taaactgggg agtctgaggc ttagcaaatg aatcgcacaa ccaggatttg 300

aactaaagtt taccttcatc taaatacgcg tttttctttc tctctctctc tctttttttt 360

tttaccccta tcgcatcata gccacatctg tggccacaca a 401

<210> 90
<211> 401
<212> DNA
<213> homo sapiens

<400> 90
tgcaaccaag cgactaaggg ggcaggaaag gggcttccgg taagagtcga agtgtgaatt 60

ttaatttcgg tggttctagg gatggcaaga tccaaggtgt agccaagtga aagagttgcc 120

aaggtgggat ggagagggag gtcgctggag gtgagcagcc caggacctgc agatgagaag 180

tgggattagt ggtccccaga aatgccgtgt tccaagggga agccggcggt gggcaaagga 240

ctagcagagg gttcagtccg aagccgccac cagggggcgc gagagagcag caagccgtaa 300

cagctcgcgt aaaaggcggg agctggggct tcctgggggt tccctagaag cggacctcga 360

gacaaggtcc tgggtgcaag cggtttattt gggggatggc t 401

<210> 91
<211> 401
<212> DNA
<213> homo sapiens

<220>
<221> variation

<222>   202
<223>   /replace="t"

<220>
<221>   variation
<222>   322
<223>   /replace="t"

<220>
<221>   variation
<222>   348
<223>   /replace="t"

<400>   91
cgagcgggat gctgtggatg acaagggagc tgctctcacc tccccgcccc gagacagatg      60

aggcactcga ggacctgcag caggcctcag agagggctag gaccagggag gggctgcgga     120

tggagagcag agtgacggcc tagaaaggag gttcccgagg gctctgggga ggcggaagag     180

gggacgcggt gtgttgagat acggggagaa gttcagagca gcatgggcat gcaggtgcct     240

gcagaggggc ccatctgggc tgcaccccag caggacaggg ccatggcttg gtgtgggttc     300

ggctggctgc acagcttctt gcaaagtttg tccccagggt ccttgggagt agaggtcttt     360

cggaaggcac catctccaga gcgtgtttct gagtaactct g                         401


<210>   92
<211>   401
<212>   DNA
<213>   homo sapiens

<220>
<221>   variation
<222>   7
<223>   /replace="g"

<400>   92
tgctgaagac tgcccatggg tagcatcatg tggagtgtgc gttgtgttca ttgggcctgt      60

gtgtcatgaa gacactgtgt actgttcata cagagaccct ttgatgagac accacaagag     120

aaactccttg taggagcctc tggcagaaga aaaatggttt tcttttgagc taccagatta     180

aaaaaaaatt gtggttcccc tgaatcctat aacagcattt gtctgcctgc aaagctcagc     240

tccaggtttg taggccaccc aacttaagcc tttgtgaaca catttttggg aactgatgct     300

accctggact tcactgaact atcttacttc atttttcctt tactcgtctt tatttaatag     360

ttttattgaa ttatttatgt aaatctcacc tcatcatgct t                         401


<210>   93
<211>   401
<212>   DNA
<213>   homo sapiens

<220>
<221>   variation
<222>   30
<223>   /replace="c"

<400>   93
caaggaatgc cctagaacaa acgcctggca catagtaagt tctacccaag tatttattat      60

tataataatt gtataaacat ttaacgactt ccttaaattg tttcctatta cttgctattt 120

tgaatagtgt ttcaattaat atccttgtga tatggttttg ctgtgtcccc aaccaaatct 180

tatcttgagc tgtaattccc acagttccca tgtatcgtgg gagaaaccta gtgggaggtg 240

atagaattat gggggcgggt ctttcctgtg ctgttcttgt gatagtgaat gagtctcatg 300

agatctgatg gctttaaaaa tggaagtttc tccacacaag ctgtctcttt gcctgctgcc 360

atccatgtaa gctgtgactt gttcctcctg gccttctgcc a 401

<210> 94
<211> 401
<212> DNA
<213> homo sapiens

<220>
<221> variation
<222> 54
<223> /replace="g"

<220>
<221> variation
<222> 356
<223> /replace="t"

<400> 94
cccccagctc ttatcacaga cactatacaa acaggaatac tttgtgttac cctatgttaa 60

tggttctctt attttgctgt gcatcagaat accctggaag gatgactaac atacagattg 120

ccaggctcag cacctgctgt gtctaagcca ggatatctag gggcatttct agtaagttcc 180

aggtaatgct gatgctgctg atttagggac aacactttga gaaatatggc tgtgtattat 240

gggtctattt gaacattcga ggaaaataag taggataaaa gtgatttggg acaacagtct 300

cattcttttg atatgattaa tacatatgac acaaatatct tctttggtaa tttttaaaaa 360

aattagatat aatagaaatt tatttttctc ttacacaatg g 401

<210> 95
<211> 401
<212> DNA
<213> homo sapiens

<400> 95
ctgtcactgt gattttcctt gcattgtcaa ttggctacag tattcttcat gatttctctt 60

gactaacatg ccttctgaca atgttgtctg aagctgtggc ttcatttggg aaatttctgt 120

gtgttttaga aaatgtaatg ctgtcatcac aatgggccat agacatttca gtctttctag 180

gtgttctgaa ggggttggct caaaaataca gggcaggctt tcacgttggt ctacttttag 240

tagtgacatc tttcagattt acctaaaata atggctaata tttgtctaaa aactttctag 300

tggagaactg tactcatact aatggctagt aaaattaatg gcttactgtg tgtattgcat 360

gctcgctgaa gtgttttctg tgtgtccact cagttactat g 401

<210> 96
<211> 401
<212> DNA

<213>  homo sapiens

<400>  96
aagaagccac accttccatt tctcactgca aagagctgtg gtgacagaca tgtaagaatt      60

ttattttctt tttaaagtaa cttagattac agtagggctg ccatgagaaa ggtgacacag     120

aagagcagac gagagggagc agaaaggtgt atgcagattc tcttgtgtcc atcagcctta     180

agaacagggg ctgctgagcc aagtcagagg ctgaagccac tcctagggat aagatccccc     240

catgtctcta ctctggggac attagctcca ctgtttagac tagcttctca ctttcaccca     300

catttttagg ccagtggaaa cttccataaa tccaggtcat acattcacat tcaaccgatg     360

tgtgtttacc cactacttcc gatctgaaat tttgggaagc t                         401


<210>  97
<211>  401
<212>  DNA
<213>  homo sapiens

<220>
<221>  variation
<222>  48
<223>  /replace="g"

<220>
<221>  variation
<222>  61
<223>  /replace="c"

<220>
<221>  variation
<222>  124
<223>  /replace="c"

<220>
<221>  variation
<222>  145
<223>  /replace="t"

<220>
<221>  variation
<222>  269
<223>  /replace="t"

<220>
<221>  variation
<222>  314
<223>  /replace="g"

<400>  97
ttccccagtg ggacgactct gcggcatgtc tgcgctggct cccagggaac agcaccccag      60

atgcccacac taataacctg gtctctcact catcctgact gggctgcctt cccttccctg     120

tctgtcttcc cctttcttct ccctctcctc ctgggatcac ctcccataca aactcctcac     180

cctcatttcc ttgtcccaag ttctactttt cagggaaccc aggatacttt cctcctggtc     240

ctaagaaaac ccaggcaaac caagtttcct gctctcatac aatttacagc atgttgggtt     300

gaacaggcaa cttaacaaac attaaaaacg aagtgtaacg agtgctgagg tcagggagca     360

tagactggcg gaaggcacag agccacaggg gaccctaagc t                         401

<210> 98
<211> 401
<212> DNA
<213> homo sapiens

<220>
<221> variation
<222> 11
<223> /replace="c"

<220>
<221> variation
<222> 32
<223> /replace="t"

<220>
<221> variation
<222> 88
<223> /replace="t"

<220>
<221> variation
<222> 156
<223> /replace="t"

<220>
<221> variation
<222> 305
<223> /replace="t"

<400> 98
ttccctgtct gtcttcccct ttcttctccc tctcctcctg ggatcacctc ccatacaaac 60

tcctcaccct catttccttg tcccaaggtc tacttttcag ggaacccagg atactttcct 120

cctggtccta agaaaaccca ggcaaaccaa gtttcctgct ctcatacaat ttacagcatg 180

ttgggttgaa caggcaactt aacaaacatt aaaaacgaag tgtaacgagt gctgaggtca 240

gggagcatag actggcggaa ggcacagagc cacaggggac cctaagctag cccaggagag 300

gtcccagaag ctgagctggg agtgactggg gctccctgcc ttttcttccc cttcttgaac 360

ttccactcct ctctgtttca tttcttctct tcctttcaga a 401

<210> 99
<211> 401
<212> DNA
<213> homo sapiens

<220>
<221> variation
<222> 83
<223> /replace="t"

<220>
<221> variation
<222> 345
<223> /replace="g"

<400> 99
tttgggattc tggagtctat tgaatatttg caacttctag gggaaggctt ggatggtaaa 60

ttgtttaatt ttgatcattt tcagctccta gcacagtagc tgctacctat ctttcacctc 120

tggaccctcc tctcttgagc aggcagcatg catgtgttcc tggagaagtt tgcacacagc 180

ttgttggagg cagggtgggc aaaaggatcc tgccctccaa atatcaggga tttgtactct 240

gactgctgat ggctgcttct gatcacaggg gtgctggcaa tgaaaaagcc attattgttg 300

ttgcatcttc ccacattatt gcaagcccgt ctacctctgg atgaactgag ttccacggta 360

tttaaaaagc tggcaccttt gttacccact tcagttttct a 401

<210> 100
<211> 401
<212> DNA
<213> homo sapiens

<220>
<221> variation
<222> 26
<223> /replace="g"

<220>
<221> variation
<222> 158
<223> /replace="g"

<400> 100
agggtaaaaa caaggacatt tcttaagaat ctatagcagt aatccaggaa aaaaataatg 60

tggtcttaaa ttggaaagtg gtgggggtaa gatatcagat tcatgtgatc atgactaagc 120

aactccagga ttttctgaca gaaagcatgt ggcatgaagg aaagagcaga gtcaaggagg 180

gctctggaac ttgggccaga aaaactagaa ggataaataa ataataaaaa gtttagcagt 240

caaattcata acagtaaata aataaattaa aaataataaa aattaatgga taaaaagttg 300

ttatgtaaat tcagtgccct attccatctc ctaggttatt ttcactagaa gttaattaga 360

actctccagt tgtcccagcc tagacagcct gctagcccct t 401

<210> 101
<211> 401
<212> DNA
<213> homo sapiens

<220>
<221> variation
<222> 290
<223> /replace="t"

<400> 101
aatgttatga atatatttat aacttttata gcaaataaaa taataatgca ttcagatttt 60

gacatggatg ctaaacaaac aatgaaaggt ttgtgtctga gagaatcagt attaataatt 120

atttagtatt aagtaagcat tgttaagtaa ttaagtacta agaattgagt acaatatttt 180

aaaatgtgac tttacagaga aagagagtac aaattgtata aaattcaaga acacataaaa 240

ccaatctata ctgatgggca tcagaatagt ggctatcttt tgggaggtgc tgactaggca 300

cacaagatgc cttcttgggg gttgtacatg gcctgcatct tgacttggtg gtgattgatt 360

acagggttgt gaacatatgt aaaaacttca tcaaggtgta c 401

<210> 102

<211>  401
<212>  DNA
<213>  homo sapiens

<220>
<221>  variation
<222>  265
<223>  /replace="t"

<220>
<221>  variation
<222>  398
<223>  /replace="t"

<400>  102
aatcaagtaa aagaatggtt tgaataataa catattgata acagataata caaacttttt      60

tcattaagct tcagaagaat cttagtagaa atgcattgtt tggaatgcat atgctttggt     120

ctcaataaaa atatccggtt gcagagatat ttcatgtggt ttgaactaac aggcaaaaaa     180

ttaaaaattc attggtatac actaattcat gcaattgtat attttggagg taagtgtaat     240

ggcataggtt tgctgtaatg gaaccgattg gaaaatgatc tcttggagaa aatttattgc     300

tggtcataag atttctttgg tttagaaaaa tgctgggttt agtaaaatct gaggcactaa     360

atgaaagtct gctttaataa aaatgtggat ctgagaactg t                         401


<210>  103
<211>  401
<212>  DNA
<213>  homo sapiens

<220>
<221>  variation
<222>  26
<223>  /replace="g"

<400>  103
gctctaactc tggggacttg tattaagccc tgactttgtt ctctgtctcc ttgaagttag      60

gaatccactg cactgagggt gccaaagtgc tcctcaatca ctggttccta cagtccaatg     120

ggcaatatca gcaaaagtgt tttgtagtgt agtgacagtg ggattcattc tctttcacat     180

atgtccacag cagtggtggc agcagctgca gcaaagtgct agtgggtgcc agggtgcttg     240

cccctctgca ggtgttcagc accgtggtgg aggcaacaca gctgtggggg gcaggggccc     300

ttgctgatga ctgtgtgcat ggtcatactg gaggtggtgt tggctcaggg gtggggcact     360

ggcagttgta gttctgggtg ccttctctgt gccccacaag c                         401


<210>  104
<211>  401
<212>  DNA
<213>  homo sapiens

<220>
<221>  variation
<222>  249
<223>  /replace="t"

<220>
<221>  variation

<222>  253
<223>  /replace="g"

<400>  104
aattttgttt cctggaggca ctctgatacc tattactgta tcaatcagga tccaggcaga      60

aagtagacat catgcttaaa ctgggtaact tgaagataat ataggaaaat agcaagcaat     120

agtctagaat cctaaaagtc tgaatctatt atcaacctta ggtgtgaatg ggcaagggga     180

gggagtggat tctgtcatac aacacatgag tgcaaaagag ctgtgtagtg ctgcttgatg     240

cctattacct ttatgtaaag acacagccag ctcacgtgag ctcttaggaa gggggataga     300

gaaataaata ccctgacctc atcttcctgc cctctgggct gctggcctcc aaaccagaag     360

cagggcactg gagcttacca acatggtaca caaaggctgg c                         401


<210>  105
<211>  401
<212>  DNA
<213>  homo sapiens

<220>
<221>  variation
<222>  85
<223>  /replace="g"

<220>
<221>  variation
<222>  145
<223>  /replace="g"

<400>  105
gtgacacatt gaattttatt actgactctg tgtttcaaaa cattttgggg actgaaaaac      60

ccaccaggca aatctcaggc cttaaaaagc agttaaactg aattgcaaat aaaaggctta     120

gaaagtcctt aaaatcataa acttagcatc tccaatagga caaatgaact ttgtcttctc     180

atctgagtta acagatttgg aagaatcaaa atcttttctg ttgtatagac tacatgctac     240

catttccaca ggcaagctgc ccctaaattt ggaaaacttt tccttctgaa accacccaat     300

ctctttagct tgatttgggc aaccgaatct cttaggcttt tgcctctttg tgtccaaaac     360

tagcactgtt cctgagagca ggctatcaat catttacata t                         401


<210>  106
<211>  401
<212>  DNA
<213>  homo sapiens

<220>
<221>  variation
<222>  38
<223>  /replace="c"

<400>  106
gcgtcgctca cgctgggagc tgtagactgg agctgttgct attcggccat cttggctcct      60

ccccaagggg aagcttttta agctttttat tctaaggact attgaggtca tttcagtgat     120

caaagtgaga tttattgttc actaatcggg ctcctatgtg atcttatatt gcatattagt     180

gcagatttta tataatttct attcacaaca gctattgatg aaacaatgtt tcccacattt     240

ttgtcctttt agtgctaaaa attaaaacca aagttagggg tatagaattt aggattatag 300

aactagggat ttgttggtcc ttaaatagct gggtgctgtt tgttgaaaga gtgattctac 360

tatgaaattg catagcatgt tgcaagttcc atcataatat a 401

<210> 107
<211> 401
<212> DNA
<213> homo sapiens

<220>
<221> variation
<222> 198
<223> /replace="g"

<220>
<221> variation
<222> 357
<223> /replace="g"

<400> 107
aactaacctc attctatgcc cacttaacct ttctaatagc tttagcactg aatgactcat 60

atgaacccat tttacaggtg agaaaattga ggcctagaga agttttgagg cacatctgct 120

tatagctagt gaaaagtgaa ttaaaactct gaactagtct caccttatag ccagagctat 180

ccatcatggt agcacgcagc atctaggtga gtttgtgctc tggagagttc atgtttttca 240

ctgctcacag ctcctgtgtt ctaggtggta cgtaatggtg aacagactgg gcccgagatt 300

aagattacat gattggaagt gcaagttcat cacttttgga gcaccttggg caaatcactt 360

gtccttcata gtcatttgtt tctgcataag taaaattggg a 401

<210> 108
<211> 401
<212> DNA
<213> homo sapiens

<220>
<221> variation
<222> 42
<223> /replace="g"

<220>
<221> variation
<222> 45
<223> /replace="g"

<220>
<221> variation
<222> 392
<223> /replace="g"

<400> 108
gtctcacctt atagccagag ctatccatca tggtagcacg cagcatctag gtgagtttgt 60

gctctggaga gttcatgttt ttcactgctc acagctcctg tgttctaggt ggtacgtaat 120

ggtgaacaga ctgggcccga gattaagatt acatgattgg aagtgcaagt tcatcacttt 180

tggagcacct tgggcaaatc acttgtcctt catagtcatt tgtttctgca taagtaaaat 240

tgggataata ataatagtgg ataatagtgc tggctcatag agttattggg agttaattac 300

atgagttaaa tcatgtaaag tgttgagggc agtgcctagg cacgtagtaa gaaatgtgag 360

tgattactgt ttttataacc ttattgttaa tactaatttg c 401

<210> 109
<211> 401
<212> DNA
<213> homo sapiens

<220>
<221> variation
<222> 139
<223> /replace="g"

<400> 109
atagtggata atagtgctgg ctcatagagt tattgggagt taattacatg agttaaatca 60

tgtaaagtgt tgagggcagt gcctaggcac gtagtaagaa atgtgagtga ttactgtttt 120

tataacctta ttgttaatac taatttgctc cagatggcat ggtatacagt ggtaacagct 180

gatgacttct ctccatttga ataaactgtg ctaacttaat acagtggctc tctggagcac 240

tacaggtcta gccaaccctg cagcacattt aaggtctcag tttacatcac ttcctccagt 300

atctcattgg ccaaaccaag tcatgtggcc aatctcatca tccatggaag ggggatcata 360

tttgtgccat taagtttcat gatagagaga aagagggatt g 401

<210> 110
<211> 401
<212> DNA
<213> homo sapiens

<400> 110
gccttggtct cccaaagtgc tgagactaca ggtgtgagcc actgtgcccg gccagttttt 60

cttttattct ctgtctgctt ccttcatttc tcaaaggcat ctcaaactca gtgggcccaa 120

aaccaaagtc aagctccatc agaacatcct gctacttcca actttaaaat atatcttaaa 180

tcacaatttc ttgctacatg caaccctttg gttcaaacta ccatcatccc tcgcttgaac 240

ttagaattga ttttcctgct tcagtatttt tttaaatgat gaaatatttc aaataaacca 300

tgtgtggctt tatacttttt attacataaa aaggatgaaa atctgccggg tgcactggct 360

cgcacttgta atcccagcac ccatcccagg ccgaggtgga t 401

<210> 111
<211> 401
<212> DNA
<213> homo sapiens

<220>
<221> variation
<222> 183
<223> /replace="g"

<220>
<221> variation
<222> 200
<223> /replace="t"

<220>
<221> variation
<222> 313
<223> /replace="g"

<220>
<221> variation
<222> 332
<223> /replace="t"

<220>
<221> variation
<222> 343
<223> /replace="g"

<220>
<221> variation
<222> 390
<223> /replace="t"

<400> 111
tttattttct tcaaaacatt aaagggaaga actaaaaaca aaaaccagca aacaaaataa 60

tatttgtgaa aagtgatttt caaaaatttc caaactgctg tggggtgatg agtgacacac 120

atttccataa agtttctcat tctatctgtg tctttcagtg tgaacaaaaa atttctttct 180

aaaatgtgaa gattttactc acttcactac tacctccttt agtggttgga agtcatttga 240

atttttcttt acttttctag caagaattct ttgttttgtg ctatgactca cgtgaataat 300

agctcctggc ttaatctgaa gctctgtgtt ggctctgcca gcatgagaca ggaagacctt 360

ctccactttt gcccagtaat tttcattctc cccccacagg g 401

<210> 112
<211> 401
<212> DNA
<213> homo sapiens

<220>
<221> variation
<222> 18
<223> /replace="g"

<220>
<221> variation
<222> 40
<223> /replace="c"

<220>
<221> variation
<222> 157
<223> /replace="g"

<220>
<221> variation
<222> 177
<223> /replace="t"

<220>
<221> variation
<222> 372
<223> /replace="t"

<400> 112

ggggccacca tctgtgtata tttgcacaac ctccccataa ctgcatgggt tttctctgag 60

tactctagtt tgctctctca ttccaaagtc atgcacatta ggttaattgg catgtctaca 120

tggactcagt gtgagtgaat gtgagcatgg gtgtgaatgt gctctgccat gagagactgt 180

cctgttcagg gctggttccc acctcactcc gtgagctgct gggatgggct ctggccaccc 240

atgaccatga actggaaata agcaggtaaa gaattatctt acttggtttt attaatcttt 300

cttaaatgta tgtatagttc acatttattt caatgtttaa tattagaaat gttttgcctt 360

tatttagaag tgtggtattt tgtgatcaga aatctgccat a 401

<210> 113
<211> 401
<212> DNA
<213> homo sapiens

<220>
<221> variation
<222> 6
<223> /replace="t"

<220>
<221> variation
<222> 30
<223> /replace="g"

<400> 113
agagactgtc ctgttcaggg ctggttccca cctcactccg tgagctgctg ggatgggctc 60

tggccaccca tgaccatgaa ctggaaataa gcaggtaaag aattatctta cttggtttta 120

ttaatctttc ttaaatgtat gtatagttca catttatttc aatgtttaat attagaaatg 180

ttttgccttt atttagaagt ttggtatttt gtgatcagaa atctgccata ggaacttcat 240

tatagtttat atcaattagc ctatggaaaa attgtttttg ttatatgtca ttttgcttaa 300

agctgcagtt tccaggactc tatcaacaac attaagtgag gactttgtgt gatttgcggg 360

gccaagtgca aaatgaaatt gtagggtacc ttgttcaaaa t 401

<210> 114
<211> 401
<212> DNA
<213> homo sapiens

<400> 114
cttaaagata acaaacatat atgtttaaca ttttgagttt ttaatctttt tttgtttaga 60

acctctttat ggaaacatgt ctagcttact aaggtaaaat tgactcactc attgactcat 120

ttagcaaatt atttattgat ctcctattta tcagacaggg tggaatatag aaaaggtctc 180

tgcccttctg gagtttctaa cccagtgtgg aaaactgata atacataact aaataactca 240

tataccagtg taattacagt ggataagtgc taggaagaaa atgattgtga tagtaggtaa 300

ccaggagggg tgtacttgga taactatcac ctcttggatc ttgactcctg gcttgatctc 360

cctcttagag ataggcaatg caagtggttt tttgtattag c 401

<210> 115

<211>  401
<212>  DNA
<213>  homo sapiens

<220>
<221>  variation
<222>  128
<223>  /replace="c"

<400>  115
tctttcttag atgatctatt caaagtgtgg ttatctactc actatttttg tttttctctg     60

tgcaggaggt accagatgcc tctagtcagc cgtcttatag cccctcctat atcgttttaa    120

tttttgtatt ggtcaaactt gcaaaaggtt gtctactttg ttgatgttaa aattttttcg    180

ttttatcgaa ttttgatttt ctaattcaca cttcaggctt ttatatttat ccattctgtc    240

cttgtagctt agttggattt attttgttgt tacttcctgt gcttctttgt ttgacgtatt    300

agtttactta tttacatccc caccagcaat gcacaaaagt tctaattccc ccacatccta    360

ccaacacttg ttactttctg ttttgttttt tttaattata a                        401

<210>  116
<211>  401
<212>  DNA
<213>  homo sapiens

<400>  116
aagggtgtat cacaacactg ggaagcatat tccttaacat taggagttta tgtgatttag     60

ctgaagagag aacacctgta ttagttaaaa gggaatattt agcagcattt aaacaaaagc    120

tatttcacat gtcactaagt atactataag aatttccaga aagaagtatc atcctttgtc    180

taatagttca gaggatttct attagtaaat gaaaaatgac agcctcattt tatcctccaa    240

atatgtcaaa attatcttga gttgttgggt accagaactg ataggtgtca tgtatttaag    300

cccatttgaa aaatacattt caataatgga gtgagactct gtctcaaaaa aatttcaaat    360

gtcttttctc aattgaaaaa acctattatt tctagataga t                        401

<210>  117
<211>  401
<212>  DNA
<213>  homo sapiens

<220>
<221>  variation
<222>  39
<223>  /replace="t"

<400>  117
cagtgccttg atcttggact gctcaacctc taaaactggg agaaataaat ttcattattt     60

ataaattacc cagtctgggt atgtttttaa tagcagcatg gatggacaaa gacagtgtgt    120

aacactcagt atcaacaaac tccaaggaaa tgggcaccct gatactctat tgtgtagtgt    180

aaattcacac aaattttgga aagcaatttg gcaatatata tcaaaatctt aaaaaagttc    240

taggcacaag aaccctgttt tagaaattaa gtctattgga gtagtctgca ggaagttgtg    300

tctgtaaatt tgtttatcac agttctgttt atactggaag ataaccctca agaaatgtta    360

tatatctaat aatagaggat tacaattaca tgatgaaatt t 401

<210> 118
<211> 401
<212> DNA
<213> homo sapiens

<400> 118
tttatttagt tctgctctga tctttgttat ttcttttctt ctgctgggtt tgggtttggt 60

ttgttcttgt ttctctggtt ccttttcagt agttaggggt taaattaaac tcagaaatca 120

gtctatgcat accctgacct cagagatgat gctgttccac cacacctttg ctgggcttgc 180

attctacttt ggaattccac atcttgagaa ttatctcttt aatggcaaaa gtgccaataa 240

gcctttccca attttctcca ttgtgaaagt aacatctctt atatcataat gtatataata 300

tccatgtcaa tgtattttgg gtgtaaaggg tgaaagttag gaagtatcca gttggaattc 360

tgtgtaaagt gcactgaatc tggggaggca gagaatgctc t 401

<210> 119
<211> 401
<212> DNA
<213> homo sapiens

<220>
<221> variation
<222> 1
<223> /replace="g"

<220>
<221> variation
<222> 76
<223> /replace="t"

<220>
<221> variation
<222> 157
<223> /replace="t"

<400> 119
agtttattta ccttttctag gattttggga aaagagaagg gccttgcaga gcccctctta 60

ttaataaccc ctgcccaacc cccctacttt tccagatcct ttccctttct aaggagaggt 120

cctgcaggac gcctaccaga gccatgcatc agacagcggc ttctgcccag tcacggctgg 180

gtgggtgtgg ccagcttatg agaaagttca cgtaagaggt tgatttaatc tctccagttt 240

taaaatgtgg aatggtatgg tctgaatggc tttgaattag aataaggtct aaactgattt 300

gagagcaagg cctggatttg aaagggagag tgatgaaatc aaagaatgtc agagccaagt 360

aatgttagag atttaacaag aactgcatgt attccctgtt a 401

<210> 120
<211> 401
<212> DNA
<213> homo sapiens

<400> 120
atctggcaaa agaaagtcat tcagattaaa aaagaataaa ttaaattatc cctctttgat 60

gatgacataa tcttacagct agaaaaacat aaagactcca ccaaaaaact cttggctccg 120

ataaataaat tcagtatagt tgcaggatac aacatacaaa aatccataca tttctatata 180

ccaataataa actagctaag aaaggaatca agaaggcaac ttcatttaca gtagcaacaa 240

agaaaacaaa atacctagga ataaattaac caaggtggtg aaaatctcta aaagtaaaac 300

tataacacat ggataaaagt aaccaaaaag cacatcaaca aatcaaaagt caccgcatgc 360

tcatggatca gaagtattca tatcattaat gtttctatac t 401

<210> 121
<211> 401
<212> DNA
<213> homo sapiens

<220>
<221> variation
<222> 328
<223> /replace="c"

<400> 121
ccaattgttt tctagcaatg aaaggaatca tgtaaaacca cactagttac cttaatccca 60

gttagaaaac actagaggaa ttcgttatga aacccaaaga catgatgacc ttcacaattc 120

tccaggcaag ttcttcatgt accatgacaa gtgggactga gagttttggg aaatgcagaa 180

gaaatgtgtc attttgaaga attctatgtg gaagattggg attctagtgg cttaaaatga 240

tgtggagtga aattgttttc aatgaaaata tttatcaaag ttatatcatg agtatatcta 300

accctatgtt tactgtattt gtgctatgat atgaatatat tttgatttag tatttgatca 360

gtctttactg attgttgaga atctactaac tcagtgcttc t 401

<210> 122
<211> 401
<212> DNA
<213> homo sapiens

<220>
<221> variation
<222> 16
<223> /replace="g"

<400> 122
gaaaagtaaa gtggaagtag gcataaagat gacactgcag gtctcaattg gagattcaaa 60

gtcagtcatt tccccagtta ggctctaaca caatgtaaca tgatgagggg agggatttgc 120

taatcacaac cagagtcacc actgagtgaa ggcatgttct atgccaggct cccaaattaa 180

ctaccctcat taatttccca atcttgcttt tggggagcca caccccacct cccttgatca 240

cactagactg tagcaaagac agatgataac aataatggtt aatatttact gagagcacac 300

caatgccagg cactgctcta agccttttgt gtgttttaac tcattttatt ccctacagtg 360

accttctgag gaagtaccta ttatgatctt tatttttcag a 401

<210> 123
<211> 401

<212>  DNA
<213>  homo sapiens

<400>  123
acctcaccca gctcccatgc aaccaaaaag gccagtctca ctcccactgt gcgcctgcca      60

acagcaccaa gtttatttcc aggcagtggg taagcagtgc tgagaacttg ccccgggcta     120

ccagcctccc agctgagaga gcaggcaagg cttttgcgct tccctgcctg ttgagtctgc     180

ccacaagatt cacgccatcc actgagttct ggtcaggaag cttcatgttt ggttggaatt     240

attaccaagt ttgtctagag gtttccttct ccctgtggtc ttttccaagt tcccctggta     300

gccctcccca aggacgccta tgagacaagt cagaaatggc ttccctgggg acccagaaat     360

cccacggggc ttttcctgct gcttcctcta ccctgtattt t                         401

<210>  124
<211>  401
<212>  DNA
<213>  homo sapiens

<220>
<221>  variation
<222>  182
<223>  /replace="t"

<220>
<221>  variation
<222>  285
<223>  /replace="t"

<220>
<221>  variation
<222>  325
<223>  /replace="g"

<400>  124
aacagaacaa agacataaaa acagataatg gtatgagtac tgcagttgaa atgcaattag      60

ggattcttta gcctacttgg tctttacacc tcttgccctg ttccccatcc ctatgtggca     120

tcacaagaaa tcttacagcc tgaaaaatat ggaacataaa acactgttct gtcatttatc     180

acacaaacag ggtcaatttc tagaataaaa gaagacattc ttaataaatt tattctggga     240

aaaagtctta aacgggtgat accaggaaaa ttgggacata cggtcatcct ggttctatac     300

cacaagagtt gaaatgagag gaaaatatgg ttaagtgaga agaaatggat tccagcccca     360

tttctatcat ccacgagctg tgtattctta aaaagttact c                         401

<210>  125
<211>  401
<212>  DNA
<213>  homo sapiens

<220>
<221>  variation
<222>  81
<223>  /replace="t"

<220>
<221>  variation
<222>  212

<223>  /replace="t"

<400>  125
atgactggca cccaaatagg cttgctgggt tggtgcactg tcaggtttcc aattctttcg      60

atgtgtctgt agccttggtc aggccaggtc tgccttgtcc tcctggatgg acatttagga     120

aatgcatcct aaccccatca gcacagtcat gcactggtct tcttttccca tatgtgtgtg     180

ccgccatctg ggacaactta cccattgtct gctagggcct ttgatgcttg attataggga     240

aagaggaaaa catcagctac aatagagcaa tctgttctgt ttgggacatc ttacaatctt     300

ttttcctcct tttctttttt tcaatgcaca tatttttgat catcttcaca tagggatgta     360

tattggtaat ggaatgtatt ctagaattct ttctcttgaa a                         401

<210>  126
<211>  401
<212>  DNA
<213>  homo sapiens

<220>
<221>  variation
<222>  348
<223>  /replace="t"

<400>  126
taaatattat atgctattaa ttctaaggtc ctgacataat actctgccat taagcttttc      60

atctggaggt tgtatattgt tttgtaatta tttgctaatt aatcaggcac tagttctaga     120

atttaggaaa caccagctca gtagcttctc aaccatgatg aagagtagac agtattagaa     180

aaacacagta gtgggagagt agaagattgg gggaaggaaa agagagtccc taagcacaaa     240

gtgtgcttct ctacctctcc tccttctcca ttctgtgctt ccctggcaga tcttctgggc     300

gaggagccaa gagaaggttt gaaagtttgt ctgcagctag ataaacaact ttagtggaga     360

gatggatttt ggcgtcactt aggaggtccc acctaatttt g                         401

<210>  127
<211>  401
<212>  DNA
<213>  homo sapiens

<220>
<221>  variation
<222>  56
<223>  /replace="g"

<400>  127
ttcaattttg tcagtgtttg cttcatatac ttgaggtgtt ctgtgaaagg gctgtatata      60

caacatgatc tccaaatgca gattgagcca agaaatgaaa aaatgagtca gacaaatcca     120

gtttgtcagt aaatggtgat ttattagagg acttacagac agaaatgtgg tcttgggtgg     180

ctgcaagcca ggaagaattc tgcagtgcaa ccctctaggc ccagggctta tatatcttgg     240

gtggggggta agtacatatc cctggaagaa atgtgtaggt gacttaaaga atactactgg     300

catcacaacc tatgatgcat ccaacatcaa gggctgtttt ggaggaaagg tgaaacttac     360

aataaatagg tatttctaca tagggagtaa tatatcaatg a                         401

<210> 128
<211> 401
<212> DNA
<213> homo sapiens

<400> 128
actttcactt tactccattt ctaagtttga tattctggta gaattaaagt aagagttttc 60

tttctttcat ctccttttta tctctctcat attgatttct tttaatggaa aattttttta 120

tttattattt tatcgttccg atagactttg ccgagcatct ctcctcaaat ctgtatccag 180

attatttcct caattgtctt aagttggctg tgctatatca ttagtttatg tctttttatc 240

ttcccatgta aatactttac taattctggg accagaatag atatctttgt atgtgtttta 300

gttagctatt gctgtataat tccagtatcc ataggcgcac tttgttaaag agtggctcat 360

ccgtctatgg tcacatgaaa ggaagtacaa ttcagggttc t 401

<210> 129
<211> 401
<212> DNA
<213> homo sapiens

<220>
<221> variation
<222> 157
<223> /replace="t"

<400> 129
aaacattgta atttaagaat ttcttttgaa atttttattt tcctctgcca gttaattcag 60

tgttatttta ttttaccagt tgggatcaat aaatctttgt tacagtcatc aaccatcact 120

atgataaatt ttgaatttca tgagctactc actcaacatt ttttaaatta tttcaaaatt 180

ggacacttaa gttcatctat aagctgggta taggaaacag ttaagaatgt gtaggtatca 240

gtgcaaactg tgtcttcctg tacctagaaa atcccctgaa tctttctccc attccaagga 300

cagaatttct ttttcctact ttgcactttt tccttctctt taattccatt aaaatgaagt 360

tgcaatctct agatggcaga tatttcttaa gcacaaaaac t 401

<210> 130
<211> 401
<212> DNA
<213> homo sapiens

<220>
<221> variation
<222> 387
<223> /replace="g"

<400> 130
ccacagcgtg tgatctcttt gcacatgctc acccccactt aaactttccg tcattagtga 60

aaacatcatg aggccattac cagatgcagc tgcctaatct tggacttttc agccaccaga 120

accatgagcc aaatatacct cttttcttta tatattaccc agcttcaggt attctgttgt 180

agcaatacaa aatagactga aacagtgtgt gaatgtggaa aagtctggga atgtgcaagt 240

aggcagtaag atgctgtcta accaaagcct gaactgaaag ggcagtgatg ggtatttctg 300

ttttcccaaa gatttttcat atcatagtgt aattatatca acaagcaccc aaagttttca 360

gaagtttcac aatatgccta cattaaagca taccagatta t 401

<210> 131
<211> 401
<212> DNA
<213> homo sapiens

<220>
<221> variation
<222> 46
<223> /replace="t"

<220>
<221> variation
<222> 69
<223> /replace="g"

<220>
<221> variation
<222> 108
<223> /replace="t"

<220>
<221> variation
<222> 323
<223> /replace="c"

<400> 131
cttcaccttc tcaggtaata cctgatagtc caccaggcag acggggttgg cgatgcttcc 60

agcactgaac actctctggg tttgctagaa ctacccaggc caacacccca ccactttttt 120

ttcctctcat ttttgtaatg tggcttctta taaatttaaa cttgtttcag catttgcctt 180

gttgcctgtc tggaaataac aagttgtttg ttttgcttta taatttgaga aacaggctac 240

cattcccttc ccatcatgga aaacattctg caatgatctg tatcatcttc caatcatggt 300

aatcactgac taacctgttt tcaccagaga ggttttttat ctaattggaa gtttgagcct 360

gaaactcaga gaagataaat ggacaatttt ggtctcacgt g 401

<210> 132
<211> 401
<212> DNA
<213> homo sapiens

<220>
<221> variation
<222> 141
<223> /replace="t"

<220>
<221> variation
<222> 337
<223> /replace="t"

<220>
<221> variation
<222> 392
<223> /replace="c"

<400>  132
tttttactcc actcttgcta tcttacctcc tgcttttttt ttcagtgtat gtctcctctg      60

gtcacatgat tatatttatg gggtgcactt gatcctctac tgtgttattt tctattccta     120

catgatctta tttagtaccg gggctttaaa gaccatctgt tgatgactcc caagtcagta     180

tttctggctc cattctcctc aaatggtaaa ctcaaactga atatctcctt ttggatatct     240

gaaaggcatc tcaaatccaa catctcaaac attactctga gttctccacc ccatatgctc     300

ctctaaaatc tttcccatgt tattaaatgg tcccacctta aacacacacc taagagcaat     360

tcttcactct tctccttccc aaaagatcta catctaattt a                         401


<210>  133
<211>  401
<212>  DNA
<213>  homo sapiens

<400>  133
aaaaataaaa gacaaaagta attaagtggc aaataaaaag tagagtggat cgttaagact      60

aaacactatt tttaaagacc agtataacag acaaatgttt tgatggtgaa agagagtgag     120

agtgagagag agaaagtgag tgagaaataa tataaacaaa caatatcagg aaaaaataat     180

ggagcacaaa catataatga agtattttag aaactatgaa ttgatatgaa caactgtatg     240

ccagtaggtt aaaagcccat gcaatggaca attttctagg aaagtgtaaa tgccatgatt     300

agtcttcaca accataaaac aagtattaat acaaattgaa atggtaatca gtatcttatt     360

cccaaaggca tcagacccac atggtttttc cagtaacttt c                         401


<210>  134
<211>  401
<212>  DNA
<213>  homo sapiens

<220>
<221>  variation
<222>  5
<223>  /replace="t"

<220>
<221>  variation
<222>  45
<223>  /replace="c"

<220>
<221>  variation
<222>  266
<223>  /replace="g"

<220>
<221>  variation
<222>  341
<223>  /replace="g"

<400>  134
ggcactattc ccaatagcaa agacttggaa ccaacccaaa tgtcgaataa tgatagactg      60

gattaagaaa aagtggcaca tatacactat ggaatactat gcagccataa aaaatgatga     120

gttcatgtcc tttgttggga catggatgaa gctggaaacc attattctca gcaaagtacc     180

acaaagacaa aaaatcaaac atcgcttgtt ctcactcata ggtaggaatt gaccagtgag 240

aacacatgga cacaggaaag ggaccatcac acactgggga ctgttgtggg gtggagggag 300

gggggaggga tagcattagg agatatacct aatgctaaat acgagttaat gggtgcaaca 360

cagcaacatg gcacatgtat acatatgtaa caaacctgca c 401

<210> 135
<211> 401
<212> DNA
<213> homo sapiens

<220>
<221> variation
<222> 323
<223> /replace="g"

<400> 135
gaatccggga ggcagaggtt gtggtgagct gagattgcgc cattgcactc cagcctgggc 60

aaaaagagtg aaactccatc aaagaaagaa agagagagag agagagaagg agagaaggag 120

ggaaatatta tgaaaaaaag tgagacagag aaaaccatac agaccagagt gaaacgcagt 180

taaagctaat ccgtggtggt agaggtcaga atatagatac cttgaagact ggctggttaa 240

ggaacccaag gaaacctttt gaatgtttct tgacatggtt gcagattaca tgagtatata 300

cataagtaaa aattcattat gcattttatc gtatgtaaac tacacctcag tttaattgaa 360

aaatagatct tatatatctt ttattatgtt tattgacttg a 401

<210> 136
<211> 401
<212> DNA
<213> homo sapiens

<400> 136
atttacaaaa tatatctttt cccttcttta aaaaaacttc atagtaaatt atgcataaag 60

cttaccatca tatcattttt aagtgtacag tttactggtg ttaagtacat tcatgtccat 120

cactgccact gatctccaga actctcttca tcttgcaaaa gtgaaactct atacctcatt 180

cctccctcca tttatgccac atcaaaccac cattctattt tctgtctgta tgaatttgac 240

tattctaggt atcttatata agatacctta tataaggtac cttatatctt atataatatc 300

atctatatta tataagtggt atcttatata agattccact tatgtggaat catatagtat 360

ttgtcctttt gtgactggct taattcacat agcataatgt t 401

<210> 137
<211> 401
<212> DNA
<213> homo sapiens

<220>
<221> variation
<222> 114
<223> /replace="t"

<220>

<221>  variation
<222>  281
<223>  /replace="c"

<400>  137
aaataagaag aaaaaaagga gtcatctctg ggactacacc ttgagaatct caagttttaa      60

tggatggctt aggaattact caagttagga ggcagaaaga gccagcagag aagcaggagg     120

aaaacaggaa agagctgtga cccagagtaa agggccatag aggtcagctg tatcagatgt     180

gaccaagagc tcaagtccaa aaaggtctga ataataaccc ttgtgatggg gaggttatga     240

ctgaccccca ggggctattt cattgaaata atgggatcag aggacagttt ggagatggtg     300

gggtgtgact gggaaataag aaagttggaa tagcaagtat ggaggcctct tcccagagag     360

gaagaggtgg catggcctct ggagccagac aatccaagtt t                         401


<210>  138
<211>  401
<212>  DNA
<213>  homo sapiens

<220>
<221>  variation
<222>  124
<223>  /replace="c"

<220>
<221>  variation
<222>  254
<223>  /replace="t"

<220>
<221>  variation
<222>  343
<223>  /replace="c"

<400>  138
tgtccatatg ttcacagcag agtttttgat gtgtttgatt gtggggtgat accgcagacc      60

ccactgaggg tataacacac accaggtgac ttcactaaaa tccccacggt gaaacacatt     120

tgcggccctt ttccctgccc ctggattttg ctaaggatct ggggggttct gtggaagtct     180

gaaggtcagt ggacaggacg agtttggaga tgcgcatttg gaaactgtac ccctcaagtg     240

acaaggaaat gagcgtggat ttaagaagat aatgaccgtt ctgtgaagtt ttaatggaat     300

ttttaagcaa gaaaaccctt accctgtgaa aacttcagtc tgcacagaag aattggtggg     360

agaaggaaga ctttgcctaa gatcacttta tatagcacaa c                         401


<210>  139
<211>  401
<212>  DNA
<213>  homo sapiens

<220>
<221>  variation
<222>  142
<223>  /replace="t"

<220>
<221>  variation

<222>  374
<223>  /replace="g"

<220>
<221>  variation
<222>  395
<223>  /replace="g"

<400>  139
tatacacaca aggaaacccc ctcccccaaa cccaccagaa tattgttttt acctctgggt      60

gttgagacta tagagtttta atttctttat atattcttct gtttttctag aataaatgca     120

tattatttta taattacaaa ggctactttt taactcttca taatcataac ttataatggt     180

aaaatttata taaaatttca atctgtgcat atgtaaataa attaactttc ttgttatgca     240

tttaagttag ttataggttt ccactgtttt cttcagacaa tgattatcat tgtgtgtaat     300

ttcaacctag taactcaatt gtttctgtag aattgatagt agaattaatt actcaatagt     360

tatggatatt ttaagactac ataaatgcta ttcaacactc t                         401


<210>  140
<211>  401
<212>  DNA
<213>  homo sapiens

<220>
<221>  variation
<222>  1
<223>  /replace="t"

<220>
<221>  variation
<222>  160
<223>  /replace="g"

<400>  140
cctagtgcac atgtattgca gtactagaat tgttaaccca tacccacatg ggaaaaagtt      60

tatcaactag agtacagtgc ttatgtacaa tgttttttgc aatctgaggc tccactgatt     120

tctaaaatta cttaggtcag catctttttt cttcacccta ttcagtgaag tgtttcatcc     180

gtttgtaata cagttagatt atttgtaaca gtctgaattt cattttgaga tccctgacct     240

gctaaattat acttttaatt ggcacatatt attgttccta ctattcactt ttttaatgaa     300

gtgaacttca cagatatgaa attaaccatt tgaatgtgaa caatttagtg gcatttagga     360

cattcacagt gttgtgtaac tacctctaac tagttccaga a                         401


<210>  141
<211>  401
<212>  DNA
<213>  homo sapiens

<220>
<221>  variation
<222>  122
<223>  /replace="t"

<220>
<221>  variation
<222>  212

<223>  /replace="c"

<220>
<221>  variation
<222>  399
<223>  /replace="g"

<400>  141
gttgctgtgt cttcgttttt ttcagataac ccccaaatca gtgaataaac tagttcagag      60

tcacacggcc agtaagaaat agggtcaaga tctgagctca gcaaagtctg tctgcttttt     120

tctacatcat accatgcttt taagcttaat acataagaca gattatttcc tgcttcattt     180

agtcgtccta tttctgcctt atttagaaat agttccaagg cgatttagtt tcttgcactt     240

ttctaccctg ttgctgctgt tttatttttc ttatacttca tttcacttga actcctcacc     300

tccagcttca tttattctct tggggtccaa aaagaaaatg attataaacc aaggaaggtt     360

gcaatgatcc tttcccccag ggaattttcc cagggctgaa g                         401

<210>  142
<211>  401
<212>  DNA
<213>  homo sapiens

<400>  142
cttgctttga ccaatgggat gtgagtggac ataatatata ccagtccaag tagagaattt      60

aacccaagtc aaacatgtgt ttggctcagt ctcctcactt ctgcccactg tcctaagagg     120

agttttctcc aggtaaacac tactgcttca acctgggtcc cagaaagaca aacacatgct     180

ctagatctgt gtcacctacc attgccagct gacccaacac agaggactta tgaaaaatac     240

ataatagttg taagctactg agatttggag ttttttatta tagagcattt tcatagccaa     300

agttgattaa ttcatacatt aaacatcttc ttaaccacaa gacttccagg gcattcagtt     360

tgctgatgtc ctctgtagat ctcaaaatta attggcccta g                         401

<210>  143
<211>  401
<212>  DNA
<213>  homo sapiens

<220>
<221>  variation
<222>  3
<223>  /replace="t"

<220>
<221>  variation
<222>  273
<223>  /replace="t"

<400>  143
aggtggaatt ttgaatcaca tgggaagttt cagtgtttca aagcatgaag tagagcttcc      60

ctaacccctg ctatgactac tctgctttca gtgttggaaa ggaaatgatc ttttaaaagg     120

tggaatatgc aaaaggggaa aacagttctc ctctgtcttt tcctggggtc gggaggggga     180

ttgaggcatt tccccaaaga tccctgctaa ctctgtgacc ttccaagatt cgttaactgt     240

gccagagaag gaaatgtacc tgcaattgct cactggggta atatttaagt caaggctgga 300

tttcaagctc aatttaagtg ctacattttg atttttttta agaagaaaga tttgcctgta 360

aaaatgctac ttttcttata caagatctat aaacatgctt t 401

<210> 144
<211> 401
<212> DNA
<213> homo sapiens

<220>
<221> variation
<222> 217
<223> /replace="g"

<220>
<221> variation
<222> 353
<223> /replace="g"

<400> 144
ttccctaatg attagtgaca ctgagcatct tttcatgtgc ttattggcca tttgtatatc 60

tgctttgggg atatgtctaa gtcttttacc cattttcaaa attgatacgc agcagatgta 120

catattttcg gaattcacat gacaatttga tacacttata taatcaaatc agattaattg 180

gaatattcgt tatcttaaat acttttcttt atactaagaa cattcacaaa attatctttt 240

agctattttg aaatatacaa tgaattaata tctgcagtca taccacctga atgcatctca 300

tctcatctgc ccatttttta attgggctac ttatattttt gttgttgaat taagaaaaat 360

tagattcata gcttaattat gatgaatgta tttatcatct c 401

<210> 145
<211> 401
<212> DNA
<213> homo sapiens

<220>
<221> variation
<222> 256
<223> /replace="t"

<400> 145
gaatatactt ttatgctcaa acataaaatc tggtaataag gttattatta tatctatatc 60

tcagcttcat ccaatttgct gtcgtctctc tttattatat acttgattga tggactgatg 120

caccatggag gcattgctga attgaagaga agagacatcg gggtggaatt aatcatgcag 180

cagattgcta cataactctt atttcccatg agatgagaat cacagtgata aaccatgaag 240

acagctgtat gtggcctaaa actaagacca gaatgctcaa ataagttcca atttatcttg 300

tttccagaga taaattagta ggaaggggtg aaaaggagaa atatttgtga atcatacagt 360

agtcacagct attggttgat gtctaggata ttaaaatgtg a 401

<210> 146
<211> 401
<212> DNA
<213> homo sapiens

<220>
<221>  variation
<222>  319
<223>  /replace="g"

<400>  146
gctggcttct gagaggggag tacttattca gagaggaata gaacttaata taaacattcc      60

tccatccaga aaaaaaaagt taactgacac tgggggcaaa agcctggtct ccagctgtct     120

ataaaactgg ctgttccaat atctgaactc acagaatccc tcccccaact gggagttaat     180

ttggagtttg agttgaaaac atgttcaaag ttttgcccta aatcagtcag ccaacaggta     240

tttctgtgcc tgtccttcga gccctgtgct gtacacttaa attttatttc ttaggagcca     300

aaaaaaaaaa aaaaaaaaaa tgaaatctat agaactgatt cataggtcgc aacaaaatgc     360

acaggttggt gctcagtttc ttcctggctc cttatctttc t                         401


<210>  147
<211>  401
<212>  DNA
<213>  homo sapiens

<220>
<221>  variation
<222>  154
<223>  /replace="t"

<400>  147
gctctaactc aacctttcat ttgtttcaag agggaggtat cagaaccaca acaaatggaa      60

tgaaacctac cactaccacc tgcctggcac aaagaaataa tttacaattc acggggaatt     120

tcctgtgttt cattttgtct gaagaactgt aaccggactt gaatgaacta agtgtctaat     180

tgttgcatta tttttcttgt agagatttta gtgttttatt actttctcca gtttacttca     240

atagtgaatt aggttccctc taccatctga aatgttacag ttcgataaaa taggtaaatt     300

aataaagtct aaagtgcttc caagctgagc agagaccttt ctgtgcgctg tgcttttatt     360

gctcttccca gctgatattt ataactgggc catatgtggt t                         401


<210>  148
<211>  401
<212>  DNA
<213>  homo sapiens

<400>  148
ggataaagtc ataggaatat ttttaaagcc tttcatggta aacagaagaa aaagaatact      60

aaaagtacta taattattga gcattgtgag gaagaagcaa gcagaaagaa ggcattgaga     120

tacctctcat agtgaaaaac caatggattt ttgtctacct ttgttaaaat tcaagaattt     180

aatgagtcat ttgttaaact actggaagct tgagattggc catgatggga atatttacat     240

cccagaaagt gaaaaaacaa acagattaga aagcaaccat gttaactaaa aacagtattg     300

aaaagtgagg caggggcagg gaggagttag cagaaggaga ctgcctgttc ttttgttgct     360

gagtatgtat ttttctatac atacgtaagt atttattacg t                         401

<210> 149
<211> 401
<212> DNA
<213> homo sapiens

<220>
<221> variation
<222> 121
<223> /replace="t"

<400> 149
gataaacctt tatagttaga aagagtctaa atgttggtgt ctgtaagcac aataagtaaa 60

tgagtttggt ttttccttcc aaaataactg aacgttcact ttaaatataa tgttagccag 120

ctttgaatcc agaatattta catcttgcag actgtgtctt gcagcctctg aatcaactga 180

ctgggagcat ccgtctctgc atgcagggct gctgaaaggt gggagcagag ataaaagaca 240

ggagatggga agaaacaaag ggagccaaga gatttcatag aatgctgcaa gagacattct 300

tttgatctat ttggactcct ccaaggcggg atccagagaa agaagagtgg caggttggag 360

accacacccc tagattagct ctcctttgaa ctgggatggt g 401

<210> 150
<211> 401
<212> DNA
<213> homo sapiens

<400> 150
ttcaaaaaat gtctctgcat gtcttttgct cgctttttaa tggggttatt tgctttcttt 60

tttttttttt tgagtgtttg agttccttgt aaattctgca tattagtccc ctgtcagatg 120

gatagtttgc aaatattttc tcccactctg caagctgttt gttcactctg ttgattgttt 180

cttttgctgt gaagcttctt cgtttaatta agtcccattt gtctactttt gttttgttgc 240

atttgtttct gaggtattag tcatgaatcc tttgcctaga ccaatgtcca gaaatgattt 300

ccctaggttt ctttctagta tttttatagt ttcaggtctt acatttaagt ctttaattca 360

tcttgagtta gtttttgcct atggtgagag acagaggtct g 401

<210> 151
<211> 401
<212> DNA
<213> homo sapiens

<220>
<221> variation
<222> 218
<223> /replace="t"

<220>
<221> variation
<222> 344
<223> /replace="g"

<400> 151
attgatagtc tagaaacttg ttagagagaa aggaatttaa tcaaaggtag aagtaattta 60

tagggtaagt ccgacatata catactttct ctaagccttg gcttgattgg ttttcatttt 120

tgggatgtgt atttgacatt tgaaacaatt ttggtcaata atgctttcat gcgaggctga 180

gagttctttg acctatacat agaaaatgag ggagagccaa gagaaatttg gtgctggtgg 240

tgactgctga actttcagga tcattaaaat atactgctaa ctgcatgaaa taagcacatt 300

tgactcaagt ttctccttgg ggatggtatt ggtcagtttc tcaagagaga agccctcttc 360

ctctccctta ccaaagactc tgtctccagt taaacaggga g 401

<210> 152
<211> 401
<212> DNA
<213> homo sapiens

<400> 152
aatttcaaat gggtcttgta ttcttaatat aaaccttatt tggatataac atatgattct 60

tttaatatat attgttgatt ttgacttgat agtattatac taaggttttt gggtgcatat 120

tcactaaaga gtagcaccta ctattttgtt ttctggaatt gtctttccct gatttggata 180

ttcttgtttt ttttaactag cgaaatgaat tagtaatttt ttgtcttttt ctgttcccct 240

gagcaattcc tgtattagag ggtctgtttc tttaatgttc agtagtatct catgcctaaa 300

tctgtgtggg cctagtgtcc ttttttccct ctctttttta ttgaaggact aaagctttga 360

atgcttttgc agtttcttcc aaagtttatc atctactcag c 401

<210> 153
<211> 401
<212> DNA
<213> homo sapiens

<400> 153
attacttaaa tttattaata catttttaat atcagatggt aagttacagg atggagctgt 60

gacacccttg tcatgaacaa gcctacagtt gtagacttat gctcacaaag ggtgctcagc 120

tccctattaa tgaattgatt gatgaatcaa ttaaatgatg ttgtgattat tggctatcac 180

tgacacttgc agaaggtagc gctctcttgt gctgatttca tgttaatctc atctaacttt 240

aaggagctca tttcatgaca catggaattt aaaggccagt actctacaat tcatggacac 300

tcttctaact caaagtagct attaaggaaa ccaatcttaa agtcaggatg acagggacct 360

taaagtacca attccatttc cagccttata gactcaacca a 401

<210> 154
<211> 401
<212> DNA
<213> homo sapiens

<220>
<221> variation
<222> 257
<223> /replace="t"

<400> 154
ccatacaata gatttcctac tttaactacc taaatttgtt tcaaaaccaa ccactattag 60

gggagttttg tgaacttact tcatgtgcca ttgaatttct aataggtgat atctaatttt 120

aaattgtagt ttagagaaaa aatgttgcta ttctattgct gaaacataat tgatatataa 180

ggagagtttg gctttgaata catttctttc tctgtatata tttattttag atttcatgag 240

cgagtatttg attcttaaaa tattgaggac attgcaaaac atgattttac tggatttttc 300

agaatcgtaa agaagaataa ttcttttata gtttagtaac tatctaaaat aatttagaca 360

cttttgacct tattttcttg atgtttcttt gagatgagga g 401

<210> 155
<211> 401
<212> DNA
<213> homo sapiens

<220>
<221> variation
<222> 227
<223> /replace="t"

<400> 155
tgtcctttct acctttgcct ggagacttag cagtgcctcc caccacagca gacagaaggt 60

acatctcact ctaccctctg acatcagctc cctcatggaa cttcctttgg ccaatgagcc 120

gagagcagag gcgtgagttg tgtctagcca gagggttcaa atgtgcttgc gtgatttggc 180

ttggcttttt gagcttcagc attctccatg ggaaaatcat gccctgggta ggcattgctc 240

cttcaacctg tgtcctggaa tgaagacaca tggaacagtc tggaacctaa aacaaagcct 300

ggagttcagc tgagatcagc taaggtgtgg ctgggacctg tgcatatgga ataaatgttt 360

gctcttatag gtaaatgcga tgtggaagtt gttcattact c 401

<210> 156
<211> 401
<212> DNA
<213> homo sapiens

<220>
<221> variation
<222> 4
<223> /replace="g"

<220>
<221> variation
<222> 78
<223> /replace="t"

<400> 156
aacattcgag gccagtttgc caaaaatctt ggtgctaact gtatgtccag gttacagatc 60

cacacgcaaa tgcagaccca acaacactga ggctcatctt ttgggtttct ccccatcttc 120

ttcaccatga ctcagttctc caagaaaaat gttgttgcta ctttcaggaa cagtccagga 180

tgagagctta aggtagagtc cggacatgtg catggaagca cagccatgat tctatctaat 240

tggctcaaac aaaagtcttg gacagtattt tggctttttt tttttttttt ttttgagaca 300

tgatcttact ctgtcaccta gcctggagtt cagtggtgcg atcataggtc actgcagcct 360

tgatctccca ggctaaagca atcctcccac ttcagcccca t 401

<210>  157
<211>  401
<212>  DNA
<213>  homo sapiens

<400>  157
tgcctagcct atattttttg tgattggttt tctgagtcta taaagaaaaa aggatgttct      60

tcagctattt ggggactttt ggctccaccc caggcccata agtgaatgca acaacaaggt     120

ttaatgactg ttggacactg tgtttatcga gagacattct gcagggaagc agaaacaaca     180

taattgccta taaaaacatc ctttaaaaag ttagcagtct aatccataaa aatgaatttt     240

ggcaggtgca aatgtagcca gggccaaggc agggcgtgcg gtggatgggg gcgggaaatc     300

cattcaggcg cggaggcttg cctgttgttg tccctggagt gcgccgtcct ctctcttgtt     360

cctgcttctt ttttactctc ctttctcctc agcagccatc c                         401


<210>  158
<211>  401
<212>  DNA
<213>  homo sapiens

<220>
<221>  variation
<222>  68
<223>  /replace="t"

<400>  158
gcacaaccag gatttgaact aaagtttacc ttcatctaaa tacgcgtttt tctttctctc      60

tctctctctt ttttttttta cccctatcgc atcatagcca catctgtggc cacacaagaa     120

atactttccc aacactcagt agctaattat tcataaggtt ttagggtgac ggtgggccat     180

gtgcagagat gctttgaaag acttccagga aaatcatgct ttatggctta gaatttgggg     240

acatgactgt acctgaattg agatggacag gaaatcattt ctccctcctt cctgcttcag     300

cacacccctc ttcccactca agagattctg gagacgcttc aaggccatct ccttgccaga     360

tgcctctgtt ttagaaaagt catttcaagt gccccaagga g                         401


<210>  159
<211>  401
<212>  DNA
<213>  homo sapiens

<220>
<221>  variation
<222>  54
<223>  /replace="g"

<220>
<221>  variation
<222>  55
<223>  /replace="t"

<220>
<221>  variation
<222>  175
<223>  /replace="t"

<400>  159

gaggttcccg agggctctgg ggaggcggaa gaggggacgc ggtgtgttga gatacgggga 60

gaagttcaga gcagcatggg catgcaggtg cctgcagagg ggcccatctg ggctgcaccc 120

cagcaggaca gggccatggc ttggtgtggg ttcggctggc tgcacagctt cttgcaaagt 180

ttgtccccag ggtccttggg agtagaggtc tttcggaagg caccatctcc agagcgtgtt 240

tctgagtaac tctgaggatg ggttccgggt tatcactgtg tgttggaaac agcagcgccc 300

aggctgccgg aggaggctgg tctccctggc aggtcctggc tgtgtctaga gttttgtggg 360

tcctggagat aaggctccgg tttgaggccc ctggggctgg c 401

<210> 160
<211> 401
<212> DNA
<213> homo sapiens

<400> 160
gaagtgtctg tgtacacagc tcctgacagt tcctgcctgc ctggctccta gcccagtgct 60

gttagaaatc tttcccaaac agctctacct ttttgaaagt ccagaccctt gacatccatc 120

tctccctact gctcagaatt gtctgtatct atcttttacc aatgggggaa aggaaagaaa 180

aggcagataa agagtgctgc accacagaac cttgggggat taaaaagaag caggctgtgc 240

tgagagccta ggatcagaaa gaatctcact gcaccctcac cttctttaac ccaggcatag 300

acttccctcc atccattcca atacctggag tgtctcattg gtgtagatga aaacacggca 360

gtcaggtata agcaggtcct ggagctctga ctagctcatt t 401

<210> 161
<211> 401
<212> DNA
<213> homo sapiens

<400> 161
caaatacatt tgttttatga gtgtcaaagg ctctaatata ttaatattac taatatatta 60

gtacaaaata ctaatataat actaatatac tacaatgtat tatgtctatt atatataata 120

catcagtata ttagttatat taatatatta ctaatgcatt aataaagaaa aaagatgtta 180

aactagacag gatccatcgt gtccttgtgg cattttgatg gaggccttca cctcaacagt 240

gagttaagcc tagagtatga ggatgagttc tgtgcattag ccactttaaa gtttgtttgt 300

cgttactcag tatacaacag agaaaacctc aaaatccctc ttcacttctc ttccaactct 360

gcttttccca gtgtgagtag aatcaaacca tatgtactgt t 401

<210> 162
<211> 401
<212> DNA
<213> homo sapiens

<400> 162
catgtaagaa ggattttatt gttcttctat gcttagcatt ccttctgcta tgtggaatag 60

tcctggagta acttgacaca cagaaagtac catattttaa acagaagcaa ttcattgtag 120

tggcttctgc gagcaataga aacttgcaga acgtacaagg aattcaaaca tctcaacaga 180

aaaaagccca ccaagtttcc acaaaaaact acaattagaa ctgccatatg atctggcaat 240

cccatttctg ggcatttatc caaagaaaag gaaatcagta tatcaaagag acacctccaa 300

accccatgtt cattgcagca ttattcacaa tagctaagat atgaaatcaa cctaggtgtc 360

caacaacaaa tgaatggata aagaaaatgt ggtatatata c 401

<210> 163
<211> 401
<212> DNA
<213> homo sapiens

<220>
<221> variation
<222> 136
<223> /replace="g"

<220>
<221> variation
<222> 276
<223> /replace="t"

<400> 163
actatgcatt atatgtatcg aaacatcact atgtacccca tgaatatgtc aattaaaaaa 60

atagtttcac caagacccac taaatcaaca gaattttgtg gcaacattat gcagacccca 120

ctgttgagag taaacactat ttgatatgat atcaagttcc atcagtttgc atcagatcat 180

cagatgagag ggccataagc gtcttctgtt ctcataatta tgcagattta ttgttttgaa 240

acaaaatcaa cttctctcta aaataagcaa ggacactggg gcaaccaact attctcagag 300

aaggagtgtt tttaattttt ttcaggattc ttaggaaaat caaatatcaa ataacataca 360

ggaatgtaga gttcaatgac aaaaaggaaa tctcttgact g 401

<210> 164
<211> 401
<212> DNA
<213> homo sapiens

<220>
<221> variation
<222> 61
<223> /replace="g"

<220>
<221> variation
<222> 126
<223> /replace="t"

<400> 164
cccactaaat caacagaatt ttgtggcaac attatgcaga ccccactgtt gagagtaaac 60

actatttgat atgatatcaa gttccatcag tttgcatcag atcatcagat gagagggcca 120

taagcgtctt ctgttctcat aattatgcag atttattgtt ttgaaacaaa atcaacttct 180

ctctaaaata agcaaggaca ctggggcaac caactattct cagagaagga gtgtttttaa 240

tttttttcag gattcttagg aaaatcaaat atcaaataac atacaggaat gtagagttca 300

atgacaaaaa ggaaatctct tgactgtatg aatagtttca tgaagtcaca gcattggttt 360

aatagcaaaa attttatttg gaaagaaact tacatttgga t 401

<210> 165
<211> 401
<212> DNA
<213> homo sapiens

<400> 165
tgttacctat tatgattaat attgttttcc tttttttctt gcaaatagtt ttaagtctcc 60

ttcagttcta ttcatttttt ttttctctaa agagcttccc cagcattcct ccattttctt 120

gaaactgttt atcatgcttg ttttgtgtca agccgtgggg ctctgtagag catgaaacaa 180

gaataagatt ctattactta accttcattt aatagtatac atttgccaat taagagcttt 240

acacaaattt tcttattaag tcttttcaac aacttctgga tatggataat gttaattctt 300

gtccatgata caattggaga aattgaggct taaagagatg aagtcacttt tcaagtgcta 360

catagatgct aagtgataaa gaagaaattc aaacccagga c 401

<210> 166
<211> 401
<212> DNA
<213> homo sapiens

<220>
<221> variation
<222> 45
<223> /replace="g"

<400> 166
cccttcttca ttttaaattt attttatttt tagttttgat taaaagaccc acacataaac 60

ataaaattta ctatctttac catttttaaa tgtacagttc agtagtgtta catatagtca 120

catcttgctg caatgtccct ccagaagatt tctgtcatct tctttggaaa attttaagaa 180

actattacca aaaagtgtac cgagatatag ttttaaggct gatgtaccaa tgacaccacg 240

tgaataaatt ctgtacattc acctgtaaag ctcctcagct tttgggcttt tttctcctta 300

ttggctggtt ctaaataaat tttatgaatt cccaataaac ggcttttggg aaatgccagg 360

tcatcattgt aataaaaagt ggtataaggg gaaaacaact c 401

<210> 167
<211> 401
<212> DNA
<213> homo sapiens

<400> 167
tgtatgacat cactgacagg gactaattta aaagatttaa ttattcccca attcacacat 60

gtcttgattt atttttgata aagagtagca aatgggtacc ctaggattct atacaaataa 120

gccagtgtaa tttgatggaa taaacactga tttggatgtt aagagatttt cagctaaccc 180

tggctcttct gataactggc gttgttactt ggggaagtca gttaatctct cgtttgtaga 240

atgatggggc taaactaact ggtctcttag aaattgtttc ttcaagacct aaccttctgt 300

ggttctcctt aatgcctgga tgcaatgacc ttactactga tggaaagatt atcttgggaa 360

atgttggcca aaactaaacc ttctccccac acatctgttc t 401

<210> 168
<211> 401
<212> DNA
<213> homo sapiens

<400> 168
acagaaatta gaagctaagc attgcaattt aaagaattat aggaacttag gaatcataac 60

aattttcttt aaatatcagg ttatttccct taaataagtt atgtgcaagc acttcgtaaa 120

ttctaaggtc ttaatcaaaa ttttgatttt attactatta ttattgggct atgtgaaaaa 180

gagaagtgaa atactaagcc catgaaaaaa taagatttta tttaacataa tttaagacac 240

agagcacttg ttcagactca agtctgttac attcttacat agagtaactc acattattgt 300

tggaaccaga gctacagttc gtatctttct tgagccttcc tcagggtcca gagttctgag 360

agaatttcat tatcaccatc tcccaagaaa aacaagcttc a 401

<210> 169
<211> 401
<212> DNA
<213> homo sapiens

<400> 169
tattttatgt atttcccatt gttctgctat tacaaatagt tgttcagtaa gcattcttgt 60

acatttatct ttgtgcaatt gaagaccaat atttctctca cttatgtagt gccacccttg 120

ctagtaggaa tgttccgtta aatgcaaaac ctgcagaact tggactcttc aacatgtttc 180

tactttgcca aaactgtcta cgcctccctg tgcttgcttg ctgctccttg gctcttcttt 240

gtctttaatg ttgcccttaa ctctaataaa agcctctgtc ttagtttagg tttccccaga 300

aacacattta agacaaggat ttgaatgcaa gtggcttatt caagaggtgg cctcaagatg 360

cacaggtagg ggagtggagg gtggggcgga acagggaagg c 401

<210> 170
<211> 401
<212> DNA
<213> homo sapiens

<220>
<221> variation
<222> 56
<223> /replace="c"

<220>
<221> variation
<222> 77
<223> /replace="t"

<220>
<221> variation
<222> 133
<223> /replace="t"

<220>
<221> variation

72

<222>  246
<223>  /replace="g"

<220>
<221>  variation
<222>  350
<223>  /replace="t"

<400>  170
actaataacc tggtctctca ctcatcctga ctgggctgcc ttcccttccc tgtctgtctt      60

cccctttctt ctccctctcc tcctgggatc acctcccata caaactcctc accctcattt     120

ccttgtccca aggtctactt ttcagggaac ccaggatact ttcctcctgg tcctaagaaa     180

acccaggcaa accaagtttc ctgctctcat acaatttaca gcatgttggg ttgaacaggc     240

aacttaacaa acattaaaaa cgaagtgtaa cgagtgctga ggtcagggag catagactgg     300

cggaaggcac agagccacag gggaccctaa gctagcccag gagaggtccc agaagctgag     360

ctgggagtga ctggggctcc ctgccttttc ttccccttct t                         401

<210>  171
<211>  401
<212>  DNA
<213>  homo sapiens

<400>  171
ggttaaatgc ctttcagggt agctgaaaac tacttaccaa tcagcagctg gcagaaatgt      60

gtaccaagag ctctcatctg gaagtgggca gctgactcat gttggagttg tcatccctac     120

tccagcagcc ttctgggtga aaagttcttc aaagcacatg catgtgaggc cagaccattt     180

ctcttctgtt ttctatgctc ctcaccaaaa aagaatgagt catggagctc tctaattcag     240

gatccagtag agcatcagac agaatgcagg ctgttgtttg aacatccgtc tccagataga     300

ctgcagtgtc atttaaacaa agatgtatgc atttgaatat ggtgccatac aattcttgtg     360

ccgaactggc atatcttctg agggaagatt ttatagaaat a                         401

<210>  172
<211>  401
<212>  DNA
<213>  homo sapiens

<220>
<221>  variation
<222>  27
<223>  /replace="g"

<400>  172
ggaaacaaaa aggattataa gaaagtaata taagcaactg tacaccaaca aattggataa      60

ctgaaatgaa atggacaatt tctagaaata cacagtgtat caaaactgaa caacaaagaa     120

aataaaaaat ttgaatagac ctataactag taagaaaatc tcctgatata gagaagccct     180

gggctccatg gctttactgg ctaattctac caaacattaa aatacaaatt aatgccaatc     240

atcccaaaaa ttttccaaaa aattgaagag gaatgaatgc ttcttaactt attctataaa     300

gccattacct taatactaaa gccagacaaa gacattacaa aaaagaccaa tattccttat     360

aaacattgat gtgaaaactc tcaagaaaat actagcaaat t 401

<210> 173
<211> 401
<212> DNA
<213> homo sapiens

<400> 173
aaagaaagat atttctagca catatgagtt tatggatgga gactcatgcc tgccacactg 60

tgaaaacatt cttcccttca aaatccctat cagtgtaatt tgaagtgaat attctagtac 120

cacatgcttt tggactctaa ggaagagata taatgtacga aaatgtggta tctgaatgca 180

gcaaattgaa aaataccagt ccagatactt tggggccatg cagagagttt cccagtcagg 240

tgtaagtgtg ggtggtggtg gtttgaatac tgaagggatg gacaggtggg ggtgacaact 300

aagagctgcc acctggggag aatcctaggc agagtctggg cctggacaac ccaaccagta 360

gtgaccgtgg gccggacgag cctctgggct ctgcagctcc g 401

<210> 174
<211> 401
<212> DNA
<213> homo sapiens

<220>
<221> variation
<222> 4
<223> /replace="g"

<220>
<221> variation
<222> 68
<223> /replace="t"

<400> 174
tacactaatt catgcaattg tatattttgg aggtaagtgt aatggcatag gtttgctgta 60

atggaaccga ttggaaaatg atctcttgga gaaaatttat tgctggtcat aagatttctt 120

tggtttagaa aaatgctggg tttagtaaaa tctgaggcac taaatgaaag tctgctttaa 180

taaaaatgtg gatctgagaa ctgtggtttt tagtaagtgg gcatttgatc cattaaaatt 240

gtattggttt tattctttac gttggctttt cctgaaatcc atcagtttga agcatgaagt 300

aagcacttgc cagatattgc tccaataatg aaacattgaa agcaaggccc tgaataactg 360

ttaaaacaag tcctattaat cagctgtgac ttgtcccttt g 401

<210> 175
<211> 401
<212> DNA
<213> homo sapiens

<220>
<221> variation
<222> 106
<223> /replace="c"

<220>
<221> variation
<222> 294

<223>  /replace="c"

<400>  175
atccaatcaa ccatcacaga aagaaagcat gaagtccaga tgactggacc aaaagaacac      60

cctaggttgg atggatgctg gatggccaac tggatgggaa aaaagagggt cttgataaca     120

ttctaattag ccccaaaccc tctattccca tgagccagtc tcttctcttt aagttgatgc     180

tagctgccgt tttgtgttat ctgttacaga ctaatacaat ttgcaataat tgaagatgca     240

atatttattg atatattatt atcaattgaa gtccatagct tacattagga ttcactctta     300

gttttgtatg ttctgtggat tttgacatat gtaaaatgac ttgtgcccac cattacagta     360

tcacatagaa tggtttcact gccccaaaat accctctgtt c                         401

<210>  176
<211>  401
<212>  DNA
<213>  homo sapiens

<400>  176
aaatggactt aagccattac ctttggctgt cctagcaggg gagggtttca gactttcatt      60

tctttcccag cctatggtgt gaggggaaaa gttgtcttgg catggagata cactgtcagt     120

gcagactcct caaagatgaa tggatgtgca tttaagaaca gatagctgag aacatctctg     180

aacatcagtt tctgcatata aaaaatagag ccaataaact cttcctcacc agtttcttga     240

gaaaattaag tgatataaca tgtgggaaat gcctgcctta ggagacatct agaccaacac     300

tgttgcaatg atggacgtct tctatgttct gtactgtcca acagaatagc caccagccac     360

atgtggccac tgaacacttg aaacatgatt aatgctatga a                         401

<210>  177
<211>  401
<212>  DNA
<213>  homo sapiens

<220>
<221>  variation
<222>  280
<223>  /replace="t"

<400>  177
attaagtgat ataacatgtg ggaaatgcct gccttaggag acatctagac caacactgtt      60

gcaatgatgg acgtcttcta tgttctgtac tgtccaacag aatagccacc agccacatgt     120

ggccactgaa cacttgaaac atgattaatg ctatgaagga aattaatttt tacttttgta     180

tgactttagt taatttaagt atgagagcca catgtgcctg gtggctacca tgttggatgg     240

ctcaggtcta gacaatatgt gtaatgttga tcatgatgac gatcatatta gttctgcttc     300

ctcaagttgg agtcaaagtt ttatgctcag ggaacttgtg cctctaggat gggatcagaa     360

aacaaaactt ttccccggca agttgtatca cagttaggtt t                         401

<210>  178
<211>  401
<212>  DNA

<213>  homo sapiens

<220>
<221>  variation
<222>  168
<223>  /replace="t"

<400>  178
ccatgttgtt gcaaatggca ggatttcttt catttgaagg ctaacattcc attggatgta      60

tatgccacat tttctttatc cattcatctg ttgataggca tttagattat ttccatatct     120

tgccttttgt gaacaatgct gcaatgaccg tggaagtact ggtattcctt tgagatctga     180

tcccaatttc atttccactg gatatatacc cagaagtgag attcctggat tgcaaggtag     240

ttctagttct ataatggcta caaccaattt acattcccac caacactgtg tggtttcctt     300

ttctccacat catcaccaga atgttttgtt ttattttatt tttaaaataa tagccaaacc     360

atttggatat ggtttaaata ttcccaatac tagatcaact a                         401

<210>  179
<211>  401
<212>  DNA
<213>  homo sapiens

<220>
<221>  variation
<222>  390
<223>  /replace="t"

<400>  179
aacttcaatg aggaatgcaa cagagagaga gaaaaaaaga aagatcgggt gattgagtag      60

acattgaggt ctgaaaaaat gtagaagtca agtcgaacaa ataagcataa acatgtactt     120

ttaggaagaa gtccatgttg ctggtgccct gctcacattg ctttggccca gccttgaagg     180

acagctgcag agtggattct ctcatctccc ttcccagagg catcctctgg ccaaataagg     240

gggcattctc ggccatcaag ctgagcaggc cagtgagggg tgggcactga ggggcacaca     300

ccagcttgtg tcccactcag aggggcggtt ctgaggcatg ctccctacag tctcccagag     360

tggccccagg agggctaagc ctcagttgcc catgatggca a                         401

<210>  180
<211>  401
<212>  DNA
<213>  homo sapiens

<220>
<221>  variation
<222>  12
<223>  /replace="t"

<220>
<221>  variation
<222>  320
<223>  /replace="c"

<400>  180
   gagtggattc tctcatctcc cttcccagag gcatcctctg gccaaataag ggggcattct      60
   cggccatcaa gctgagcagg ccagtgaggg gtgggcactg aggggcacac accagcttgt     120
   gtcccactca gaggggcggt tctgaggcat gctccctaca gtctcccaga gtggccccag 180
   gagggctaag cctcagttgc ccatgatggc aatgctcatt attgcaccct cgtggcttcc 240
   ttctctttcc tgcctcattt cgtactccca agctgtgctc ccctgcatca cttcctaaac 300
   taaatccctt catctaagaa cttatcccag ggtctgcttt ggggaagaac aaaagacaaa 360
   gcacttatga tatactcaag actaacagaa gcataagctt c 401

<210> 181
<211> 401
<212> DNA
<213> homo sapiens

<220>
<221> variation
<222> 6
<223> /replace="g"

<220>
<221> variation
<222> 348
<223> /replace="g"

<400> 181
gttttataaa atttatgatg tgaggttaca atatgccttt ttcttgtgat tcatccttct 60

tgcaagcttc tatgtagcaa gccggcaaaa ggcagtggaa gggacttctg gggacggtcc 120

tgtcatttcc tgcagtattt tcgtgagtca ttcagcatcg gtctcttccc tactcctatt 180

gtctggctgg agagaagcac cctttcatag gtgagctttt cctacaaaca tgagagatta 240

aaaataagat cttcaaatga agaaataatt tgacatttat tcattgaaaa tcactcatct 300

tcaactcatc ttccatttac agctctaccc ctttctttta tggatggaga aaatgaggcc 360

cagaaaaata aatggatttg tccaagataa ccctgattgt t 401

<210> 182
<211> 401
<212> DNA
<213> homo sapiens

<220>
<221> variation
<222> 213
<223> /replace="g"

<400> 182
tttaaggtat ggatacatct caacttgctt attcattcac ctgttggtgg gcatgtgagt 60

tgtttaagtt ttgggttatt acaaataaag ctgcttgaat gtttatgtat atgactttgt 120

gtgtatttat gctttcattt tttctttggt aaaaaccaag aatggaatag ctgcatcata 180

tggtgattgt gttttttttt aaactttata agaaactgac acattgtttt ccaaaggggc 240

tgtatcattt tacattctca ccagcagtga atgagattct agttcttcca tatcctcacc 300

aatacttgct gtggtcagtc tttttagcta ttctaacaga tatctcatta tagttttagt 360

ttgcttattc ttaatgacta atgatgtcaa gcatctattc a 401

<210> 183
<211> 401
<212> DNA
<213> homo sapiens

<220>
<221> variation
<222> 63
<223> /replace="c"

<220>
<221> variation
<222> 328
<223> /replace="c"

<220>
<221> variation
<222> 359
<223> /replace="g"

<400> 183
gttggtgaat ttttgtctta gcatctgctt ccggcagaat ccaaacaaag acatgagaat 60

taaatttttg gtttcattct tgttttattt tcttaacata catgcttata tgtaaaccaa 120

ttttgaattt tatgtatcaa caatttaagt tctactgctt taagattata tggacattaa 180

atgtacagaa aaatagaaac caacaaaaga taaataaatt aaatacattt ttaaaaaagt 240

ctcgcgagtt gtggtggctt aatattgccg tgagctaatg ccttgagggc tactctctct 300

ggagtgggaa tggagctctc agtaattaga cagttttggc cagtggcttg tcacacggat 360

tgtgcattca cctggcttta ggaggaagag gaggcatggc a 401

<210> 184
<211> 401
<212> DNA
<213> homo sapiens

<220>
<221> variation
<222> 172
<223> /replace="g"

<400> 184
taatgtcctt aagatttaag cctcagttct ttgcgctttc tcttacttgc agcttaaagc 60

attcctaact gatgtttaag aagtttggaa aagaagacta gaagagaaaa acttactcat 120

tgtccttctt cttttttttg ccctataccc aaaataaaaa ttttcctttc tatgtacaaa 180

tttgcacata gctatgacca aatggtgtat gtacaactgt ctttgttgtt tttaaatcta 240

tcttttatct taaaattaaa tcatgtgttc attgcagaaa aactgaagaa tatagaaaat 300

ggtaagggag aaaataaaat cactcagata cctaccaaga gagaacaaat gttaacatac 360

taaggtttct ttttgtaatt tttcataaat ttgatgtcct t 401

<210> 185
<211> 401
<212> DNA
<213> homo sapiens

<220>
<221>  variation
<222>  142
<223>  /replace="t"

<220>
<221>  variation
<222>  252
<223>  /replace="t"

<220>
<221>  variation
<222>  297
<223>  /replace="t"

<220>
<221>  variation
<222>  308
<223>  /replace="t"

<220>
<221>  variation
<222>  329
<223>  /replace="t"

<220>
<221>  variation
<222>  383
<223>  /replace="g"

<400>  185
ccaccagttt ttcctgagga caaggagcct cttaaatctg aactatggtg aagtgtttta      60

gtttgagatc ccgggaaata gactctgaga caaagaactg tgagtaggag gtttcatgga     120

gagtgctctc aggatccaca cctgcgaaat aactataata ataataataa taataataat     180

aataataata atagtgaagg cctggactgg gtagaggaag aaactgatcc aaaacaaacg     240

tgcaatgagg cctcagccaa ttcctggggg gcagatctgg ctgtgtacag cagttcctga     300

ataatgtcgg ttcattcgat gttctttcct tataacgttg atgagaaaac aattgatttg     360

cacctggggc caccatctgt gtatatttgc acaacctccc c                         401


<210>  186
<211>  401
<212>  DNA
<213>  homo sapiens

<220>
<221>  variation
<222>  48
<223>  /replace="t"

<220>
<221>  variation
<222>  93
<223>  /replace="t"

<220>
<221>  variation
<222>  104
<223>  /replace="t"

<220>
<221>  variation

<222>  125
<223>  /replace="t"

<220>
<221>  variation
<222>  179
<223>  /replace="g"

<220>
<221>  variation
<222>  318
<223>  /replace="g"

<220>
<221>  variation
<222>  338
<223>  /replace="t"

<220>
<221>  variation
<222>  362
<223>  /replace="g"

<400>  186
gactgggtag aggaagaaac tgatccaaaa caaacgtgca atgaggcctc agccaattcc      60

tggggggcag atctggctgt gtacagcagt tcctgaataa tgtcggttca ttcgatgttc      120

tttccttata acgttgatga gaaaacaatt gatttgcacc tggggccacc atctgtgtat      180

atttgcacaa cctccccata gctgcatggg ttttctctga gtactctagt ttgctctctc      240

attccaaagt catgcacatt aggttaattg gcatgtctac atggactcag tgtgagtgaa      300

tgtgagcatg ggtgtgaatg tgctctgcca tgagagactg tcctgttcag ggctggttcc      360

cacctcactc cgtgagctgc tgggatgggc tctggccacc c                          401


<210>  187
<211>  401
<212>  DNA
<213>  homo sapiens

<220>
<221>  variation
<222>  357
<223>  /replace="c"

<400>  187
tagcctatgg aaaaattgtt tttgttatat gtcattttgc ttaaagctgc agtttccagg      60

actctatcaa caacattaag tgaggacttt gtgtgatttg cggggccaag tgcaaaatga      120

aattgtaggg taccttgttc aaaatgcaag aaaaaagtgt cattagaggt acagaaacac      180

actttttttt tctatctgtg gtgtctcaaa ttgttatggt ggtgttattt tcttctaaat      240

gtctttttaa ggaaagaaaa ataaaaattt aaattattag caacgatttt tgccattcat      300

ctttatatta tgcattgcta attttaaaca caagtgtgtt taattaactt gtatggagac      360

tcaccaaaat atacattttg tattttatta ctcatgcgtg t                          401


<210>  188
<211>  401
<212>  DNA

<213>  homo sapiens

<400>  188
cagcctcccg aataactggg attacaggca tgcgccacca tgcccggcta atttagtatt      60

tttagtagac atggggtttt gccatgttgg tcaggctggt cttgaactcc tgacctcagg     120

tgatccgcct gccttagcct cccaaagtgc tgggattaca ggcgtgagtc accatgcctg     180

gccgatcata acagtcttaa ctgcaatttg ttaaataata atttaaactg aaatgatatt     240

tacctgtaga gaccccagga tccacagtta tatctgtttc ccaacatcag agactttctt     300

ttatggggct tgcaggaatt ctaccaatat ttactgagta cttctgatga atcagacatt     360

ggtctaagaa ctgtaagaga ctgaaagatc aataagaaaa a                         401

<210>  189
<211>  401
<212>  DNA
<213>  homo sapiens

<400>  189
ttttgagaca aagtcttgct ctgtcactta ggctggagtg caatggcgag atctccgctc      60

ggtgcaacct ctgcctcctg ggttcaagca attctcctgc ctcagcctcc ctagtagctg     120

ggattacagg tgcccaccac cactcctagc taattttttg tattttagta gagatggggt     180

ttcaccatgt tggccttgaa cgcctgatct caagtgatcg gccccccttg gcctcccaaa      240

gtgctgggat tacagacatg agccaccatg cctggcctgc atgactgact cttattgttt     300

gtgactataa acattacttc cccagaaact tgcttcctaa cctacaactt aaaatagtgt     360

ttggtcacta tttgaagagt agacaatcac actttaaata g                         401

<210>  190
<211>  401
<212>  DNA
<213>  homo sapiens

<220>
<221>  variation
<222>  392
<223>  /replace="t"

<400>  190
gggattacag acatgagcca ccatgcctgg cctgcatgac tgactcttat tgtttgtgac      60

tataaacatt acttccccag aaacttgctt cctaacctac aacttaaaat agtgtttggt     120

cactatttga agagtagaca atcacacttt aaatagataa tctaatgcta tgccctattg     180

tattcccctc ataacagtta ctagtgtctt gatattttat ttctatttgt ttattttttt     240

tgtttgtttc ccatctgagg agaataaaag tctggtgaaa gcagagacca tatttatttt     300

attcatcatt ctgagaattg gatgggacaa tcagagctca gtaaatattt gtaatgaatc     360

tgtaaaccaa ggacaacggt aggatccctc tcacaggact t                         401

<210>  191
<211>  401
<212>  DNA

<213>  homo sapiens

<220>
<221>  variation
<222>  10
<223>  /replace="t"

<400>  191
taacagttac tagtgtcttg atattttatt tctatttgtt tatttttttt gtttgtttcc      60

catctgagga gaataaaagt ctggtgaaag cagagaccat atttatttta ttcatcattc     120

tgagaattgg atgggacaat cagagctcag taaatatttg taatgaatct gtaaaccaag     180

gacaacggta ggatccctct cacaggactt tatgagtgtt aaccaagata atgcatgtaa     240

acaccttcgt acagtgcctg gaacacagtc aggtctcagt ggatgccaaa tcattaggtt     300

caggtaataa aattgtatat ggagctccat tcttgccctc aagtgcctca aaggccagag     360

tgaagaagga gatggctcac aaatgaatat gtcatttact a                         401

<210>  192
<211>  401
<212>  DNA
<213>  homo sapiens

<400>  192
gaataattgg tgtgtctgga ctcagtaaca ccagtgtcag catatgccac tctggtgccc      60

aaggacagtt cattgatgac accattggtg cttgaggact ggcctatgtg gcatcccagt     120

tccccacaaa acttcaccac aacctccact aacaactgca ccctaagctg ccaagaaatt     180

atagacactg ctgatgctgt gtccagctga agaagccaca cacaaactac actactgcac     240

atgtccagaa tcaaagccaa gatgccgtac acaaccaacg ttatagctac atctccagga     300

aaatgttttc ctttacaaaa gcaactggaa aaaattggaa gaagtgacca ttacaccagg     360

tgcaaagata tcagtgtaag gatagaggat acatgaaaaa g                         401

<210>  193
<211>  401
<212>  DNA
<213>  homo sapiens

<220>
<221>  variation
<222>  48
<223>  /replace="t"

<400>  193
agtatagata cacaatgaaa tactattcag ttatttaaaa aaaagaacga aatcaagtca      60

tttgcagcaa caggaatgga actagaggtc attatgttaa gtgaaataag ctggacatga     120

aaagacagat accatacatt ccactcatct gtgggagcta aaaatgttga tctcatggag     180

gtagagagta gaatgatgga caccagatgc tggaaagggg gtgtgagtgg gaggtggtga     240

tgaagagggg ttggtcaata ggtgaaaaca tgcaattatc tagaaggaat aagttccaat     300

gttcaatagc aaagcagggt gactatggtt atcgacaatg tgttatatac ttcaaagtag     360

ccagaagaca gaacttgaaa tgttctcaac acatagaaat g                         401

<210> 194
<211> 401
<212> DNA
<213> homo sapiens

<220>
<221> variation
<222> 122
<223> /replace="g"

<220>
<221> variation
<222> 308
<223> /replace="t"

<400> 194
ttctattttt taagagtcca gatttttata tcatttgttt tttatagaaa atgatgctaa 60

cttggtaaac ttgctccagt cactgttctt caacaggatc ctaaaattaa tttcgatttt 120

gagagatttt cctctgctaa ggccaagcga atgaattttc aacacaattc tttaagtgaa 180

atttagaaga gaaatgaagg gtgaaatttg catgtcatgt tcaaatgttg taattctgtc 240

acacctgata tgctattatc atagaaatta taaaaatatt ttaactgtat tatatagatt 300

gttatgacgc atacaaatta tcacttcttt tacaataata tcaggatcca tgcagggaag 360

atatatagtt cctggattct tctttcaaat ttttctgtgc t 401

<210> 195
<211> 401
<212> DNA
<213> homo sapiens

<220>
<221> variation
<222> 15
<223> /replace="g"

<220>
<221> variation
<222> 94
<223> /replace="t"

<220>
<221> variation
<222> 369
<223> /replace="g"

<400> 195
taatttcgat tttgagagat tttcctctgc taaggccaag cgaatgaatt ttcaacacaa 60

ttctttaagt gaaatttaga agagaaatga agggtgaaat ttgcatgtca tgttcaaatg 120

ttgtaattct gtcacacctg atatgctatt atcatagaaa ttataaaaat attttaactg 180

tattatatag attgttatga cgcatacaaa ttatcacttc ttttacaata atatcaggat 240

ccatgcaggg aagatatata gttcctggat tcttctttca aatttttctg tgctaggata 300

ttatcaagaa aacacaaaat atcatgtctt gtttaattta tttaaatctc tcttcacaca 360

gcactataat atccataaaa tactctctgc atttattttg g 401

<210> 196
<211> 401
<212> DNA
<213> homo sapiens

<400> 196
atgttttgga tccattttta ctcccacatt tggcaaataa tttattcatg aacaatgcaa 60

atgaaaattt tttctcagtt gagctgttgt gaaattatta cacaaaacat tttgaaggtt 120

atcaattgta atgtgagagc caaaaccacg tgtgtgcagg cccatctttc tggttgggga 180

ttatactatt tgatataaat cacctcaaga aatagccgta gatgagagaa ataaacattt 240

taattgttct tataatgaat gataagtgaa gggaactata acctcaattg caaatgttaa 300

catttacaag atgacctgtt tataaatgta gctctgcctt atacatgtgc tcacatccat 360

tttgaagtat ccaaccagag cgctgataga ttagtagttt t 401

<210> 197
<211> 401
<212> DNA
<213> homo sapiens

<400> 197
taaagaattt gcaaacaagg gaccaatcat ggtagtgagt aacttctttg tctcttgctc 60

cttttgtaca aaatatctga tttttctcct gcacttgtct gtatctgact cactggttgt 120

cttcttcttt agtttaaggc ctctggaagc ccagagatag gaggcaggga cgagtggagg 180

gcagttttct ctcgactctt cctgcagtta tttaacttct gtttctaatt tataaggctt 240

tcttggcata tgtaagccta tgcagtcaaa ctgagttgtg gtccaggaga ggtcatggac 300

cagtgtcagt catgtgccac atcacagagt gacgtgagat tcggagatga gaaaaatgta 360

aaaagaccct actttttttt cttggcctat cccaataaaa t 401

<210> 198
<211> 401
<212> DNA
<213> homo sapiens

<400> 198
aaccaggacc acactgtaac actgccacct ctgcaacata catattagtg caaattgggg 60

taaagggtgg ggctgataga atgtagagtg taatttagga accagcctat ggtgagatct 120

tgcaactgct ggcaaagtcc gagccactat cctttgagca atccattcac aatggaacca 180

gaaaaacaat gtctatttag ccagaaaaaa tttgaatgga caagagtgat gtctctttac 240

caatacaaga gagataagat tactaggaat agaagagtta catggtttct ctgactgcct 300

tcacaaagtt cagggtggct ggaggtctct gaagaaaagc ctggacatca gtgatgaatc 360

aatctctcaa agcctcctgt agcaaacatt gccaagggcc t 401

<210> 199
<211> 401
<212> DNA
<213> homo sapiens

<220>
<221>  variation
<222>  307
<223>  /replace="t"

<220>
<221>  variation
<222>  308
<223>  /replace="t"

<400>  199
tttttttacc aaagatcgaa actctcaaaa cattttcaga ggcttcataa aatcgttatt      60

tgtgttggaa ttttatatac cacccaaagc tggatgaaat caaagagcca atggctaggc     120

ttggaggtca ttttaaaaac tgcatttttt caccattata atatttattg caatgggcta     180

ggccctacgc atcagattgg cgatattatt aaggcatcag aaggccaaga aaatctgaaa     240

ataaaatggt ttcccaggta aagccatggt aagggtaggc aaaactggca tcaggccaac     300

cgtttcaaac catgcatcaa acttttgagg tcaggcaaaa ttgaaaacac atttggagaa     360

gttaattcac cttaattttg aaacccatgt gtatttcaag g                         401

<210>  200
<211>  401
<212>  DNA
<213>  homo sapiens

<400>  200
ctaagttatc tagtttacca gcctggagtt atctgtagta tgtgatagta tagtatagca      60

tacattttcc cccttttagt atctacatgg tttgtaatga tttcccctct ttcattcttg     120

gggtaatctg tcttcctcta ctctcctgac acatactttg ggcagatccc acctctaggt     180

acagaatgtg attggcaatt cctgtggacc aatctgagca ttgctttcac ttggctgtcc     240

tgattgcttt gaatggagag agtaaggcgc atcaggcaga tacaaagcat tttgctgaga     300

ttactggcac tatataaacc ctgcactccc agggagcagt agaaatcttg agaatggatc     360

cattagagtg taaggcagac gttaaagatg gagaaaccaa c                         401

<210>  201
<211>  401
<212>  DNA
<213>  homo sapiens

<220>
<221>  variation
<222>  35
<223>  /replace="t"

<400>  201
atctccaccc agaaatggag tgctgtagta aggcccactc aagataacga tggcaatggg      60

aagctggagt gacagatggc acacgatggc aaacagccca tacacgttag tgctttctgt     120

ggaaaataat tttgtatata tataaatatc ttttataatt taggaatttc cacattatta     180

agtcaggata gccagtatag cagagataca ggtgtccaat atccctttca tccttcttcc     240

tttagtaata gtttatctga gtgtatactg agtgtggtcc cttgtccaaa ggaaccttta     300

aagtggtggt ggtcacatgc taaacttata gtcagaggta tgtgaatggc gatggtggat 360

atcacctctc agtcatgctc atatctttcc ttctgtccct t 401

<210> 202
<211> 401
<212> DNA
<213> homo sapiens

<220>
<221> variation
<222> 37
<223> /replace="g"

<220>
<221> variation
<222> 102
<223> /replace="g"

<220>
<221> variation
<222> 273
<223> /replace="c"

<400> 202
aagtaaacag aaaatgaata agctgtggac tttgaaagat agtttttgag ttagaaatct 60

gaagggggag aaaaggccac agaaacaggg taaacagtta aagcaaacat tttagaccaa 120

aatagccagt agacagcagc tgagaatgga cctcactgct ggtaaagagg cccaagtgga 180

ttaaattgat caaaacagtg cttgggagat agcaaggaat tttatttttc attatttctc 240

ttcaaaaagg catgatggaa gattagactt ctgtaagaac aaacgtttga aacttttcta 300

ttgctactgt ttctgtgctt tgcatttgat gttggttatg aaacgtgttt ttctcagtat 360

agatatatat gtatattttt atacatacac atacatatat a 401

<210> 203
<211> 401
<212> DNA
<213> homo sapiens

<220>
<221> variation
<222> 40
<223> /replace="g"

<220>
<221> variation
<222> 110
<223> /replace="t"

<400> 203
agtttagtat gttatttaga ctaatatttt ataaatcata ttctggaatt aagctgagta 60

aggccaggaa gaaagagggt tgaggatgga tgtaatgtag gaaccatgcc taggaagcga 120

ggaaaagcat cataaaagga cagcaacaat gtagaggaga gaagttaagc agtcagtgag 180

aggaaggcct ctgaacccac ccaggtactt agctgtagat ggacatcagt taattcgatg 240

acccaccacc ccatctcacc aataaaaacc aaactctttg ggaactggga ccagtactga 300

actagtgggt caaaataaag taaaaaagaa gcccacttca agaaatactc ctctaaataa 360

cttcagagaa aaggaaattt tgagaaacaa gcaatcacac a 401

<210> 204
<211> 401
<212> DNA
<213> homo sapiens

<220>
<221> variation
<222> 55
<223> /replace="g"

<220>
<221> variation
<222> 215
<223> /replace="t"

<220>
<221> variation
<222> 224
<223> /replace="g"

<220>
<221> variation
<222> 285
<223> /replace="t"

<400> 204
ggctatgata accaaaacaa catagtactg gtacaagaac agacacataa accaatgtaa 60

cagactagag aactctagaa acaagaccac ttgcctacaa ccatctgatc ttcaataaac 120

ctgacaaaaa caagcaatgg ggaaaggatt tcctatttaa taaatgttgt tgggagaact 180

ggctggccac atgaagacaa atgaaactgg actccttcct tacaccatgt acaaaagtta 240

acccaagatg gattaaagac ttaaatgtaa aacaccgaac tataaaaaaa aaaaaaaacc 300

ctaaaagaaa atctaggcac taccattcag gacacaggca caggcaagga tttcatgatg 360

aaaatgccaa aagcaattgc aacaaaagca aaaattgaca a 401

<210> 205
<211> 401
<212> DNA
<213> homo sapiens

<220>
<221> variation
<222> 167
<223> /replace="t"

<220>
<221> variation
<222> 190
<223> /replace="g"

<400> 205
atatgggtag cttcctagaa acatacaacc taccaagact gaatcattaa gaaatagaaa 60

atcttaacag acccataatg agtaagaaga ttgaattagt aattaaaaat ctcccaaaaa 120

gaaaatttcc agacattatg acctcatggt tgaattctac tgaacaagta aagagctaaa 180

atcaatccta taaactctta caaaaattga acaagaggga atacttccaa actcatttta 240

ctaggctatt attaccctga taccaaagca agacacaaac actataaaaa aagaaaacta 300

caggccaata accctgatga acatagataa aaaaatcctc aagaaaatga cacaaactaa 360

atttaacagc acaataacaa agatttatga accattatag a 401

<210> 206
<211> 401
<212> DNA
<213> homo sapiens

<220>
<221> variation
<222> 71
<223> /replace="c"

<220>
<221> variation
<222> 148
<223> /replace="g"

<220>
<221> variation
<222> 230
<223> /replace="c"

<220>
<221> variation
<222> 396
<223> /replace="t"

<400> 206
gcagacccca ctgagggtat aacacacacc aggtgacttc actaaaatcc ccacggtgaa 60

acacatttgc ggcccttttc cctgcccctg gattttgcta aggatctggg gggttctgtg 120

gaagtctgaa ggtcagtgga caggacgagt ttggagatgc gcatttggaa actgtacccc 180

tcaagtgaca aggaaatgag cgtggattta agaagataat gaccgttctg tgaagtttta 240

atggaatttt taagcaagaa aacccttacc ctgtgaaaac ttcagtctgc acagaagaat 300

tggtgggaga aggaagactt tgcctaagat cactttatat agcacaaccc agaccgggga 360

gaaggcagag ttatgcccag catatagcct tggcagcata t 401

<210> 207
<211> 401
<212> DNA
<213> homo sapiens

<220>
<221> variation
<222> 134
<223> /replace="t"

<220>
<221> variation
<222> 346
<223> /replace="t"

<400> 207
gaaagcagct ttctcccaaa agcagacaga ggatgggata atgacagtga agtgagaatt 60

gaagagttaa tagtgtccat gttatgctct ttaaagcaga cctgttctgt tgtgtatttt 120

ggcacatggt tttcagacat attttagtca atctttgcaa agttgaaagt tgattcagag 180

ccagatgttc agaaatagac agagaaggct attgtaattt ttcatccctc agtttttgta 240

cagaaacttc attgtaaaaa cccttctaga ctgatggctg cctcattgaa aactgggtca 300

aaaatatggc tctgttagaa aatctcttgg attacaaaac aatgcctaaa caaaaacaaa 360

tagaaatgga aaccgcccta cagatgtgag atttgtgcca a 401

<210> 208
<211> 401
<212> DNA
<213> homo sapiens

<220>
<221> variation
<222> 360
<223> /replace="g"

<220>
<221> variation
<222> 401
<223> /replace="g"

<400> 208
tatacgcata tctataaata tttctatatg gaagcagtgt ctataggaag ctacacatga 60

gttcatactg cgatcatcaa ctttaatcca ttaccacatg aatcattcta acctgctccc 120

cttgcctatc tctaaattct tactccaaca gaaaaaaaca ggctctcaac atctgccatc 180

tacttactta attattcaaa cctagtgcac atgtattgca gtactagaat tgttaaccca 240

tacccacatg ggaaaaagtt tatcaactag agtacagtgc ttatgtacaa tgttttttgc 300

aatctgaggc tccactgatt tctaaaatta cttaggtcag catctttttt cttcacccta 360

ttcagtgaag tgtttcatcc gtttgtaata cagttagatt a 401

<210> 209
<211> 401
<212> DNA
<213> homo sapiens

<400> 209
tgtatttggg actctgagtt acagcagcct ttttcctact ccaccctcta cttatttggt 60

cagttgtttc tcacaggctt tgtacagcga attcacccta gtaagtacag caggctgata 120

cttaatttta ctctgaatgc tggtagatga aaacaacagt gtcattgcct agtaaatgga 180

actaattctc agacttagtg ccaaggagcc agaaagggca gtgatttcat gacctagtta 240

aaggcacagt cattactcag ctcccactaa ttttggtcat gtaaaaacac aggcccaatg 300

atactagata ttatactttc tgcagataac cacggcaaca tttcccattc atatgctttt 360

ctagaacctt gcctctcacc attaagaaag gaagcgtgtg g 401

<210> 210
<211> 401
<212> DNA

<213>   homo sapiens

<400>   210
ctattattgt ccccatttta caaatgtgga cactgaggca cagaaaaaat aagtaatttt      60

cctggtgtca tgcagacagt aaatgtggaa gctagggagc ccggctgcca ctgcttctca     120

gcacagcacc gggctgcctt tgggataatt gtcatattcc ctttcttggc ctcaagggct     180

tccatatcca gtcttcccca cgaaccccca acagcttcaa cacatgtcaa ccacccccag     240

tttaaagcaa gttgcccaga ggaatttttt gcgacttttg agtcttttca tttaagaata     300

tacttttatg ctcaaacata aaatctggta ataaggttat tattatatct atatctcagc     360

ttcatccaat ttgctgtcgt ctctctttat tatatacttg a                         401


<210>   211
<211>   401
<212>   DNA
<213>   homo sapiens

<400>   211
tcttgtttcc agagataaat tagtaggaag gggtgaaaag gagaaatatt tgtgaatcat      60

acagtagtca cagctattgg ttgatgtcta ggatattaaa atgtgaaagt attacgacta     120

aaaatcctct gctgtctaat ctctcacagc ttatatttga tgttccctaa aatcacggtg     180

tccaaagaca gaccatgttt ctctagaatt cctgtaatga agatctttgc aagatgccgc     240

aattttgaat gtcccaaacc acaaagaggt ttcagttctt cctttcaatc cccaaccttg     300

aactgaagcc tctgcagaaa gctcccagct cactagtgtg gacaggcaac tacttgtgct     360

tctatgagag agaaaaagtg agcagtttcc aagagacaga a                         401


<210>   212
<211>   401
<212>   DNA
<213>   homo sapiens

<220>
<221>   variation
<222>   248
<223>   /replace="t"

<400>   212
ctaacatgta ttatttcatt tttctaataa gaaaaaataa acaatgtgcc aatgtaaata      60

caaatcacaa aatagttcac ctttctggct gcctgctagc agatttttgt tttattttac     120

tgcatgtgtg gacaagccat ttcatgtact tttatctctg gtttaagtta tacagaagcc     180

attctgtttc cctttgtaag agtttcattt aaatgtccag gtgtatgtgg gtgtggtgga     240

attaggagga agagcgcttc gcccccatca attctgcagt tgccgtggca gcttagccgt     300

gagtgggtca ggtggccaaa gcagaggccg acccccgcca gccgttgctg tcaggccccc     360

tctcgctctc tggttcagga gaacgtctct agaagagcgt c                         401


<210>   213
<211>   401
<212>   DNA

<213>  homo sapiens

<220>
<221>  variation
<222>  11
<223>  /replace="c"

<220>
<221>  variation
<222>  26
<223>  /replace="g"

<220>
<221>  variation
<222>  51
<223>  /replace="t"

<400>  213
tttgcttcat aacaactgtc ttggaataat ttttatttac ctaaaaataa ggtaagagta      60

tgtcaaagtt taggcatagt gtttattaaa tgcatagttt gtatcaatgc ttgtaagctg     120

taattaggaa tgatatgtgg cattatattt tacttttgtt aatgttcact gttaacttcc     180

tgaaatgtaa gctgtgctca cgtgaaaaaa aaaaatggat cagtttttct gagaagacac     240

tgtaacacaa aaagacctac aggatagtat ttgaatagtt aattttaatg caaacaaaaa     300

cttctatttc agcattaaga acacatattc ttgatataag aaatagaaaa tattctagca     360

catacctgac actggtaaaa tatatgtatt ctttttccct a                         401


<210>  214
<211>  401
<212>  DNA
<213>  homo sapiens

<400>  214
ctctcaggtt tgaaactgtg tgtgtgactg tgggagtaaa tagtctagtt tcccactctt      60

catgtgtaaa tagacacaaa caggtgactt ttcagagcct gggacagaga agacgacccc     120

ccagtggaga cgactgaaca ggcccctcat gcatacagca tgccaccagt attgcccaga     180

aatactgttt gtagaaaatc ggaaaggtag ccgggcccgg cattaatgct agggtgtctg     240

agatgagtcg aaggaacagc tattcttttt ccctttgtta tgtttcttcc atttccaagt     300

ggatgaagct ctaactcaac ctttcatttg tttcaagagg gaggtatcag aaccacaaca     360

aatggaatga aacctaccac taccacctgc ctggcacaaa g                         401


<210>  215
<211>  401
<212>  DNA
<213>  homo sapiens

<220>
<221>  variation
<222>  388
<223>  /replace="g"

<400>  215
gaaataaaaa agaaaaagat ctttcttttc tcctggcctt taacttggca ggctttatct      60

gctcttcaga gtcactgaga tgagaaccag gtggcaaact ctgctttcct tctcctccag     120

gcaacgcaga ggcattctta actcttaacc tgcagacttt gaaactaggc acagaactcc 180

agaggggata ataacaccag caaatgttta gctaaaaaat ttagccatac atgccacaca 240

ttgtgctaca cattgagctc tttggtcctc acatcaacgc tgaattagga gctatgatta 300

tccccattct ttagattagt aagctgggta tcaccaagga gaagtacttg ccccaaagtc 360

acatggctca tatgtgttgg aagcaagatt tgtgacagat c 401

<210> 216
<211> 401
<212> DNA
<213> homo sapiens

<400> 216
tttttatttg tgaaacaatc ctaggtcttt gtgttccttt tacctgattc ccaaactgaa 60

caaacccaag taccaaccat tccccttgga tttttagtca actgaaggtt cagagagctg 120

ctcccttggg gaagaggctg tggacatgac atttgcacat gcagcatatc aacttggaat 180

aatttccccc tcatgtgggg gctactccca aggagtttat tctaaaccca aagtacatta 240

gttgcctgtg gcaccttaga aaaataaact atcctggggt ccctggtttt ggaatgtcat 300

ttcctttatg atatgtgtct ttgaaaggca aggagtggca gtaagcagag tgatataaac 360

atggggcctg ggctttaatt ctgtctgccc tcatccttta t 401

<210> 217
<211> 401
<212> DNA
<213> homo sapiens

<220>
<221> variation
<222> 138
<223> /replace="g"

<220>
<221> variation
<222> 335
<223> /replace="t"

<400> 217
ctctttcctt ctttctttct ggcttgcttt ccctcctttg ctgctggtga ccacccatgc 60

ttgagcagtg ttttggctgc tttcccactg atgagttttc caaaccttgt caggagctgg 120

cagttaaatg ttctgccacc agcttacaga aaatacaagt tggattgcct tgttcctgct 180

ttctttcaaa cttgcatgtt aatccatctt caccttgatt cctagaagag tcaaacatct 240

gatcctcagg tcaagcctga aggaaatggt tgtcccagct gaaaataaga tcgatcccaa 300

tttgctttac agtactcttg ttaaagatca gtgtgtaata ttttctctgt gtgatgcaat 360

gcttcttaat aaaaacaaaa agggatgttt gaccttaact t 401

<210> 218
<211> 401
<212> DNA
<213> homo sapiens

<220>
<221> variation
<222> 226
<223> /replace="g"

<220>
<221> variation
<222> 294
<223> /replace="t"

<220>
<221> variation
<222> 319
<223> /replace="t"

<400> 218
tccatgtcaa cagcaggcgg cttatattcc agaaagttaa gtctgtgcca ttctctgcct 60

gagccagtat gttacgaaga gcattaccat ggcgatttca cattctgaaa ctgtgcttgt 120

ctcttaaatg ccacgtgcca atgagtcata gaggtgtttt gttctagagc catgtacccc 180

ccaaaaaggg agtttgtaca gatttaccaa gaaactatta ttattatcta cagttcatgc 240

agttacaaaa ctatctttgc ctccttttac tcacaatttg aacccaatct tttctaaggt 300

gctggcctcc caatattgct gtagataatt cagccttggt ttcttatgat taaagattat 360

gactcacacc agcctacaag cctggagaat tatactgaag t 401

<210> 219
<211> 401
<212> DNA
<213> homo sapiens

<220>
<221> variation
<222> 229
<223> /replace="t"

<400> 219
agaattgctg gatgctttta agtcatcctc caacatgaac cagcttctta taaaagatga 60

gacagttgca tttatatatc caaagctagc tgctatattt tctaagtcat gtctcaagta 120

gagcatctag aaaaaacctg tatcagtagt gcttaagtct ggcactgcag ggaagctggt 180

ttaaaattac agagcctctg gaattttcaa atgttcaaaa catccaacct agagcctggg 240

aatctgtatt tttaatgagc atcgctcatg cattgctatg gaacacacat tgcatatata 300

gcattttcta acatatagga tcaggccaga ggaagaatgt ctgtgtatga gaactcccta 360

aatgatgcac agttccactg tctggttaca gttgaaaccg t 401

<210> 220
<211> 401
<212> DNA
<213> homo sapiens

<400> 220
tgaggcccca ttggtttaaa tatctcattt cttacttgtc aaagtgcatg aatgtccagc 60

attagaaatg cagttccttg gggtctggag ggcattgagg gctcccagcc ctgcttctta 120

agtgcatctg ctgctgcata gtatcaggct taagtcttag catccacacc ttgtcagcaa 180

ttaggtgaca gctaactgtg gtcaaggctt tctggggact gaatgaagaa ctggggtggc 240

acaggtagtg aaggagggtg agaggctggt ggggccatga cagggggtgt tgggggttgg 300

cagagcccaa acttccctgc tttccctgga actggctgca ccaagaagtt gaggctcctg 360

gtttgtttgg aatcaggtat ggtacaatcc attgtcccgt g 401

<210> 221
<211> 401
<212> DNA
<213> homo sapiens

<220>
<221> variation
<222> 267
<223> /replace="t"

<220>
<221> variation
<222> 272
<223> /replace="t"

<220>
<221> variation
<222> 291
<223> /replace="t"

<220>
<221> variation
<222> 341
<223> /replace="t"

<400> 221
gcctcttctg gacgtgagac aatttgagtg cagtatttct ctagttgctg acccttccca 60

gctcctcctt tcaggggatg tggtgtcatc ctggcattgt cctgtcctaa cctagccctg 120

ctggtctcca tggctgcagc tggatatcca aacacccaac acacataatc atggctctgt 180

gccgtgagga ggagacaaga accccccaaa ccattctgtc gagtttccta tggttgggtt 240

ctggttgtat ttctcttctt ttctttcctt tccttttctt tttttttttt ctttgagacg 300

gagttttgct ctttgttgcc caggctggag tgcagtggcg cgatctctgc tcattgctac 360

aacctctgcc tctcggattc aagtgattct cttgcctcag c 401

<210> 222
<211> 401
<212> DNA
<213> homo sapiens

<220>
<221> variation
<222> 53
<223> /replace="g"

<220>
<221> variation
<222> 137
<223> /replace="t"

<400> 222

cacatgtgag agaacagctt tccagaaacc acacttgagt ccacagcaga gcagaagggg 60

caggaggcat caggaccatt tccagatttc tctctggccc tgattgtcta gatttgcatc 120

caatcactgc aagagtgtat acaaaagttg ttagaaagat ttgggtttca cacagagtta 180

tgaaaacaag gtagaagcct gatgtcttca tttcccatga aatcttgtgg gtgtaaatga 240

aacatgtttt agtctcaaat cctggagctg tggggttcac aatggagaaa tatccaggtc 300

tcttgaacaa gactggacga taaccaggga cagttccctt tttcccccac attcatttat 360

gagttggtca aactgttgtc tattcacagg agattcttag a 401

<210> 223
<211> 401
<212> DNA
<213> homo sapiens

<220>
<221> variation
<222> 329
<223> /replace="t"

<400> 223
actcttcttg agcagtgaac atcaagagat catggattaa atgggaaaat tagtgtcaag 60

attacatcag aagaaaggca agtatcgcat catttcactg cccacaaaaa tacattcttt 120

ccctttccct ccactatttt aagacttcat tgaccttttc taaggtccca atttccattt 180

aacctctgtg ccagtcaaga gagcagtccc aagtctctaa ctttcttcac ccaatgatca 240

ctctttgaac tcaccaagca tgctacaaac agaaggaagc cattagcagg gggagttcga 300

acctatatag ccaaccataa attagaagcc caaggaagca ttttcaacca ctacgaggtc 360

actcaggctg actgctggca gtggcttctc atcgatgaca g 401

<210> 224
<211> 401
<212> DNA
<213> homo sapiens

<400> 224
gaattctgga atcagagagg agaaaccact tacctcaggg gctgaaggag cctttgggct 60

tcctggctag agtgctgagg aacttggtgc tctcaggctg ctggtgggag gagagacctg 120

ggtttgagga ggtgactggc caggcttatg gacggggatc cacatgatga atttctccag 180

cccctgtgca ctcaaactgc ggtaaaatca gttagtgggt ttgcgctttg agcagctcta 240

gtaattcatt tggtgtgtct ttatagttta caaggcactc ttgcttctga gtttcccttg 300

gaggctgagg ccactcccag aagcccagta aagagcagtg gccaagtgca caaactgggc 360

ctgggctcag atcccagctc tgtcgctttc taactgtgtg a 401

<210> 225
<211> 401
<212> DNA
<213> homo sapiens

<220>

<221>  variation
<222>  292
<223>  /replace="t"

<220>
<221>  variation
<222>  341
<223>  /replace="c"

<400>  225
cattgacctc ccagatctgc ctgtgatttg gggcttatag agatgtgttg attctgtcta      60

gtcaaagtga gagtcattgc agaacaaaac cagaatctta gtggtgactg tgcaaagcca     120

tttggtctaa tgattttacc aagagcatct cagaacacta gtattggtag tatgtagcct     180

tgttaataaa tatatacaag tacatgttga aatgacttgt tattatatgg ctagagcaaa     240

cccacgtcat cctcatgatg tcttgctgtc acaatctcat tctctcttgg ggtctgacct     300

gactcctcaa gcaagagaac aagggttggc agagctctcc aggaatgcac agccccaggt     360

gcccagcaga gggcatcaag tatgacagat attggaatga c                         401


<210>  226
<211>  401
<212>  DNA
<213>  homo sapiens

<220>
<221>  variation
<222>  61
<223>  /replace="t"

<220>
<221>  variation
<222>  152
<223>  /replace="t"

<220>
<221>  variation
<222>  278
<223>  /replace="t"

<400>  226
aagagcatct cagaacacta gtattggtag tatgtagcct tgttaataaa tatatacaag      60

cacatgttga aatgacttgt tattatatgg ctagagcaaa cccacgtcat cctcatgatg     120

tcttgctgtc acaatctcat tctctcttgg ggtctgacct gactcctcaa gcaagagaac     180

aagggttggc agagctctcc aggaatgcac agccccaggt gcccagcaga gggcatcaag     240

tatgacagat attggaatga catctgttgt gtgtgcagag cagactgact gagtggaacc     300

atgaccaaag agaactcagg tagactcaag tgcttctggg aaatttgaga aacttccctg     360

acagagaatt tacttcaagt cagatacctc cccaataatt g                         401


<210>  227
<211>  401
<212>  DNA
<213>  homo sapiens

<220>
<221>  variation

<222>  268
<223>  /replace="c"

<220>
<221>  variation
<222>  328
<223>  /replace="t"

<400>  227
aaatttagta aagcaatatt ttattgttct agcaacaaaa aaattaagtt attacattaa      60

ttagaaagtg atctgagaac cttattttga tataggaggg gagcagggaa gtgctgggaa     120

gggaagggca tggtccctgg ctaggaatcc atccctgggt ttgtgcccat ggacctaggt     180

gaggacaggg atttctgttt ttgtgtccaa atgttgcatt tcccaagacc gctctggcca     240

accatgcccc catcctgtgc ctgtaaaaac cctgagacat tagcaggcag agacacaagt     300

agctggacgt cgagaggaat gtaccggcag aggaggacat caacagacgc ccgcagacac     360

caggccagta ggccgtcgac aaatggaact atgtggtgat g                         401

<210>  228
<211>  401
<212>  DNA
<213>  homo sapiens

<220>
<221>  variation
<222>  134
<223>  /replace="t"

<220>
<221>  variation
<222>  261
<223>  /replace="t"

<400>  228
gtgatctgag aaccttattt tgatatagga ggggagcagg gaagtgctgg gaagggaagg      60

gcatggtccc tggctaggaa tccatccctg ggtttgtgcc catggaccta ggtgaggaca     120

gggatttctg tttctgtgtc caaatgttgc atttcccaag accgctctgg ccaaccatgc     180

ccccatcctg tgcctgtaaa aaccctgaga cattagcagg cagagacaca agtagctgga     240

cgtcgagagg aatgtaccgg cagaggagga catcaacaga cgcccgcaga caccaggcca     300

gtaggccgtc gacaaatgga actatgtggt gatggattgg caagaacctt gaatcctgga     360

catgaccatt ttacaatgtt attgttaaat ttttttcttg a                          401

<210>  229
<211>  401
<212>  DNA
<213>  homo sapiens

<220>
<221>  variation
<222>  74
<223>  /replace="t"

<220>
<221>  variation
<222>  141

<223>  /replace="c"

<400>  229
gcatggtccc tggctaggaa tccatccctg ggtttgtgcc catggaccta ggtgaggaca      60

gggatttctg tttctgtgtc caaatgttgc atttcccaag accgctctgg ccaaccatgc     120

ccccatcctg tgcctgtaaa aaccctgaga cattagcagg cagagacaca agtagctgga     180

cgtcgagagg aatgtaccgg tagaggagga catcaacaga cgcccgcaga caccaggcca     240

gtaggccgtc gacaaatgga actatgtggt gatggattgg caagaacctt gaatcctgga     300

catgaccatt ttacaatgtt attgttaaat ttttttcttg agcaaggtat gtataagtaa     360

gtaaataagg gcataatgtc aggtaaactg aatggctgga a                         401


<210>  230
<211>  401
<212>  DNA
<213>  homo sapiens

<220>
<221>  variation
<222>  175
<223>  /replace="t"

<400>  230
ttagcagtgc ctcccaccac agcagacaga aggtacatct cactctaccc tctgacatca      60

gctccctcat ggaacttcct ttggccaatg agccgagagc agaggcgtga gttgtgtcta     120

gccagagggt tcaaatgtgc ttgcgtgatt tggcttggct ttttgagctt cagcattctc     180

catgggaaaa tcatgccctg agtaggcatt gctccttcaa cctgtgtcct ggaatgaaga     240

cacatggaac agtctggaac ctaaaacaaa gcctggagtt cagctgagat cagctaaggt     300

gtggctggga cctgtgcata tggaataaat gtttgctctt ataggtaaat gcgatgtgga     360

agttgttcat tactcaacat tattgcagca gaagcctggt g                         401


<210>  231
<211>  401
<212>  DNA
<213>  homo sapiens

<220>
<221>  variation
<222>  127
<223>  /replace="g"

<220>
<221>  variation
<222>  324
<223>  /replace="t"

<400>  231
tacatgtacg tgtgcttgta tgcatgggtg tgcattatgt gcgtgtttgc ctgtgtgtgc      60

atgcatgcgt gtgtgtgtgt gtgtgttctc tgtggaggga ggagactgga gattctcttc     120

cccaacattc gaggccagtt tgccaaaaat cttggtgcta actgtatgtc caggttacag     180

atccacacgc aaatgcagac tcaacaacac tgaggctcat cttttgggtt tctccccatc     240

ttcttcacca tgactcagtt ctccaagaaa aatgttgttg ctactttcag gaacagtcca 300

ggatgagagc ttaaggtaga gtccggacat gtgcatggaa gcacagccat gattctatct 360

aattggctca aacaaaagtc ttggacagta ttttggcttt t 401

<210> 232
<211> 401
<212> DNA
<213> homo sapiens

<400> 232
ggaatctgct ttactcaaag cccatcgagt ccaatgttaa tctcatccaa acacaccctc 60

acagcaacat ctagactggt gtttgaccaa acagctgttg ggtaccatag cctagccaag 120

ttgacacata aacttaatca tcatgggatc agtcccacca tgccccatgc ccatctttgg 180

accagtgttc tagcctcctg actgttcagg tgagttatgg gagaaaactt aggaccccat 240

gcaaatgcag actttctttt acattgtaaa taccactgac attcttgctc atccaactgg 300

gacatttttg ccattcagtt tgtcaagcaa atataacgat gcatattttc agcacacact 360

tccactgttt agcagatggc acatctgaaa catggtacat g 401

<210> 233
<211> 401
<212> DNA
<213> homo sapiens

<400> 233
gggggcgcga gagagcagca agccgtaaca gctcgcgtaa aaggcgggag ctggggcttc 60

ctgggggttc cctagaagcg gacctcgaga caaggtcctg ggtgcaagcg gtttatttgg 120

gggatggctg caggaggcac agggtaggag cagggctggc agggaaggga gagaagggag 180

gaagggcagt gaagagcaca aggatggagg ggctactccc gggcctgtgt gcagagcctc 240

agccctggct ctgtcaggca gaaggagctc ccgacacctc tgtcatctgc tggctgcgga 300

gaacctgggg ctgacgccgt cctccgggac ctccatccag aaaagggcca cagaggctga 360

gggcgtcggg gcgggcactc cagggtctta ttctgtttgg g 401

<210> 234
<211> 401
<212> DNA
<213> homo sapiens

<220>
<221> variation
<222> 80
<223> /replace="g"

<220>
<221> variation
<222> 81
<223> /replace="t"

<220>
<221> variation
<222> 227
<223> /replace="t"

<400> 234
ggagagcaga gtgacggcct agaaaggagg ttcccgaggg ctctggggag gcggaagagg 60

ggacgcggtg tgttgagata cggggagaag ttcagagcag catgggcatg caggtgcctg 120

cagaggggcc catctgggct gcaccccagc aggacagggc catggcttgg tgtgggttcg 180

gctggctgca cagcttcttg taaagtttgt ccccagggtc cttgggagta gaggtctttc 240

ggaaggcacc atctccagag cgtgtttctg agtaactctg aggatgggtt ccgggttatc 300

actgtgtgtt ggaaacagca gcgcccaggc tgccggagga ggctggtctc cctggcaggt 360

cctggctgtg tctagagttt tgtgggtcct ggagataagg c 401


<210> 235
<211> 401
<212> DNA
<213> homo sapiens

<220>
<221> variation
<222> 372
<223> /replace="g"

<400> 235
agaggcagag gattgtagtt gtgaaaagtt tgtacattgg agtctggctt tgagtcccag 60

ttttcccgtg gacaggtcat gtgaccttgg caaaataacc acctcccatg cctcaggtgc 120

ctcatctgtg aaatggggat aataataaca cctacttcaa ataaaacaat gcaaggaatg 180

ccctagaaca aacgcctggc acatagtaag ttctacccaa gtatttatta ttataataat 240

tgtataaaca tttaacgact tccttaaatt gtttcctatt acttgctatt ttgaatagtg 300

tttcaattaa tatccttgtg atatggtttt gctgtgtccc caaccaaatc ttatcttgag 360

ctgtaattcc cacagttccc atgtatcgtg ggagaaacct a 401


<210> 236
<211> 401
<212> DNA
<213> homo sapiens

<220>
<221> variation
<222> 168
<223> /replace="t"

<220>
<221> variation
<222> 277
<223> /replace="c"

<220>
<221> variation
<222> 359
<223> /replace="g"

<400> 236
gatgtgtgtg tgcatatatg tgtgtgtgta tatatatata cacatacata tacagaaaga 60

gagagagaga gtctcactct gtcttccagg ctgaagtgca gtggcatgat cttggctcac 120

tgcaacttct gcctcctggg ttcaagcgac ttttgtgcct cagcctcctg agtagctggg 180

actataggtg caagttacca ggccctgcta atatttttgt atttttaata gagacagggt 240

ttcaccatgt tggccaggct ggtctcgaat tcctgaactc aagtaatcca cccacctcag 300

cctccaaaag tcctgggatt acaggcgtga cccactgtgc ccagctccca aaatgtataa 360

ctaagtgaga agggaaggat gtagaatagg gtgggtgttt t 401

<210> 237
<211> 401
<212> DNA
<213> homo sapiens

<220>
<221> variation
<222> 215
<223> /replace="t"

<220>
<221> variation
<222> 360
<223> /replace="g"

<400> 237
ctcaggtgat cctcccacct cagcctcctg gctagctggg actatagaca tgtgccacta 60

tgcttagcta atttttgta tttttttgtt gagacaaggt tttaccatgt tgcccaggct 120

gttcttgagc tgggctcaag tcatccacct ggcttggcct cccaaagtgt ggggattaca 180

gcatgagttt ctgggcctgg tctctttgct acaccttgtc agggtctttt gcaggaattc 240

cttgtggtcg ttggcaggtg tcatgcctta tcactggtag gcaagccttg gaaagctcct 300

gtgaagattg tcctattagt atcagtttac ttttttgaaa aaatattttt attgatttta 360

gagacagaat cttgctttgt cacccaaatt ggagtgcagt g 401

<210> 238
<211> 401
<212> DNA
<213> homo sapiens

<220>
<221> variation
<222> 296
<223> /replace="c"

<400> 238
aattctactt ataatgttct tggcctggaa gtcaaggcct cacatatctt acttcaatcc 60

actatttctg gcaggacacc tcaccacact tcccttgctc ctgtccacat gagctcttag 120

gccaggggca tgcttctctg tttgaccaca tgcacctcct ctcctgttat ttctgccatt 180

tgagatgctc cccttcctac tgctgcatta actgaaagcc acacttcatg atccagaaga 240

gatgccacct cctctgccaa gctggccctg actttccagg cagaaatgat gtattaccat 300

atggcattca ttgctttaca gtgaattaaa gctattattt ttagttgtaa aaatacattt 360

tttaaaacta caagtgtaaa atacatttgt gttgcaaaag t 401

<210>  239
<211>  401
<212>  DNA
<213>  homo sapiens

<220>
<221>  variation
<222>  106
<223>  /replace="t"

<400>  239
tgctcctgtc cacatgagct cttaggccag gggcatgctt ctctgtttga ccacatgcac      60

ctcctctcct gttatttctg ccatttgaga tgctcccctt cctaccgctg cattaactga     120

aagccacact tcatgatcca gaagagatgc cacctcctct gccaagctgg ccctgacttt     180

ccaggcagaa atgatgtatt accatatggc attcattgct ttacagtgaa ttaaagctat     240

tatttttagt tgtaaaaata cattttttaa aactacaagt gtaaaataca tttgtgttgc     300

aaaagtccag ataatacagg aaaataaaag cttcccgttc cctcagctct cttctaattt     360

taattcttgg agctattact aagagtgggg gccgggcgtg g                         401

<210>  240
<211>  401
<212>  DNA
<213>  homo sapiens

<220>
<221>  variation
<222>  137
<223>  /replace="g"

<220>
<221>  variation
<222>  334
<223>  /replace="t"

<400>  240
taagcttcag aagaatctta gtagaaatgc attgtttgga atgcatatgc tttggtctca      60

ataaaaatat ccggttgcag agatatttca tgtggtttga actaacaggc aaaaaattaa     120

aaattcattg gtatacacta attcatgcaa ttgtatattt tggaggtaag tgtaatggca     180

taggtttgct gtaatggaac tgattggaaa atgatctctt ggagaaaatt tattgctggt     240

cataagattt ctttggttta gaaaaatgct gggtttagta aaatctgagg cactaaatga     300

aagtctgctt taataaaaat gtggatctga gaactgtggt ttttagtaag tgggcatttg     360

atccattaaa attgtattgg ttttattctt tacgttggct t                         401

<210>  241
<211>  401
<212>  DNA
<213>  homo sapiens

<220>
<221>  variation
<222>  262
<223>  /replace="g"

<220>

<221>  variation
<222>  279
<223>  /replace="g"

<220>
<221>  variation
<222>  355
<223>  /replace="g"

<400>  241
agggcaggca agatggatga ggcaaataac tcaatgagca agtgacggca ggcccttcag      60

gccgccttct catgcaattg atagttgctc cttcctcaca tttggtttgg ttccctaaat     120

gttaaatcat ctctaggata agcaggtgat agcccatgaa tggagccaga tgctgagatg     180

ttgttcagct tgagtggcct gcagttcatt cctgaccaag ctgtggtttg ttcctgctgg     240

ctgttggatc acctccttca aagattcatc actggataag ccactgttgg ccattggagc     300

tattccacca agactgatct gaactctccc tatgagactt ttctttttcc agtaagctcc     360

atatctgatt ctatgaaaag ttcagcccaa atgagaggac c                         401

<210>  242
<211>  401
<212>  DNA
<213>  homo sapiens

<220>
<221>  variation
<222>  198
<223>  /replace="c"

<400>  242
agacatgcaa aatataaaca cacatttcat tgttattaga ttccacaagc ataaaattat      60

tctgtcagag tgactatttg gttctttctc atctctatag ttatctcttc gcagacaggt     120

aacagcctgt ggaggaccac actgggtatc atttgtccgg caggtgctgc gatcctaaga     180

gggtaacatt ctccccagcc actggaagtg ggtggaatgg agccactata gtaaatgttg     240

ttggatgcct acttagcagc ctgcctcctg ccccctgcct ccttcctgag agactccaat     300

ttactcagct gtgcactgga cacaccaccc tgattgtggc tggcctcacc tgtaggttgt     360

ggcaaacttg actggcctaa gggtaatccc atatgcctta c                         401

<210>  243
<211>  401
<212>  DNA
<213>  homo sapiens

<220>
<221>  variation
<222>  13
<223>  /replace="c"

<220>
<221>  variation
<222>  108
<223>  /replace="t"

<400>  243
atgggaaaaa agagggtctt gataacattc taattagccc caaaccctct attcccatga      60

gccagtctct tctctttaag ttgatgctag ctgccgtttt gtgttatctg ttacagacta     120

atacaatttg caataattga agatgcaata tttattgata tattattatc aattgaagtc     180

catagcttac attaggattc actcttagtt ttgtatgttc tgtggatttt gacatatgta     240

aaatgacttg tgcccaccat tacagtatca catagaatgg tttcactgcc ccaaaatacc     300

ctctgttcca tctatttatc cctcttcctt tccatggacc cttgacaacc actgatcttt     360

ttattgtttc catagttttg ccttttccag aatgtcatgt a                         401

<210>  244
<211>  401
<212>  DNA
<213>  homo sapiens

<400>  244
agtttcctat gaaggcagtc gctaaagaac attccacttt gcataaaaga cttcatttca      60

aggctttcag tcaaatgcat ggctagctga tggatcccac tgggcttaac aagcacaatt     120

aaggtcttca tcaaacctta atagattggt gtcattgagc actgaccagt gcttggaatg     180

tttttcttct ctgcctatca tccatgtctg aaattttcct caaatattgg caaagagaac     240

atataagggt ggattagtag ttctccttta ttcatatatt gtgatatttt aaaggcatct     300

ttcagtaaat gcgttatcat attagcatct aactgactga tccaatgggt tatatcaaaa     360

tgcaaggtgc gtacacatgg gactcatatc tcaaattcat t                         401

<210>  245
<211>  401
<212>  DNA
<213>  homo sapiens

<220>
<221>  variation
<222>  313
<223>  /replace="c"

<400>  245
gcctgtttct aggtccagaa tggtattgcc taggttgcct tctaggagtt ttatagattt      60

gggttttaca tttaagtctg taatccatac tgagttaatt tttatatata gcataaggaa     120

agtgtcctgt ttcaatcttc tgcatatggc tagccagtta tcctagcaca atttattgaa     180

tagggaatcc attcctcatt gcttgttttt tgtcaggttt ttcaaggatc agatagttgt     240

atgtgtgcag ccttatttct gggctctcta ttctgttcca tcagtctatc tgtctgtttc     300

tgtatcaata ccatgctgtt ttggttacta tagccctata gcatacttca acgttgagta     360

gtatgatgcc tccagctttg ttcttttgct taggattgtc t                         401

<210>  246
<211>  401
<212>  DNA
<213>  homo sapiens

<220>
<221>  variation

<222>  310
<223>  /replace="g"

<400>  246
tttaaaatag ttttttctag ttttgtggag aaatatcaat aatggtagtt tgataggaat      60

agcattgaat ctataaattg ctttgggcag tatggccatt ataaagatat tgcttgtcat     120

tatccatgag catggaatgt ttttccattt gtttgtgtca tctttgtttt gtagttctcc     180

ttgtagaggt ctttaacttt ttcggttagc tgtattccta agtatttttt tttcttgtgg     240

caattgtgaa tgggattgtg ttcctggctt ggctcttggc ttgactgttg ttggtatata     300

gggatgttaa tgatttttgt acattgattt tgtatcctaa gacttttctg aagttttgct     360

gatgttgttt atcagcttaa ggaaattttg ggcttagact a                         401

<210>  247
<211>  401
<212>  DNA
<213>  homo sapiens

<220>
<221>  variation
<222>  150
<223>  /replace="t"

<220>
<221>  variation
<222>  318
<223>  /replace="t"

<220>
<221>  variation
<222>  343
<223>  /replace="t"

<400>  247
atctgctgtc ctctgggcct agtttcactc ccatggcagt gttagcatga gggctggatg      60

ctggtagggg cagggatggc tagttctgtg tctgtccagg ctccatctgt aatggtgata     120

agaagggaga gggtgggtgg actgcactcc ttgggcaatg gtgggaccag gaaagcaaaa     180

ctcacctgtg cagacacatg tcagcaaagc aatgtgggga gttgttgtgg gcctggggga     240

agcagtgctt tggggaggga gtgggagggc tggtgcatgg caactgcctc actggagttc     300

tctgctgctc aggcatggtc cactgttgca gaagctatgg tgcggtccca cagggcagcc     360

aagacagccc tgcaagcagg catggccagg ctggggtcct g                         401

<210>  248
<211>  401
<212>  DNA
<213>  homo sapiens

<220>
<221>  variation
<222>  31
<223>  /replace="g"

<220>
<221>  variation
<222>  112

<223>  /replace="g"

<400>  248
ctggcatcct ggagctcaga gcagaggaga agaggtcaga cagtggatct agaggggcag      60

acaaaaatat ccagaacaag tttcactctc catgggcctg aactagtggt tacaattgct     120

ggaaggtaga aagtgttagg gggtgttcag gtatgggatc cagatgaagt gggagagaat     180

tttagtatgc tttgtgaaga tttcccatga tttgaaattg tgtaaggact gtcccaaatt     240

ttgcaaatga tgctatatca taagataaag aagacagtca tactcatttg gtagctatat     300

ttatataatg tattcatata aatgtataaa tatgtatatt atgtaagtgg ctgatgttta     360

catttgctta atcatatgag aatatagcaa aattataact g                         401

<210>  249
<211>  401
<212>  DNA
<213>  homo sapiens

<220>
<221>  variation
<222>  234
<223>  /replace="t"

<400>  249
tatttcactt agcatagtgt ccttcaggtt catccatgtt gttgcaaatg gcaggatttc      60

tttcatttga aggctaacat tccattggat gtatatgcca cattttcttt atccattcat     120

ctgttgatag gcatttagat tatttccata tcttgccttt tgtgaacaat gctgcaatga     180

ccgtggaagt actggtattc ttttgagatc tgatcccaat ttcatttcca ctggatatat     240

acccagaagt gagattcctg gattgcaagg tagttctagt tctataatgg ctacaaccaa     300

tttacattcc caccaacact gtgtggtttc cttttctcca catcatcacc agaatgtttt     360

gttttatttt atttttaaaa taatagccaa accatttgga t                         401

<210>  250
<211>  401
<212>  DNA
<213>  homo sapiens

<220>
<221>  variation
<222>  274
<223>  /replace="g"

<400>  250
tttctgagaa gtgcacgtta aacagcatag catttcagcc ttgaagtgag aagtgtagac      60

tatttgtgat aatgaatata caactgaggt gttccactgg agcaagtcac ttagaatcac     120

agaaagcgta ttatcaggcc catgatgtaa atttaaaatg tcttattcct cattttacaa     180

acttatgtgg gcatctttcc ttctcacagg aagcccattt cagtgtggaa aatcagtctt     240

gtatgaacca gatttagaac agtgccatga ttcattcata ttcaagagtt aactgcttct     300

acacagagtt ggtgtcagaa ggcatgttgc tgagtgccag caactccttg ggggttgagc     360

ttagatggtg tcctctgttc tagcatgtga atgctgtaaa c                         401

<210> 251
<211> 401
<212> DNA
<213> homo sapiens

<220>
<221> variation
<222> 82
<223> /replace="t"

<220>
<221> variation
<222> 388
<223> /replace="t"

<400> 251
tgtcccactc agaggggcgg ttctgaggca tgctccctac agtctcccag agtggcccca 60

ggagggctaa gcctcagttg cccatgatgg caatgctcat tattgcaccc tcgtggcttc 120

cttctctttc ctgcctcatt tcgtactccc aagctgtgct cccctgcatc acttcctaaa 180

ctaaatccct tcatctaaga acttatccca gggtctgctt tggggaagaa caaaagacaa 240

agcacttatg atatactcaa gactaacaga agcataagct tctgaggatt taaatgcaaa 300

tagaaacaat gcaatagaaa tagactaaaa ggcctgtgct ctccttaacc cacccagcct 360

atttcagact gatgggcttg gggtcagttt ctgccagagt g 401

<210> 252
<211> 401
<212> DNA
<213> homo sapiens

<220>
<221> variation
<222> 94
<223> /replace="g"

<400> 252
ttttactttt tggaaagcca agcaggtccc agagtcgtgg ggcagtactt ggcctcatct 60

ctgaggccac ccattctgct gctggcagcc acaatgatgt acccttggca ctatgtcgct 120

ccccctactt ggcatcctca gtttttctaa actcatgggt gagagacgaa tgacgtcaga 180

ggcccaaacc tttggatgct gatctaaaag catcaaccaa aacccaacac ccgagcttcg 240

taagttccca tttgaagcag gagcagtttt tatagcaaca gacaaaggat tgctctgtcc 300

ttgttggaag tgcttgagga ccaagcacag aggtgggcaa agatcgtcat cagggcttct 360

gctacctgtc ttagccccag cattcttcaa aagagatcct c 401

<210> 253
<211> 401
<212> DNA
<213> homo sapiens

<220>
<221> variation
<222> 69
<223> /replace="t"

<220>
<221> variation
<222> 242
<223> /replace="t"

<220>
<221> variation
<222> 310
<223> /replace="c"
/replace="g"
/replace="t"

<220>
<221> variation
<222> 334
<223> /replace="c"
/replace="g"
/replace="t"

<400> 253
taaatttgaa gtgttatggt gagatggtat cctggagacg tatgtgtgaa taataaaaat 60

gtcagcacct gctgcaaaga accactgaat gaaaggaaag gaaaatacca tacatcaaag 120

aaagaggttt atttagattc aaagtcccct gggttaggac cacacctagt acactgtcac 180

atccaataaa tgttgagaag agactgtcta gcacatgaga cacagttgtg gagatgtcct 240

aggcaggctc caccacagaa gcaaaacatg atccagttgc aatggaaatc cactctttgc 300

tcatgtgcta tttcccaagt acttgcttat taaaatataa ctctgctaat gaaccaaaca 360

cagggagttg ctttctagag ccgaaaaaaa gggactgtga g 401

<210> 254
<211> 401
<212> DNA
<213> homo sapiens

<220>
<221> variation
<222> 35
<223> /replace="c"
/replace="g"
/replace="t"

<220>
<221> variation
<222> 59
<223> /replace="c"
/replace="g"
/replace="t"

<400> 254
gttgcaatgg aaatccactc tttgctcatg tgctntttcc caagtacttg cttattaana 60

tataactctg ctaatgaacc aaacacaggg agttgctttc tagagccgaa aaaaagggac 120

tgtgagaaaa agctagggtg gtctaaggag tgaattagtg acaatgtcag tggatcaact 180

cccaacacat aggttttcag acagggaatt agtctaggca gaagttcatc agacccagtt 240

cttagatgtc tttggtcttt gtggaagatg ctgttggtgc cctgttcgtg tactccatgg 300

gctcttactg gaagcaccat ggctcattca ttatcagggg gctttcttga gcacactttc 360

ttgcacaagg tggggcacag gctggcattt gtaggggagg t 401

<210> 255
<211> 401
<212> DNA
<213> homo sapiens

<400> 255
actgtcctct agggtgctaa cacagtaaga ctggatacct ccttagacca gtgcacagca 60

tggtcaaatg cactgaatcc catccacaaa gacagcctca ttaactattc agaaaattgc 120

tattttgtgc ttctaatctc tgttaagaca ctgacaagaa gtcattctag tcatgtgtta 180

cagacatgga gatacaaagc tcagattcgc cttcaaggaa ggacttgtgg cccagatgca 240

ggggtgtggc tatcaggtgc ctccagggtg tgtctcagct gcagagatca tccttcctag 300

ggtagcgtgc atctggtggc tgagtgaaat ggagaataaa agtcttgcag ttccccctga 360

tgcttgatga cttgcgcaat actcactcta gaggtccctg t 401

<210> 256
<211> 401
<212> DNA
<213> homo sapiens

<220>
<221> variation
<222> 35
<223> /replace="t"

<220>
<221> variation
<222> 94
<223> /replace="t"

<220>
<221> variation
<222> 145
<223> /replace="t"

<220>
<221> variation
<222> 190
<223> /replace="t"

<220>
<221> variation
<222> 222
<223> /replace="t"

<220>
<221> variation
<222> 276
<223> /replace="g"

<220>
<221> variation
<222> 298
<223> /replace="c"

<400> 256
gaggtttcat ggagagtgct ctcaggatcc acacctgcga aataactata ataataataa 60

taataataat aataataata ataatagtga aggcctggac tgggtagagg aagaaactga 120

tccaaaacaa acgtgcaatg aggcctcagc caattcctgg ggggcagatc tggctgtgta 180

cagcagttcc tgaataatgt tggttcattc gatgttcttt ccttataacg ttgatgagaa 240

aacaattgat ttgcacctgg ggccaccatc tgtgtatatt tgcacaacct ccccataact 300

gcatgggttt tctctgagta ctctagtttg ctctctcatt ccaaagtcat gcacattagg 360

ttaattggca tgtctacatg gactcagtgt gagtgaatgt g 401

<210> 257
<211> 401
<212> DNA
<213> homo sapiens

<220>
<221> variation
<222> 42
<223> /replace="g"

<220>
<221> variation
<222> 64
<223> /replace="c"

<220>
<221> variation
<222> 181
<223> /replace="g"

<220>
<221> variation
<222> 225
<223> /replace="g"

<220>
<221> variation
<222> 396
<223> /replace="t"

<400> 257
tgagaaaaca attgatttgc acctggggcc accatctgtg tatatttgca caacctcccc 60

ataactgcat gggttttctc tgagtactct agtttgctct ctcattccaa agtcatgcac 120

attaggttaa ttggcatgtc tacatggact cagtgtgagt gaatgtgagc atgggtgtga 180

atgtgctctg ccatgagaga ttgtcctgtt cagggctggt tcccacctca ctccgtgagc 240

tgctgggatg ggctctggcc acccatgacc atgaactgga aataagcagg taaagaatta 300

tcttacttgg ttttattaat ctttcttaaa tgtatgtata gttcacattt atttcaatgt 360

ttaatattag aaatgttttg cctttattta gaagtgtggt a 401

<210> 258
<211> 401
<212> DNA
<213> homo sapiens

<220>
<221> variation
<222> 81
<223> /replace="g"

<220>
<221>  variation
<222>  108
<223>  /replace="t"

<400>  258
attgattttt atgtctatgc ttacaccagg agcacactgt tttcattttt gtagcttttt      60

agagagtttt gaaatcaata agtgtctttc aattttgttc accttttcaa aaataatttt     120

ggttattctt gagtttgcac ataaaatttg gaatttctga aaaaacaaaa actgagattt     180

ttatagggct tgtattgaat ttttatatca gttttgggaa tattgtcatc ttaacaatat     240

tgagtcttcc aatccatgta tatggatgtc ttttcattta tttatgtctt taaaattttt     300

tttaaataat gtcctgtagt tttcattaaa tatcttacac ttctttggtc aaatttattc     360

ttaaatatat tataattttt gatattactg taaatgatat t                         401


<210>  259
<211>  401
<212>  DNA
<213>  homo sapiens

<400>  259
aaattgggac aacttagtgt tcgagaaata gagaaatagc tcaaggagct attagatacc      60

aacccatacc atgttgtttt ctgagggtgg gagaaactag aagaaagttt gacattactc     120

tcattccatg agacaacctc aagaacacaa acccaaacta tccaggtagg acttttagtt     180

tagggagacc accaaaagaa atggagccca tttcctctgg tgattcaaca agattaacag     240

cccctcctga agtttttgtc cagtggccta gaatctaaat atgtgctcat acccagttct     300

ttgtcttcaa ttcccgagtc ttctgtgtga ctcattctga aagtccaggt gtggaaggga     360

gaggcgcatg gaggaaaaag ccaggctcag aatttatatt t                         401


<210>  260
<211>  401
<212>  DNA
<213>  homo sapiens

<220>
<221>  variation
<222>  41
<223>  /replace="g"

<220>
<221>  variation
<222>  139
<223>  /replace="g"

<220>
<221>  variation
<222>  399
<223>  /replace="g"

<400>  260
atgtatctat ggttgagaaa ttcccattaa atattggggc aaggttcaga tgctgtagat      60

atttgtttac attttatcaa atgctttctt acatgtgaac ccctcaatct ctgagaaatc     120

acctaaggga agccatggat ctgcctcctc caggtttcat tattgcatgg aatgtgaggg     180

gatttctgtc ttaattaatg tataagccag ctcttctgag tttgggtgga tatccttgag 240

gagaaaggta gctaaaatta gcagcctcta ctctcttctg tgttccttat aaaaaaaaag 300

agaaactgtc tcatctttaa gaatggacca gttatgaact tccaaccttt ccattaatgc 360

tttgaagcaa tgatggtgct ggctgatgtg cttaagtaag a 401

<210> 261
<211> 401
<212> DNA
<213> homo sapiens

<220>
<221> variation
<222> 156
<223> /replace="t"

<220>
<221> variation
<222> 290
<223> /replace="c"

<220>
<221> variation
<222> 388
<223> /replace="g"

<400> 261
gatttaacaa gaactgcatg tattccctgt tatgtgcatg taggtatgag tgtatgtgtg 60

ttatgagtgt gtatgtgtct atatgtatgt gtgtgtttgt gtctgttggg gagagaaatg 120

ggataaaaag gatcagaatc tcacgtagac ttttcctaag tcattttccc cccaaagtct 180

catttgtcca cctctatgaa acttacttgg ctgagaactg ctgccaaatg atccattttc 240

taatgtgtta gtaatcacta catgaatcct cttgagaaag agctgggcca ggagaactta 300

atgacttcca aggtcacaca gcaaatccag ggccgagcct ggttccccgc tcctgttcca 360

ggctcctccc atgccaccac atcatcaagc ttgacatatg t 401

<210> 262
<211> 401
<212> DNA
<213> homo sapiens

<400> 262
aaagcatctt ccaacaaaaa aataattcag gaccaggtgg cttcatggct gaattctact 60

gtactttaaa agaacaccta actctaattc tcctgaaact attccaaaaa ttgaagagga 120

gggaattctt cctaattcat tccgtgaggg cagtattgcc ctgatattaa agccagacaa 180

tgatgcaaca aaaaagaaaa tggatgaaca tagatacaaa aattctcaac aaaatactag 240

caaactgaat ccaagggcac ataaaaaaat acaccacgct caagtggaat ttatcccatg 300

gacacaaaga tgattaaacg tatgcaaatc aataaatgtg atacatctta tcaacataat 360

aaatacaaaa accatgtgat catctcaata gatgtggaaa a 401

<210> 263

<211>  401
<212>  DNA
<213>  homo sapiens

<400>  263
acgtaagtgt ggcagcatct aaacaaatct gtcttcattt caaggaagag aacgctgata      60

atgcacatgg atgttgcaac aatgtcaaat tgctatgaaa atttctaaat gaattttctg     120

ctcaattgcc taccttgttg caaattaata acaggcagtt aacataactg gtagtgttct     180

gcagaccaca ttctgagtaa tactacacaa tttactgcct gcatccctag aggggatggg     240

ggaagaaata ggctaaatcc taaaatgcca tgatgccata ttcaaatagt gttttatgaa     300

gaattgttca tttggcattc agcactgggc agtgattgaa cttgagggag gcaaggagtt     360

ttagaacctg taatatacca ggtaagaatt tataaaaagc t                         401

<210>  264
<211>  401
<212>  DNA
<213>  homo sapiens

<220>
<221>  variation
<222>  153
<223>  /replace="t"

<400>  264
tcatttggca ttcagcactg ggcagtgatt gaacttgagg gaggcaagga gttttagaac      60

ctgtaatata ccaggtaaga atttataaaa agctaacaga acaggttttc ataaaagtgg     120

gactgagagt tttagggaca tgcagaaaaa atggggatat tgttaattat cccttatcct     180

tatttattac acttgtcatt agttattata attaattatt agtgatcctt aattaatctt     240

aaatatttta tgattattta aacatgaatt ttatgaatat ttaaataatt ttgtaaatat     300

cataaaatat tttatgatat ttatcaattc agttatttca gataaaagca ctgaattagt     360

agattctgaa caatcagtta agactgatca aatactaaat c                         401

<210>  265
<211>  401
<212>  DNA
<213>  homo sapiens

<220>
<221>  variation
<222>  129
<223>  /replace="g"

<400>  265
ttgggggaat cctttccatg cctttctctt taactgatct caggcaaaat gtttgtcctg      60

gatgtacaat acttaaaaga gttgatttaa aatttcctga aaataagttc atctcagctt     120

ttgtgtgaaa aaggaagttc tatgttggct ggtgaccatt tcaaagctac cctcagtctc     180

acaattagtg tgcttggcaa tgctattgga cattggaatg caaatgttca catcccacct     240

tgagcttgca ggattttgat gcatttcctt tttgttggac aaaatagtga gtattcccta     300

agattacttc cagttgcaac ctgaacaaag agaaatgcat ctaaatatcc tgctccaaag     360

cctttgaact tcaccaatgc ataactcatg ggacacttat c 401

<210> 266
<211> 401
<212> DNA
<213> homo sapiens

<220>
<221> variation
<222> 88
<223> /replace="t"

<220>
<221> variation
<222> 161
<223> /replace="g"

<400> 266
ggctgtttct aagactgatg tggcttaaca aggaattttc ccagaccatc tctactctcc 60

acaattttca aacaggatta ttactttgtc tttcacctca gtgcccatgt gtttgtggat 120

ccctctagat acttgctttg ctatttctta catctctaat atgaagaatt aatgtcccag 180

ttataagaac tttatgtcct gtacgtagat gttctctagt cacacccctg acatgtgtga 240

ggcctgggaa gaggcccaca tcccatgggc acaatattta aaagttgtaa atcaagtggg 300

aaagttgtta aataaaacag attctattct tctacattgt cagatattcc accttcatca 360

tgataaaata gaaaaatatg tgcaaacagg tttttgtgtg a 401

<210> 267
<211> 401
<212> DNA
<213> homo sapiens

<220>
<221> variation
<222> 13
<223> /replace="g"

<220>
<221> variation
<222> 26
<223> /replace="t"

<220>
<221> variation
<222> 247
<223> /replace="t"

<220>
<221> variation
<222> 275
<223> /replace="g"

<400> 267
ctatccaatc taatctaatc taatgcaatc taatctaatc taatctaatc tctatctagt 60

ctatcatcta tctatctaat ctatcatcta tctatgtatc tatctaatct atcatctata 120

tctacatatt tttcaagtgt ttacatgaag acagatatta tgggttggaa ttaatgggac 180

agctgagact atggtttgag tcaatcagaa gagcttggat gctaaatctt gctaggtaag 240

agaaaccttg agaaagaagg gtaaaaggaa gtggacaaag ttacataatt tatttaattt 300

gtaatgaaac acagtggata gtattaatta taattttaca tataatttct gggataagaa 360

acaaaagcag ttttattagg taaaatatta gtctgaaaca t 401

<210> 268
<211> 401
<212> DNA
<213> homo sapiens

<220>
<221> variation
<222> 214
<223> /replace="t"

<220>
<221> variation
<222> 348
<223> /replace="c"

<220>
<221> variation
<222> 381
<223> /replace="g"

<400> 268
cacgtaagaa gtgccttttg cctcctgcta tgattctgag gcctccccag ccctgtggaa 60

ctgtaagtcc agttaaagct ctttttgttc ccagttttgg tcatgtcttt atcagcagtg 120

tgaaaatgaa ctaatacgga tggatagaag ggtggaggga tagatggata gaatgggtgg 180

actcaatctt gttgcccttc tttttcttca ccccgtttct ctaccctctg caggaagagc 240

ccaccttact tctcagtcta agaaagtggc aagcactgct gggctctttc cacacctgta 300

gttcctcagt tttctgaaac ataggttgtg tgtttctaat ctggcaaaga cttgaaggaa 360

aaaaaatcct ttttaaagtc attgtaaaca gatattactt t 401

<210> 269
<211> 401
<212> DNA
<213> homo sapiens

<220>
<221> variation
<222> 39
<223> /replace="g"

<220>
<221> variation
<222> 54
<223> /replace="t"

<220>
<221> variation
<222> 55
<223> /replace="t"

<220>
<221> variation
<222> 164
<223> /replace="g"

<220>
<221> variation
<222> 395
<223> /replace="t"

<400> 269
tttgtgtact ttttgattgg ttgtctcagt aggcatcaaa tcacttgttg cggcgtgttg 60

gggggagctt gttgatgcac agatagtggg gttccatcgc cagggtttct ggttcagaag 120

gtctggtgtg gagactgaga atttgcattt ctaacaagtt cctaggtgaa ctgcagaatt 180

acattttaag aacaactgtt tggagcaccc ttgccttaat aatgctgcag agactttctg 240

ggctggtcca ggtgtcttgt ggctccgctt ctgctgtgtc aatttttgaa cagcatgtgg 300

aggaaagatg ccctctccag ggtgtggtga ggagtgagga caatgtgggc atgtctgagc 360

tgtagtgggc atgggtgatt cagcaaggac tgaccggaca a 401

<210> 270
<211> 401
<212> DNA
<213> homo sapiens

<220>
<221> variation
<222> 321
<223> /replace="g"

<220>
<221> variation
<222> 386
<223> /replace="g"

<400> 270
tgcagttcag acacagtggg tttcctgcaa ctcacttttg ctatggcgac ttttgttatg 60

cacagtgaac aggaagtaga aatttgcaga atataagcct tcttcacagg aaacgtattt 120

tggagctcac cacaccatac aggctgctga tttaatacaa ccaggatatg tgcaaatctc 180

cgaggaatgg caaatgccat tcaccttcac ttttttcacc ttccttctcc tcctcctcct 240

caccttcatt tttcaataac ccaattattt gctggtgcat ataaaagtaa acagaaaatg 300

aataagctgt ggactttgaa agatagtttt tgagttagaa atctgaaggg ggagaaaagg 360

ccacagaaac agggtaaaca gttaaagcaa acattttaga c 401

<210> 271
<211> 401
<212> DNA
<213> homo sapiens

<220>
<221> variation
<222> 305
<223> /replace="t"

<220>
<221> variation
<222> 324
<223> /replace="g"

<400>  271
ttttgtagca gaggaatcat tcttgaaggt cccaagatcc tttctgctag acttcctatt      60

cttatagttg tttctaaact taattctttc tggaaaacag aataactgca agaaaaaatc     120

tacatcctga aagggttgaa cccatcttga aaatggcaaa atctccatcc tggtgatcac     180

tggcaccatc actcccagta tagccatcac tctatgggat cctgggcact gcttagcact     240

aggaaggcga atggctttaa attatgattc ctgtcctcaa gagataagtc acattgttga     300

aaacctgaca tcaacgtaca aagattcata atcataaaag gtaactcttg gtaagtcatt     360

ataatcttat taattgcttc taatggtaag tcacatcaat g                         401


<210>  272
<211>  401
<212>  DNA
<213>  homo sapiens

<220>
<221>  variation
<222>  38
<223>  /replace="c"

<220>
<221>  variation
<222>  63
<223>  /replace="c"

<220>
<221>  variation
<222>  279
<223>  /replace="g"

<400>  272
tattatcttt gatgcaatgt tatcattata ctttatgact tttatcatgc tttctcttag      60

ttatttgaac ataattataa tggctatttt gaagtctttt tctcttaaat ctgatgtttt     120

gtcattctca cagacaaatt tatgttttct gctttttctg gtatatggct catatttttc     180

tgtttcattg catgcctcat tactttcttt tggaagttgg acaatttaga tcatatgtat     240

tgcagcaact gtgggtagta gcccttcccc aatgcttaac ttgtattgtt atttgcttct     300

ttatgtttta gtgagtggct ggattatttt agcgatgtct gtttacctcc aacagtggta     360

aacctctgag gtcgtttctc agggaggtgc agttttgagt a                         401


<210>  273
<211>  401
<212>  DNA
<213>  homo sapiens

<220>
<221>  variation
<222>  4
<223>  /replace="t"

<220>
<221>  variation
<222>  382
<223>  /replace="g"

<400>  273

gaagtctgac agagagtgtg tgtggttata gtggactgta tcagttcaaa gacccaaaga 60

ttagctattc gtttgtgctt gggttttaaa gtggagtttt ctgagctttc tttgttcagt 120

attcactaca attgagtaga ttttggggaa gaaatctgta ctgtatttgt gtaaaattac 180

aaaataacac tggtttatgt gtgatgttat atgttttccc caattttctt tttattatcc 240

tttgtaaaat tgcaaggcaa tacagccttc caattacagg gaccactgac aaacgtaaac 300

agccatcaag taccagttgg cacacatctc ctgtagtgca tgccattgcc atcgcaccac 360

tcctcaaaaa tgcacccaca aacttattca caattacgga t 401

<210> 274
<211> 401
<212> DNA
<213> homo sapiens

<400> 274
gactgggcaa tttacaaaag aaagatattt aagggactta tagttccaca tggctaggga 60

ggtctcacaa tcatggcaga aggcaaggag gagcaagtca tgttttccat tgatggtggc 120

agtcaaagag agagctgtgc aaggaaactc ccatttttaa agccatcaga tctcatgaga 180

ctcgttaact atgaggggaa gagtgcagga aaaacccaac cccataattc aatcacctcc 240

caccaggttc ttcccgtcat acatgggaat tgtgggagtt acacttcaag atgagatttc 300

agtggggaca cagtgaaacc atatcattcc accccagacc cctcccaaat ctcatgtcct 360

cacatttcaa aatcaatcat gccttaccaa cagtccccca a 401

<210> 275
<211> 401
<212> DNA
<213> homo sapiens

<220>
<221> variation
<222> 38
<223> /replace="t"

<220>
<221> variation
<222> 45
<223> /replace="t"

<220>
<221> variation
<222> 118
<223> /replace="g"

<220>
<221> variation
<222> 144
<223> /replace="g"

<220>
<221> variation
<222> 349
<223> /replace="g"

<400> 275
tattcattaa actattccac aaataaatgt catatgagaa cttacagctt taataagagg 60

atgaaacaac tctggaaaat cagaaaagaa tgaatgggaa tcaactcact gatgtgcaat 120

gatggaggat gctgagggct ttaagctgca gcaacttact taataagttc ccaagcagga 180

gagaacttgg cttctacagg gaattaaaaa gtttgccaga acaagttggt ctggagacac 240

aggcagctca gagtcttatt ggtcatgcta aggagatctg agtcttagtt acatgaacaa 300

tcaaaaactc ctgaagagtt ctaattctgt tgacaaatta tcagatttat cttctgaaag 360

gaacactctg acagtcttgt ggaaaagaaa ttggaaatgc t 401

<210> 276
<211> 401
<212> DNA
<213> homo sapiens

<220>
<221> variation
<222> 45
<223> /replace="t"

<400> 276
ttggacacat tgcattgcat acatttattt atcaagtcat tccacaagaa tgtactgatt 60

gtcttctgtg tgtcaggcat tgttgcaggc tctggggatt cagcaataca taatttgatg 120

atcgaatttg atgtatcttc acaacatcca agaggaacta tcagataggc ctttggacat 180

atttggtctg gacctcagaa tgtaggtgtg aattctgagt tgcaactata catgtatgtg 240

tcatttgtgt gtaggtgaca attgaagctt ggggcaaaga tgagatgctc tgattcttag 300

attctaaggt gagatcaata aataagaaga aaaaaaggag tcatctctgg gactacacct 360

tgagaatctc aagttttaat ggatggctta ggaattactc a 401

<210> 277
<211> 401
<212> DNA
<213> homo sapiens

<220>
<221> variation
<222> 214
<223> /replace="t"

<400> 277
aactcaggat tttgctcact attagaactt agaggaggtc ccagacacca gatgctatga 60

cagtaccaca gaaaagcatc aaataaatgg cttaaataaa ttatctgtca ctcaaggaaa 120

tactcttacc atctgcagag gtaagaactc ctggtctcac ctctgcatgt caaaaactcc 180

aaagagctac aagaagaaaa gcttgaaact catgttctcc caatgttcag tcttcttctc 240

aacacaaggc atatcagatt ggaagctgac ccctgtgtcc tgacaccatg gattctataa 300

acttgctgaa tgactgatgg agactgatgc catcttccta aaaaatgagc cattgtggtc 360

cttagcagtg ggcaataaaa aggagagcta tctactgcat t 401

<210> 278
<211> 401

<212>  DNA
<213>  homo sapiens

<220>
<221>  variation
<222>  273
<223>  /replace="g"

<400>  278
aatccatttt gtctcttctg ctattttgga gactgctctg aaaatgtttt tcatggtttt      60

gcatgactct tgggatcacg atttgcatct taaaaaaagg agtcatggaa aatattgcaa     120

gatatgcatt tggtggagag agaatgcttt tcagcttgaa gctatttggg ggtctgatga     180

tctgatgatc tttctgttgt tggagctttg agcaggcaca tgctgtagag atgctcatgt     240

gtcactgggg agctggtttc tgatgcgact ggaacttata gatatttgat aagtcatctt     300

ttccttctga tactgccgtc aattcagtga attgaataca tacagagtct atggtaggag     360

agcaaaagat caaaaggccc actatgttac ctccccacac t                         401

<210>  279
<211>  401
<212>  DNA
<213>  homo sapiens

<220>
<221>  variation
<222>  292
<223>  /replace="t"

<220>
<221>  variation
<222>  356
<223>  /replace="t"

<220>
<221>  variation
<222>  361
<223>  /replace="t"

<400>  279
ggaaatgccc ttgaagctga ggctggaagg acacggagga gctgcccaga gggaacagcc      60

agtgctattt catgatggga cagagcatgg caggtatgag caagagaaag aggccagtgt     120

ggctggagcg taatgcaaga aagagagtgt gacaaggtgc agctgagcag gtgggagggg     180

tggcatcaaa cggggcctag gatttacgat gtgacttgca gaacaatagg aagcaccaaa     240

gattgaaagc ctagtagtga catgagttta tgatgtttaa ggattcttcc acctgcaatt     300

caaggtggat tggggggtca gatggggcaa tagtgggtga gattttaatt aggaagctcc     360

ggtaacccag ccaataagga tggcagcatt agctatggga t                         401

<210>  280
<211>  401
<212>  DNA
<213>  homo sapiens

<400>  280
tggaaagtaa ataggagagg agaaactcaa tggcacattt tcttctcttt caaacacatg      60

ttcttattta ataaattctc tctcaaataa aataagcctg tagttattgg tggtcagttt 120

catgtaatag gagctactac agttttccca ttaaaaaaat aatacaagac tgcaataaag 180

catatagtga gtcactttat tgggtacgaa gtaataaacc agtggttaca atgataacgt 240

ctatgatgtg tcttagaaga aacaaccttg attgttttgt ttggtttggg gtggggcttt 300

ttggtttagt ggcacagaaa accaatatgg gatcagaccc cacccttcaa atcagtgttc 360

ttatagcaac tggaaaattt cactctcagc aatgatcaca a 401

<210> 281
<211> 401
<212> DNA
<213> homo sapiens

<400> 281
agagggttaa gtttacaagc atcctatctt catagcactc aataatagat atggctttat 60

tgtgtggtca catccattca tctaggctgg tgatataaat tagtggtcaa agcattcgct 120

ttggaatcat acaagcctta atttaaatta tagccccacc acttaagaga taggagacct 180

tgggaattca tgtatgttct tcgagactta atttccttac ctgcaaaatt aggataataa 240

tattaatagt tacattataa aattgtgaag attaactgat atatataata agcttagcac 300

aatgtctgac acagagcata agctcaagaa atgatagctg atgttactac ttttagaggt 360

ccatgtccat aaaacccttt atagagaaat tggaagtagg a 401

<210> 282
<211> 401
<212> DNA
<213> homo sapiens

<220>
<221> variation
<222> 41
<223> /replace="t"

<220>
<221> variation
<222> 231
<223> /replace="g"

<400> 282
ttaactgtgc cagagaagga aatgtacctg caattgctca ctggggtaat atttaagtca 60

aggctggatt tcaagctcaa tttaagtgct acattttgat tttttttaag aagaaagatt 120

tgcctgtaaa aatgctactt ttcttataca agatctataa acatgctttg acaacctttg 180

aaattgatta aaagtgggct tatatttcta aatggagatg ccttgagtca agatgagtaa 240

gctaagaaaa tctattcatt ttagatatat tttaactctc cttactgtgc attgaaatta 300

tcaataattt ccagttattc tttgtttata ttttggcacc attttattcc cctcccctca 360

aaaaaaaact ccaaacacta aggtgaaatg taagcaaata g 401

<210> 283
<211> 401
<212> DNA

<213>  homo sapiens

<220>
<221>  variation
<222>  76
<223>  /replace="g"

<220>
<221>  variation
<222>  296
<223>  /replace="g"

<400>  283
gtaagcaaat agtactggaa attttataaa ttttatacat agcaatcaga agtttataaa      60

gtagatcatt ctgaaagcag attatgttaa tcagtcaaac tatttaaaag caggagtgcc     120

aatgtggggg taaactcaac tctttcgaag agctcatttg ttaacacatt tatcgatatc     180

cccagttgcc atctccttaa tcttttgctc actcggagga aaattcctaa tagcaagata     240

cacatgaaga cagcagaaca tttacaagaa agtcagccag acatgtggca gccaaagtct     300

gtcaaagtgt agagaatttt ttgtatgtgg caaaatacac ataacataaa attgaccatt     360

ttaaccattc ttaaatgtac agttaagtgg cattaagcac a                         401

<210>  284
<211>  401
<212>  DNA
<213>  homo sapiens

<220>
<221>  variation
<222>  357
<223>  /replace="c"

<220>
<221>  variation
<222>  371
<223>  /replace="t"

<400>  284
ttggtgagtt ttcccatttc tcctcttcca acaaccttat tcagaagaag ctgtagctac      60

ctgtttttgg cagaggccag agagtttgag caccttttca ccagctcacc aactcaatgg     120

tggactttag caaagcatta tctaatttta gtacccacct gcttacccac tatactataa     180

actacaaaaa gcagaacagc gtagcatttt tgcataaatg gtggatcaac agaccagaac     240

tgggagccct ggaatgaatg tgtgttagag gaaccattgc ctgtcaagga atatgatatt     300

attcaaagat gacttaccta tttggaaaaa taaaacaaag cattctcata ttatatgaca     360

tattaaaata cattccacct ggattaagga gttatcgaaa a                         401

<210>  285
<211>  401
<212>  DNA
<213>  homo sapiens

<220>
<221>  variation
<222>  36
<223>  /replace="g"

<220>
<221> variation
<222> 37
<223> /replace="t"

<220>
<221> variation
<222> 188
<223> /replace="g"

<220>
<221> variation
<222> 283
<223> /replace="g"

<220>
<221> variation
<222> 293
<223> /replace="g"

<400> 285
aacgggatgg tattctcaaa cttttctgtg cataaaggtt atggagagct tttaaatatc 60

cccatggcct ggacacatcc aagaccaata aatcgcaatc tcagttattc aaatttgcag 120

gtaatttcaa tgggtggtag ccaagtttga gattaaagta aagaagcaag gttctgtaaa 180

cttttcaagt gatcccatct tgcaaacgag gaaaactgca atctgctttt tcacagccat 240

cgtgtgtttc agcaattgca aaatattctc agactatctc taagagtccc taagactctg 300

gtcacatcac ctcctgcagc aagttgacgg aaggccacta tacttggtga cttttctctc 360

atgcattcac tttcacctcc tagagctttc ttttggaccc a 401

<210> 286
<211> 401
<212> DNA
<213> homo sapiens

<220>
<221> variation
<222> 199
<223> /replace="g"

<400> 286
ggtagcatgg gttatctatg ttattattat acctcttcaa gcctgtctta aaaccgcccc 60

atccaggaag ttccaggtta ggaaagcgtc ttctttttaa actaagcctt tgggcttaag 120

atattgtcga gatctgcgaa ctgtgagtaa tggctcattc ttctccatgt gtatcttgat 180

tttttgacac aggcttcaaa tcctacctaa actgcagacc aatctctgaa agcaactttg 240

ttcaattgca gcctgactag gtaaggcttt gagaatttgt ttctgtttta aatcctacag 300

gataaaagag gtggagggtg gtctataata ggatgaagtg gtatataata agtatttaac 360

ttaaaagaaa atctaccatt taaaactgta actagattat t 401

<210> 287
<211> 401
<212> DNA
<213> homo sapiens

<220>
<221> variation
<222> 248
<223> /replace="g"

<400> 287
tattcttttt ccctttgtta tgtttcttcc atttccaagt ggatgaagct ctaactcaac 60

ctttcatttg tttcaagagg gaggtatcag aaccacaaca aatggaatga aacctaccac 120

taccacctgc ctggcacaaa gaaataattt acaattcacg gggaatttcc tgtgtttcat 180

tttgtctgaa gaactgtaac tggacttgaa tgaactaagt gtctaattgt tgcattattt 240

ttcttgtaga gattttagtg ttttattact ttctccagtt tacttcaata gtgaattagg 300

ttccctctac catctgaaat gttacagttc gataaaatag gtaaattaat aaagtctaaa 360

gtgcttccaa gctgagcaga gacctttctg tgcgctgtgc t 401

<210> 288
<211> 401
<212> DNA
<213> homo sapiens

<220>
<221> variation
<222> 9
<223> /replace="g"

<220>
<221> variation
<222> 217
<223> /replace="t"

<220>
<221> variation
<222> 324
<223> /replace="c"

<400> 288
tttttaccat aactattttt attaggttcc taagtgataa gatcaggaca aagacaggtt 60

cattatgcta gttagacatt ttcctaaagt atgataaaat aaatgaaaag gaaaacaata 120

cctgtttatc tgagatagaa gaagtgttta caagttgtta tgctgcattc tagttgtgct 180

aggggttaca gggatctata tgtgtgttta tgctcacggg atgtacacca aaaagaagtc 240

aattttactg tatgttaatt acaaaaagaa agctattaaa tcttcctcaa atcctagaaa 300

aatataagta cacattattt cccacacaaa ctcttgtgaa gggttaacaa aactcatctg 360

gctttatttc atgcaagact tagaaaggct aatagtacgg a 401

<210> 289
<211> 401
<212> DNA
<213> homo sapiens

<220>
<221> variation
<222> 185
<223> /replace="t"

<220>

<221>  variation
<222>  308
<223>  /replace="c"

<400>  289
ttttattagg ttcctaagtg ataagatcag gacaaagaca ggttcattat gctagttaga      60

cattttccta aagtatgata aaataaatga aaaggaaaac aatacctgtt tatctgagat     120

agaagaagtg tttacaagtt gttatgctgc attctagttg tgctaggggt tacagggatc     180

tatacgtgtg tttatgctca tgggatgtac accaaaaaga agtcaatttt actgtatgtt     240

aattacaaaa agaaagctat taaatcttcc tcaaatccta gaaaaatata agtacacatt     300

atttcccaca caaactcttg tgaagggtta acaaaactca tctggcttta tttcatgcaa     360

gacttagaaa ggctaatagt acggaactgc agatcaaacc a                         401


<210>  290
<211>  401
<212>  DNA
<213>  homo sapiens

<220>
<221>  variation
<222>  281
<223>  /replace="g"

<400>  290
cctccctcaa aatacttgca tctggaattt ggctcctttt ctccctgctt aaatggtata      60

gtgcaggtga taactgcgta gataaacctt tatagttaga aagagtctaa atgttggtgt     120

ctgtaagcac aataagtaaa tgagtttggt ttttccttcc aaaataactg aacgttcact     180

ttaaatataa tgttagccag ttttgaatcc agaatattta catcttgcag actgtgtctt     240

gcagcctctg aatcaactga ctgggagcat ccgtctctgc atgcagggct gctgaaaggt     300

gggagcagag ataaaagaca ggagatggga agaaacaaag ggagccaaga gatttcatag     360

aatgctgcaa gagacattct tttgatctat ttggactcct c                         401


<210>  291
<211>  401
<212>  DNA
<213>  homo sapiens

<220>
<221>  variation
<222>  291
<223>  /replace="t"

<400>  291
ttgaaaagat agtgtcaaaa ttgtaccaca atctggcagc tcccaaatta ttgttgataa      60

gccaatttca gacttgtcta catatcaaaa gttatttttt tctcagggaa aagaagagcg     120

tgcatgtgca aacgcctgcc actcaacaac cgtaagacta tgagagggcg ggactcactg     180

gaaggcagag agccttttta tttctctttc tctgacctat gatgcttagc catgtgcttg     240

ttattagtgg acaatcaaca aatattttct gaattaaaac ccagatttga atttttgaaa     300

cataagattt attttattaa aataagaaaa tatacctagc catatacctc cccaaattga     360

aaaaaggtat tcagacaaag acttatacac aatattcata g 401

<210> 292
<211> 401
<212> DNA
<213> homo sapiens

<220>
<221> variation
<222> 38
<223> /replace="t"

<400> 292
ccaaggtcct gaggtaacag atgtccccat taactcagcc aggaggggct gaaaggcaca 60

tcagactaaa gagagagaga gaggtaaaag gtcctagctc ctcaaactga gtgccagtgg 120

atagtgttgc acagagtggg aagagggctc atggcaggag ccaaacccaa gtgtcccacg 180

ggatgtgtgt cttgagacaa tatggccagc cccacccctc ccctcatccc tcagtgaaaa 240

tgacatccaa gtgggacagc ccagtgccat ggcagcaaga cagctggagc tggaagcaca 300

gaggggtgga gagatctctg tgggctggtg gtggggccag ggctggtggt ggggctgctg 360

gtctatgtgg tcacccccag gcacctgttg tgccatggga t 401

<210> 293
<211> 440
<212> PRT
<213> homo sapiens

<400> 293

Met Glu Gln Thr Tyr Gly Glu Val Asn Gln Leu Gly Gly Val Phe Val
1               5                   10                  15

Asn Gly Arg Pro Leu Pro Asn Ala Ile Arg Leu Arg Ile Val Glu Leu
            20                  25                  30

Ala Gln Leu Gly Ile Arg Pro Cys Asp Ile Ser Arg Gln Leu Arg Val
        35                  40                  45

Ser His Gly Cys Val Ser Lys Ile Leu Ala Arg Tyr Asn Glu Thr Gly
    50                  55                  60

Ser Ile Leu Pro Gly Ala Ile Gly Gly Ser Lys Pro Arg Val Thr Thr
65                  70                  75                  80

Pro Asn Val Val Lys His Ile Arg Asp Tyr Lys Gln Gly Asp Pro Gly
                85                  90                  95

Ile Phe Ala Trp Glu Ile Arg Asp Arg Leu Leu Ala Asp Gly Val Cys
            100                 105                 110

Asp Lys Tyr Asn Val Pro Ser Val Ser Ser Ile Ser Arg Ile Leu Arg
        115                 120                 125

Asn Lys Ile Gly Ser Leu Ala Gln Pro Gly Pro Tyr Glu Ala Ser Lys
    130                 135                 140

Gln Pro Pro Ser Gln Pro Thr Leu Pro Tyr Asn His Ile Tyr Gln Tyr
145                 150                 155                 160

Pro Tyr Pro Ser Pro Val Ser Pro Thr Gly Ala Lys Met Gly Ser His
                165                 170                 175

Pro Gly Val Pro Gly Thr Ala Gly His Val Ser Ile Pro Arg Ser Trp
            180                 185                 190

Pro Ser Ala His Ser Val Ser Asn Ile Leu Gly Ile Arg Thr Phe Met
        195                 200                 205

Glu Gln Thr Gly Ala Leu Ala Gly Ser Glu Gly Thr Ala Tyr Ser Pro
    210                 215                 220

Lys Met Glu Asp Trp Ala Gly Val Asn Arg Thr Ala Phe Pro Ala Thr
225                 230                 235                 240

Pro Ala Val Asn Gly Leu Glu Lys Pro Ala Leu Glu Ala Asp Ile Lys
                245                 250                 255

Tyr Thr Gln Ser Ala Ser Thr Leu Ser Ala Val Gly Gly Phe Leu Pro
            260                 265                 270

Ala Cys Ala Tyr Pro Ala Ser Asn Gln His Gly Val Tyr Ser Ala Pro
        275                 280                 285

Gly Gly Gly Tyr Leu Ala Pro Gly Pro Pro Trp Pro Pro Ala Gln Gly
    290                 295                 300

Pro Pro Leu Ala Pro Pro Gly Ala Gly Val Ala Val His Gly Gly Glu
305                 310                 315                 320

Leu Ala Ala Ala Met Thr Phe Lys His Pro Ser Arg Glu Gly Ser Leu
                325                 330                 335

Pro Ala Pro Ala Ala Arg Pro Arg Thr Pro Ser Val Ala Tyr Thr Asp
            340                 345                 350

Cys Pro Ser Arg Pro Arg Pro Pro Arg Gly Ser Ser Pro Arg Thr Arg
        355                 360                 365

Ala Arg Arg Glu Arg Gln Ala Asp Pro Gly Ala Gln Val Cys Ala Ala
    370                 375                 380

Ala Pro Ala Ile Gly Thr Gly Arg Ile Gly Gly Leu Ala Glu Glu Glu
385                 390                 395                 400

Ala Ser Ala Gly Pro Arg Gly Ala Arg Pro Ala Ser Pro Gln Ala Gln
            405                 410                 415

Pro Cys Leu Trp Pro Asp Pro Pro His Phe Leu Tyr Trp Ser Gly Phe
            420                 425                 430

Leu Gly Phe Ser Glu Leu Gly Phe
        435                 440

<210>  294
<211>  2838
<212>  DNA
<213>  homo sapiens

<400>  294
aaattgattc ccgcacgctg cagcctcccg gtcagacgaa tttctcccaa tcggatgaag      60

ttcaccctgg gcctggggtc gcgggcgtgg agagtgtcct gggagggggc agcagcggcg     120

gcggcaggcc ctggagcggg cggcagcgcg ctccgctgcc gcgcacagcg cgtctccagc     180

ccgcggctgg gccgccgcgg ctctcggctc tcgggcgccc tccctctatg cctctcacgc     240

ggcggcggcg gcgcccaagc tctcccggac tgcgccgggc ccagccccgg ccaccccggc     300

caccccggcg ccaggcagct ggccggcccg ctcgctatgg agcagacgta tggcgaggtg     360

aaccagctgg gcggtgtgtt cgtcaacggc cgccccctgc ccaacgccat ccgcttgcgc     420

attgtggagc tggcgcagct gggcatccga ccctgtgaca tcagtcggca gctccgcgta     480

tcccacggct gcgtgagcaa gatcctggcg cgctacaacg agaccggctc cattctgccc     540

ggggccatcg gggggagcaa gccccgcgtc accactccca acgtggtcaa gcacatccgg     600

gactacaagc aaggagaccc tggcatcttt gcctgggaga tccgcgaccg gctgctggcc     660

gacggcgtct gtgacaagta caatgtgcct tcggtgagct ccatcagccg catcctgcgc     720

aacaagatcg gcagcctggc gcagcccgga ccgtacgagg caagtaagca gccgccgtcg     780

cagcctacgc tgccctacaa ccacatctac cagtacccct accccagtcc cgtgtcgccc     840

acgggcgcca agatgggcag ccaccccggg gtcccgggca cggcgggcca cgtcagcatc     900

ccgcgctcat ggccctcggc acactcggtc agcaacatcc tgggcatccg gacgtttatg     960

gagcaaacag gggccctggc tgggagcgaa ggcaccgctt actctcccaa gatggaagac    1020

tgggccggcg tgaaccgcac ggccttcccc gccacccccg cagtgaatgg gctagagaaa    1080

cctgccttag aggcagacat taaatacact cagtcggcct ccaccctctc tgccgtgggc    1140

ggctttctcc ccgcctgcgc ctacccggcc tccaaccagc acggcgtgta cagcgccccg    1200

ggcggcggct acctcgcccc gggcccgccg tggccgcctg cgcaaggtcc tcctctggcg    1260

ccccccgggg ccggcgtagc tgtgcatggc ggggaactcg cggcagcaat gaccttcaag    1320

catcccagcc gagaaggaag cctcccagct ccggcagcaa ggccccggac gccctcagta    1380

gcttacacgg actgcccatc ccggcctcga cctcctaggg gcagctctcc ccggacccga    1440

gcccggaggg aacggcaggc ggacccgggc gcacaggtct gcgcggcggc cccggcaatc    1500

ggcacgggca ggatcggagg actcgcggag gaggaagcca gtgccggccc gcggggtgca 1560
cgcccagcca gcccccaggc ccagccctgc ctctggccgg acccaccaca cttcctttat 1620
tggtctgggt ttttaggctt ctctgaactt gggttttaga ctgccgtacc ctcctcacaa 1680
tccttgctct gacgtggcct ccttcgctct gccagcttca aatttctttt ttgtcactcc 1740
cttgtccgtc tccgtctcgc ctctctccct gtttccttcc cccctctttc tttctcactc 1800
tccctccctt ccctttctct ttcccttcct ccctaccttc caccccggct ttccccgacc 1860
ttccagggct ccctctgccc ttccactctc ttttccttgc tccgacctct gctccagtcc 1920
cgcttcttcc cccgtctcct ctttctagtc ctctatatgc tatcagccct ttttcctggt 1980
ccatccctgt cgttccttct ctctctgtct ctggttctac tgtcaagggc cctagttccc 2040
tttgttttac tgcttcccca accctccctc agtccctgag agccctagag ccatctcggt 2100
gacagtccct tctctggcac ctgggacttt gtagccttca ctcctctgag cccaccagcc 2160
ctaatggagg ggggctcaaa cctaggaagt ggacagaaaa gccacagcct ttattttgta 2220
tttttttgt aataacaaac cctaaaaaac cctccatgaa ctgtcctctc ccgtcgatcc 2280
ccgcagcata ttgtctggaa atctgttttc tttctcagag cccagtggcc gccgggcttc 2340
tcttcccttc tgagatgatg tcaccccaaa gaagactggg gtaggagtag ggagcacagg 2400
ggttgttggt gttttcagtg cttagaaaac tgggttgtcc tgcaggtgtc tcctcccacc 2460
cctccactcg caacacttgc tagggaatcc gaccagtggt aaacacatca gcactttttg 2520
gtgttcaggg gtcagcacat ttgttaattt aaaccacaga ctgcttccag gtacaaaggt 2580
tcatttggac ttctggtttg caagggatga gggttaaaaa aaacaaaaca aaacaaacaa 2640
caacaacaac aaaaacaacc cagaaagtat cttctagagc cagccgcaag agctgtgagc 2700
tgcttccaag ttaggcgagc tggtggtggt accctggaca taatgcaatt ctgtagtcag 2760
agcaatatcc ctggcgagag cctgacatcg cctgttaaag gtggctgcct ctgaataaag 2820
gaaagcgtct gaaaggtg 2838